# EUROPEAN PATENT SPECIFICATION

(11) **EP 2 799 093 B1**
(45) Date of publication and mention of the grant of the patent: **26.02.2025**
(21) Application number: 12862310.5
(22) Date of filing: 27.12.2012
(51) Int. Cl.: A61L 27/12, A61F 2/28, A61L 27/38, A61F 2/30, A61P 19/00, A61P 19/08, A61P 19/10

(54) **SCAFFOLD-FREE SELF-ORGANIZING THREE-DIMENSIONAL ARTIFICIAL TISSUE AND ARTIFICIAL BONE COMPOSITE FOR OSTEOCHONDRAL REGENERATION**
SELBSTORGANISIERENDE GERÜSTFREIE DREIDIMENSIONALE KÜNSTLICHE GEWEBE UND KÜNSTLICHER KNOCHENVERBUND ZUR OSTEOCHONDRALEN REGENERATION
TISSU ARTIFICIEL EN TROIS DIMENSIONS AUTO-ORGANISÉ SANS ÉCHAFAUDAGE ET COMPOSITE OSSEUX ARTIFICIEL POUR LA RÉGÉNÉRATION OSTÉO-CARTILAGINEUSE

(30) Priority: 28.12.2011 JP 2011289662
(43) Date of publication of application: 05.11.2014
(73) Proprietor: Twocells Company, Limited, Hiroshima-shi Hiroshima 732-0816 (JP)
(72) Inventor: Yoshikawa, Hideki, Osaka, 5650871 (JP); Nakamura, Norimasa, Osaka, 5650871 (JP); Shimomura, Kazunori, Osaka, 5650871 (JP); Moriguchi, Yu, Osaka, 5650871 (JP)
(74) Representative: Uexküll & Stolberg
(86) International application number: PCT/JP2012/008410
(87) International publication number: WO 2013/099273

(56) References cited:
- WO-A1-2005/012512
- WO-A2-03/094703
- JP-A- 2008 126 005
- JP-B2- 4 522 994
- JP-B2- 4 522 994
- US-A1- 2005 064 042
- BIOMATERIALS, vol. 27, 2006, pages 4120 - 4131, XP005402075
- TISSUE ENGINEERING, vol. 15, no. 1, 2009, pages 55 - 63, XP055076541
- TISSUE ENGINEERING, vol. 11, no. 1/2, 2005, pages 331 - 339, XP055076542

## Description

### [Technical Field]

The present invention relates to the field of regenerative medicine. More particularly, the present invention relates to a composite tissue comprising a synthetic tissue capable of functioning after implantation, a method for producing the same, and use of the same. The composite tissue of the present invention has biological integration capability and achieves a significant effect in the treatment of an osteochondral defect.

### [Background Art]

Recently, regenerative therapy has attracted attention as a novel method of therapy for an osteochondral defect or the like, which utilizes genetic engineering, cell tissue engineering, regenerative medicine and the like. A large number of researchers throughout the world are vigorously working on this important and challenging subject of research in advanced medical practice.

The scale of the market associated with regenerative medicine (tissue engineering) is estimated to be about 48 trillion yen globally and about 5 trillion yen in Japan according to materials prepared by the New Energy and Industrial Technology Development Organization. Tissue engineering products alone account for about 10 trillion yen globally. Thus, regenerative medicine has great expectation as the next-generation industry in the field.

The present inventors have made efforts to develop regenerative therapy in the field of musculoskeletal and cardiovascular tissues, and have reported a combination therapy of cell implantation and growth factor administration as well as a tissue implantation regeneration therapy based on tissue engineering. However, it is of urgent and utmost importance to secure a source of autologous cells for such regenerative medicine based on cell or tissue implantation. Many cells in musculoskeletal tissues have a high level of self-repairing ability. It has been reported that there are cells with a function as a stem cell among the cells of the musculoskeletal tissues.

Osteoarthritis (OA) is a common disease that causes arthralgia or deformation and dysfunction of joints, affecting several hundred millions of people worldwide [Non Patent Literature 1] . Clinical options for OA treatment include total joint arthroplasty, osteotomy, osteochondral implant and the like, depending on the extent of joint damage. Recently, initiatives utilizing tissue engineering and regenerative medicine have been considered.

In order to repair an osteochondral lesion accompanied by a subchondral bone defect, it is important to stabilize the subchondral bone and to promote recovery of each layer of subchondral bone and cartilage [Non Patent Literatures 2-3]. Diphasic and triphasic constructs have been developed as a scheme to regenerate these structures by each layer [Non Patent Literatures 4-12]. These structures have been reported as contributing to excellent osteochondral repair in vitro and in vivo. However, there are still several issues involving the long-term safety of these materials due to use of scaffolds or plasmids. Thus, a hybrid material that can overcome such potential issues is needed for clinical applications . As another issue of a diphasic graft, a reliable biological integration with an adjacent cartilage and regeneration of a bone/cartilage boundary can be an important factor in determining the therapeutic outcome.

Artificial bones, such as hydroxyapatite (HA) and β-tricalcium phosphate (β-TCP) , are extensively used in clinical settings for the treatment of a fracture or a bone defect after removal of a bone tumor [Non Patent Literatures 13-15]. Thus far, the present inventors have reported usefulness of novel and sufficiently interconnected hydroxyapatite (HA) artificial bones for repairing a subchondral bone [Non Patent Literature 16]. Furthermore, the present inventors have developed a three-dimensional synthetic tissue (TEC) that is non-dependent on a scaffold, consisting of allogenic mesenchymal stem cells (MSC) derived from a synovium and an extracellular matrix (ECM) synthesized by these cells [Non Patent Literature 17]. The obtained TEC was demonstrated to be useful in repairing cartilages in a study with large animals [Non Patent Literatures 18-19].

Cells derived from skeletal muscle (Non Patent Literature 20), fat (Non Patent Literature 21), umbilical cord blood (Non Patent Literature 22), tendon (Non Patent Literature 23), bone marrow (Non Patent Literature 24), synovium (Non Patent Literature 25) or the like are demonstrated to be undifferentiated and to have the potential to differentiate into various cells.

Conventionally, when cell therapy is performed for repair or regeneration of tissue, most researches have employed a biological scaffold for maintaining the accumulation of cells, allowing cell grow, stabilizing a differentiation function, protecting cells from mechanical stress on a treated site, or the like. However, most scaffolds contain a biological (animal) material, a biomacromolecule material, or the like, whose influence from use thereof on the safety of organisms cannot be fully predicted in the long term.

As has been reported in Non Patent Literature 27 and the like, a cell sheet engineering technique, led by the group of Okano et al, utilizing a temperature sensitive culture dish is a typical cell implanting method without a scaffold. Such a cell sheet engineering technique is internationally acclaimed as a cell transplant method that does not use a scaffold due to its originality. However, a single sheet obtained by this technique is often fragile. Thus, when using this cell sheet technique, it was necessary to stack multiple sheets in order to obtain strength that can withstand surgical manipulation, such as implantation.

When such a nano-biointerface technology is used to fix a temperature responsive polymer (PIPAAm) onto a plastic mold for cell culture, such as a Petri dish, the polymer surface is reversibly changed at 31°C between hydrophilicity and hydrophobicity. Specifically, when the temperature is 31°C or above, the surface of the Petri dish is hydrophobic so that cells or the like can adhere thereto. In this state, the cells secrete extracellular matrix (for example, adhesion molecules which are proteins having a function like a "glue") and adheres to the surface of the Petri dish so that the cells can grow [Non Patent Literatures 26-28].

However, when the temperature is 31°C or below, the surface of the Petri dish changes to be hydrophilic. Thus, the cells which have adhered to the Petri dish up to this point are readily detached while still retaining adhesion molecules. This is because the surface of the Petri dish itself to which the cells have adhered up to this point is no longer 31°C or above.

Even when such a Petri dish having a fixed temperature responsive polymer (e.g., trade name: UpCell and RepCell) is used to culture and detach cells, an extracellular matrix or the like is not appropriately distributed in three-dimension. Thus, a practical implantable synthetic tissue has yet to be developed [Non Patent Literatures 26-28] . Conventional methods for producing sheets have the following drawbacks: it is not possible to produce a very large sheet; it is not possible to produce a synthetic tissue having biological integration in three dimensions; and when a sheet is detached from a culture substrate after sheet production, the sheet falls apart into pieces; and the like. Thus, it is not possible to provide a synthetic tissue, which can withstand an implant surgery, can be used in an actual surgery, and can be produced by culturing. Further, it was difficult to isolate a synthetic tissue produced by a conventional technique from a culture substrate after tissue culture. In addition, it was practically impossible to make a large tissue fragment. Therefore, there were issues with conventional synthetic tissues, such as tissue sheets, not being able to withstand use in medical application in terms of size, structure, mechanical strength, and the like. Production of a synthetic tissue using conventional techniques is difficult in itself. Therefore, there was an issue of the quantity of supplies being limited.

Furthermore, it is reported in Patent Literature 1 and Patent Literature 2 that cells are cultured on a semipermeable membrane using alginate gel. However, the resultant tissue is poorly integrated with an extracellular matrix and is not free of a scaffold. In addition, the cells in the tissue are not self-organized. The tissue has no self-supporting ability. The cells no longer have a differentiation potential. The tissue loses morphological plasticity in terms of three-dimensional structure. Therefore, the tissue is not suitable for cell implantation.

In this regard, some of the inventions have developed and filed for a patent on a technique that does not use a scaffold, which was deemed important due to issues of side effects in implant medicine (Patent Literature 3).

Patent Literature 4 discloses conventional calcium phosphate-based bone substitute materials.

Some of the inventors have further filed for a patent on a safe preparation method of a cell tissue-hydroxyapatite complex (Patent Literature 5).

### [Citation List]

### [Patent Literature]

[PTL 1] International Publication No. WO 00/51527
[PTL 2] International Publication No. WO 03/024463
[PTL 3] Japanese Patent No. 4522994
[PTL 4] Japanese Laid-Open Publication No. 2001-137328
[PTL 5] Japanese Laid-Open Publication No. 2008-126005

### [Non Patent Literature]

[NPL 1] Harris ED, Jr., Arthritis Rheum 2001; 44:1969-1970
[NPL 2] Gomoll AH, Madry H, Knutsen G, van DijkN, Seil R, Brittberg M, et al. , Knee Surg Sports Traumatol Arthrosc 2010; 18:434-447
[NPL 3] Kon E, Delcogliano M, Filardo G, Busacca M, Di Martino A, Marcacci M., Am J Sports Med 2011; 39:1180-1190
[NPL 4] Hung CT, Lima EG, Mauck RL, Takai E, LeRoux MA, Lu HH, et al., J Biomech 2003; 36:1853-1864
[NPL 5] Marquass B, Somerson JS, Hepp P, Aigner T, Schwan S, Bader A, et al., J Orthop Res 2010; 28:1586-1599
[NPL 6] Oliveira JM, Rodrigues MT, Silva SS, Malafaya PB, Gomes ME, Viegas CA, et al., Biomaterials 2006; 27:6123-6137
[NPL 7] Sherwood JK, Riley SL, Palazzolo R, Brown SC, Monkhouse DC, Coates M, et al., Biomaterials 2002; 23:4739-4751
[NPL 8] Ahn JH, Lee TH, Oh JS, Kim SY, Kim HJ, Park IK, et al., Tissue Eng Part A 2009; 15:2595-2604
[NPL 9] Alhadlaq A, Mao JJ., J Bone Joint Surg Am 2005; 87: 936-944
[NPL 10] Gao J, Dennis JE, Solchaga LA, Goldberg VM, Caplan AI., Tissue Eng 2002; 8:827-837
[NPL 11] Kandel RA, Grynpas M, Pilliar R, Lee J, Wang J, Waldman S, et al., Biomaterials 2006; 27:4120-413
[NPL 12] Chen J, Chen H, Li P, Diao H, Zhu S, Dong L, et al., Biomaterials 2011; 32:4793-4805
[NPL 13] Tamai N, Myoui A, Hirao M, Kaito T, Ochi T, Tanaka J, et al., Osteoarthritis Cartilage 2005; 13:405-417
[NPL 14] Tamai N, Myoui A, Kudawara I, Ueda T, Yoshikawa H., J Orthop Sci 2010; 15:560-568
[NPL 15] Shen C, Ma J, Chen XD, Dai LY., Knee Surg Sports Traumatol Arthrosc 2009;17:1406-1411
[NPL 16] Tamai N, Myoui A, Hirao M, Kaito T, Ochi T, Tanaka J, et al., Osteoarthritis Cartilage 2005; 13:405-417
[NPL 17] Ando W, Tateishi K, Katakai D, Hart DA, Higuchi C, Nakata K, et al., Tissue Eng Part A 2008; 14:2041-2049
[NPL 18] Ando W, Tateishi K, Hart DA, Katakai D, Tanaka Y, Nakata K, et al., Biomaterials 2007; 28:5462-5470
[NPL 19] Shimomura K, Ando W, Tateishi K, Nansai R, Fujie H, Hart DA, et al., Biomaterials 2010; 31:8004-8011
[NPL 20] Jankowiski RJ, Huand J et al, Gene Ther. 9: 642-647, 2002
[NPL 21] Wickham MQ et al., Clin.Orthop.2003, 412, 196-212
[NPL 22] Lee OK et al., Blood, 2004, 103:1669-75
[NPL23] SalingcarnboriboonR., Exp.Cell.Res.287:289-300, 2002
[NPL 24] Pitterger MF et al., Science, 284:143-147, 1999
[NPL 25] De Bari C, Dell'Accio F, Tylzanowski P, Luyten FP., Arthritis Rheum.2001 44:1928-42
[NPL 26] Okano T, Yamada N, Sakai H, Sakurai Y., J Biomed Mater Res.1993; 27:1243-1251
[NPL 27] Kushida A, Yamato M, Konno C, Kikuchi A, Sakurai Y, Okano T., J Biomed. Mater. Res. 45:355-362, 1999
[NPL 28] Shimizu T, Yamato M, Akutsu T et al., Circ Res.2002 Feb 22;90(3) :e40.

WO 2005/012512 A1 discloses a combination of a synthetic tissue and artificial bone which is useful in the treatment of bone or cartilage defects. WO 03/094703 A2 discloses osteochondral implants comprising chondrogenic cells in a cell-associated matrix on a scaffold of natural bone or a bone substitute material. US 2005/064042 A1 discloses an osteochondral plug having a subchondral bone that is covered by a cap of hyaline cartilage which is implanted into a bone defect. Kandel et al. (2006), Biomaterials, vol. 27, 4120-4131, discloses biphasic bone constructs that are deemed useful for repairing osteochondral defects. JP 2008 126005 A discloses a method of repairing of osteochondral defects with biphasic cartilage-calcium phosphate constructs covered with a cartilaginous tissue, wherein the construct is press-fitted into the defect.

### [Summary of Invention]

### [Solution to Problem]

In the present invention, it was found that a significant therapeutic result is achieved, especially in osteochondral disease by a synthetic tissue with a property of being readily detachable from a culture dish due to culturing cells under a specific culture condition, such as culturing in a medium containing an extracellular matrix synthesis promoting agent, so that cells form a tissue, which is conjugated with another artificial tissue such as an artificial bone. The present invention provides applications of such a complex in this area of the present invention. Further, preferred embodiments of a composite tissue were found for osteochondral diseases, and the present invention provides a novel material based on such knowledge.

A composite tissue comprising an artificial tissue provided by the present invention has properties, such as not requiring a scaffold, having self-supporting ability, readily formed into a three-dimensional structure, having morphological plasticity, having excellent ability to biologically adhere to the surrounding, and having a differentiation potential, so that the composite tissue is effective for a replacement or resurfacing therapy at a defective site. The present invention also has excellent therapeutic results, such as excellent integration with a defective site.

A composite tissue of the present invention can be constructed into various shapes and has sufficient strength. Therefore, surgical manipulation such as implantation is readily performed for the synthetic tissue of the present invention. According to the present invention, a large quantity (e.g., 10⁶ to 10⁸) of cells can be reliably supplied to a local site by means of tissue implantation. Further, cell adhesion molecules, such as collagen (e.g., type I, type III), fibronectin, and vitronectin, are present in large amounts in the matrix. Particularly, the cell adhesion molecules are integrated throughout the matrix.

Therefore, composite tissues of the present invention have an excellent ability to biologically adhere to surroundings of an implantation site. Thus, a complex biologically integrates with a tissue of an implanted site in a very short period of time. In addition, by changing culture conditions, the composite tissues can be induced to differentiate into a bone or cartilage tissue. Such composite tissues are effective as a safe and efficient cell therapy system.

The present invention achieves a clinical application of the joint tissue regeneration using such a composite tissue. The present invention makes it possible to develop therapies for bone regeneration at a conventionally intractable site, in which both periosteum and bone cortex are inflamed, partial thickness cartilage defect which does not reach the subchondral bone, and defect of a meniscus, a tendon, a ligament, an intervertebral disk, cardiac muscle in an avascular area or a site with poor circulation.

For an ideal osteochondral repair, it is important to promote "reconstruction"=restoration of each layer of a cartilage and subchondral bone. Some of the inventors have thus far reported the possibility of materializing novel and sufficiently interconnected hydroxyapatite (HA) artificial bones for repairing a subchondral bone (Tamai N, et al Osteoarthritis Cartilage 2005 13(5) :405-417). Furthermore, the present inventors have developed a three-dimensional synthetic tissue that is not dependent on a scaffold which is derived from mesenchymal stem cells (MSC) from a synovium for repairing a joint cartilage (Herein, also may be referred to as simply "three-dimensional synthetic tissue" or "synthetic tissue". Herein, a three-dimensional synthetic tissue may be denoted as tissue engineered construct=TEC. However, each term is used in the same meaning). The present invention has enabled the materialization of a composite tissue (herein, also referred to as a "hybrid graft", but each term is used in the same meaning) comprising a TEC and an artificial bone such as HA for repairing an osteochondral defect by using a rabbit osteochondral defect model.

In one embodiment, the present inventors made an osteochondral defect in an intercondylar section of a femur of a rabbit with a mature skeleton under anesthesia. A complex (hybrid) of HA and a TEC derived from synovium MSCs was formed without using an adhesive immediately prior to implantation, and the diphasic graft was implanted in the bone defect without suturing. In a control group, HA was implanted. The present inventors further prepared normal untreated knees as a control group for a biodynamic test. The injured section to which an implant was made was morphologically evaluated at 1, 2, and 6 months after surgery. Furthermore, biodynamic analysis was carried out at six months after surgery.

The TEC immediately integrated with an HA block to yield a complex having strength that can sufficiently withstand a surgical implantation. An osteochondral defect treated with this composite tissue (hybrid material) exhibited excellent biological integration with an adjacent cartilage and a response to repair a subchondral bone and cartilage at an earlier stage in comparison to HA alone. In addition, when the osteochondral tissues treated with this composite tissue (hybrid material) was repaired, rigidity equivalent to that of normal osteochondral tissues was restored.

The present inventors demonstrated that composite tissues of the present invention (hybrid graft) histologically and biodynamically improve osteochondral repair significantly. In particular, repair of subchondral bone from an early stage and reliable and excellent biological integration of a tissue to an adjacent host tissue can guarantee durability over an extended period of time. Since a TEC is not dependent on a scaffold, a substance derived from an animal or chemical substance is not contained. In addition, HA is extensively used in clinical settings. Thus, hybrid materials by the present inventors are suitable for efficient and safe repair of an osteochondral defect.

It is especially noteworthy that TECs can be developed without an exogenous scaffold, so the risk of potential side effects induced by an artificial object or an exogenous biological substance contained in a scaffold is minimized in TEC implantation. Furthermore, an important biological feature of TECs is the property of adhering to a tissue. The characteristic contributes to a fast and reliable adhesion of a TEC to an artificial bone. Thus, a hybrid graft consisting of a TEC and an artificial bone can be quickly and readily made and is potentially suitable for repair of a clinically-relevant osteochondral lesion.

The present invention uses a rabbit osteochondral defect model in one Embodiment to investigate the effectiveness of a hybrid graft of a TEC and an artificial bone to confirm the effect thereof.

Thus, the present invention provides the following.
(1) A composite tissue comprising a three-dimensional synthetic tissue and an artificial bone for use in a method of treating or preventing a disease, disorder, or condition associated with an osteochondral defect, wherein the artificial bone is smaller in size than a depth of a defect of a bone section in the osteochondral defect by 2-4 mm, the three-dimensional synthetic tissue is substantially made of a cell and an extracellular matrix derived from the cell, the extracellular matrix contains fibronectin, collagen I, collagen III, and vitronectin, the extracellular matrix is diffusedly distributed in the tissue, the extracellular matrix and the cell biologically integrate to form a three-dimensional structure together, and the composite tissue has an ability to biologically integrate with surroundings when implanted and has sufficient strength to provide a self-supporting ability.
(2) The composite tissue for use according to (1), wherein a total of depths of the artificial bone and the three-dimensional synthetic tissue is the same or 1 mm shorter than the depth of the osteochondral defect.
(3) The composite tissue for use according to claim (1) or (2), wherein the artificial bone is smaller in size than the depth of the defect of the bone section in the osteochondral defect by twice the thickness of a cartilage or less.
(4) The composite tissue for use according to any of (1) to (3), wherein the artificial bone is smaller in size than the depth of the defect of the bone section in the osteochondral defect by 2-3 mm.
(5) The composite tissue for use according to any of (1) to (4), wherein the three-dimensional synthetic tissue and the artificial bone are diphasic, or the three dimensional synthetic tissue and the artificial bone are attached to each other.
(6) The composite tissue for use according to any of claims 1 to 5, wherein the osteochondral defect is in a mammal.
(7) The composite tissue for use according to any of (1) to (6), wherein the artificial bone is made of a material selected from the group consisting of hydroxyapatite and *β*-tricalcium phosphate.
(8) The composite tissue for use according to any of (1) to (7), wherein the disease, disorder, or condition is selected from the group consisting of osteoarthritis, osteochondral injury, osteochondral lesion, osteonecrosis, rheumatoid arthritis and bone tumor.
(9) The composite tissue for use according to any of (1) to (8), wherein the cell is selected from the group consisting of a myoblast, mesenchymal stem cell, adipocyte, synovial cell, and bone marrow cell and an extracellular matrix derived from the cell, the extracellular matrix contains more of the collagen I and/or collagen III than collagen II, and the extracellular matrix is diffusedly distributed in the tissue.
(10) A kit comprising a composite tissue comprising a three-dimensional synthetic tissue and an artificial bone for use in a method of treating or preventing a disease, disorder, or condition associated with an osteochondral defect, wherein the artificial bone is smaller in size than a depth of a defect of a bone section in the osteochondral defect by 2-4 mm, the three-dimensional synthetic tissue is substantially made of a cell and an extracellular matrix derived from the cell, the extracellular matrix contains fibronectin, collagen I, collagen III, and vitronectin, the extracellular matrix is diffusedly distributed in the tissue, the extracellular matrix and the cell biologically integrate to form a three-dimensional structure together, and the composite tissue has an ability to biologically integrate with surroundings when implanted and has sufficient strength to provide a self-supporting ability.

### [Advantageous Effects of Invention]

The present invention is understood as further encompassing use of any combination of the above-described features. Hereinafter, the present invention will be described by way of preferable examples. It will be understood by those skilled in the art that the examples of the present invention can be appropriately made or carried out based on the description of the present specification and commonly used techniques well known in the art. The function and effect of the present invention can be readily recognized by those skilled in the art.

The present invention provides a composite tissue consisting of a scaffold-free synthetic tissue and another synthetic tissue (e.g., artificial bone) . By providing such a composite tissue comprising a scaffold-free synthetic tissue, a therapeutic method and a therapeutic agent for providing an excellent therapeutic result after implantation can be obtained. The present invention, being a composite tissue comprising a scaffold-free synthetic tissue, solves at once a long outstanding problem with biological formulations, which is attributed to contamination of the scaffold itself. Despite the lack of a scaffold, the therapeutic effect is not only comparable, but better than conventional techniques. Although it is not desired to be constrained by theory, usefulness of use of a synthetic tissue in cartilage regeneration is passed on when using a proven composite tissue consisting of this scaffold-free synthetic tissue and an artificial bone with usefulness and safety that are already proven as bone regenerating implant. This is recognized as an advantageous point in comparison to conventional synthetic tissue, composite tissue and the like.

In addition, when a scaffold is used, the alignment and cell-to-cell adhesion of implanted cells in the scaffold, in vivo alteration of the scaffold itself (eliciting inflammation), integration of the scaffold to a recipient tissue become problematic. However, these problems can be solved by the present invention. Similarly, an artificial bone preferably uses components of actual bones in the present invention and is free of biological formulations and synthetic polymers. The usefulness of use of a synthetic tissue in cartilage regeneration is passed on when using a proven composite tissue consisting of an artificial bone with usefulness and safety that are already proven as a bone regenerating implant. This is recognized as an advantageous point in comparison to conventional synthetic tissue, composite tissue and the like.

The composite tissue of the present invention is also self-organized and biologically integrated in the inside. Thus, the present invention is also distinguished from conventional cell therapies on this point.

The versatility of the composite tissue of the present invention should be noted, as the composite tissue is readily formed into a three-dimensional structure for designing a desired form.

The composite tissue of the present invention is biologically integrated with recipient tissues, such as adjacent tissues and cells. Therefore, the composite tissue of the present invention achieves excellent effects such as post-operational stability and reliable supply of cells to a local site. As an example of an effect of the present invention, such excellent biological integration capability allows the formation of a composite tissue with another synthetic tissue or the like to enable a complicated therapy.

As another effect of the present invention, differentiation can be induced after providing a composite tissue . Alternatively, differentiation can be induced before providing a synthetic tissue and/or a complex so that the synthetic tissue and/or the complex are formed thereafter.

From the viewpoint of cell implantation, another effect of the present invention is that the implantation of the composite tissue of the present invention achieves effects such as a tissue replacement capability in three-dimension and a comprehensive supply of cells for covering an implanted site in comparison to cases of implanting only a synthetic tissue (artificial bone or the like) and cases of utilizing only a three-dimensional synthetic tissue, such as conventional cell-only implantation and sheet implantation.

The present invention provides an implantable composite tissue comprising a synthetic tissue with biological integration capability. The above-described features and effects of such tissues make it possible to treat a site which could be considered as an implantation site with conventional synthetic products. The composite tissue comprising a synthetic tissue of the present invention has biological integration within tissues and with recipient tissues and actually functions in implantation therapies. The composite tissue comprising a synthetic tissue is not provided by conventional techniques, but is provided for the first time by the present invention. The composite tissue of the present invention has sufficient capability to biologically integrate with adjacent tissues, cells or the like during implantation (preferably due to extracellular matrix). Therefore, post-operational result is excellent. Such a synthetic tissue, which has biological integration capability extending three dimensionally, cannot be achieved by conventional techniques. Therefore, the present invention provides a therapeutic effect which cannot be achieved by a conventional synthetic tissue.

In addition, the present invention enables medical treatment that yields a therapeutic effect by filling, replacing, and/or covering a lesion.

Further, when the present invention is used in combination with another synthetic tissue (e.g., an artificial bone made of hydroxyapatite, a microfibrous collagen medical material, etc.), the present invention biologically integrates with another synthetic tissue to yield improved therapeutic results (e.g., improved establishment of a synthetic tissue) that could not be conventionally achieved. In particular, it was revealed that combined use with another synthetic tissue in a specific embodiment (in particular, an embodiment in which a synthetic tissue is smaller in size than a depth of a defect of a bone section in the osteochondral defect) in a preferred embodiment of the present invention significantly enhances post-operational results, dramatically enhances the condition of biological integration, and enables therapy with barely any injury scar.

An extracellular matrix or a cell adhesion molecule, such as fibronectin or vitronectin, is distributed throughout a synthetic tissue used in the composite tissue of the present invention. In contrast, in cell sheet engineering, cell adhesion molecules are localized on a surface of culture cells which is attached to a Petri dish. The most prominent difference is that cells are major components of the sheet in cell sheet engineering, hence a sheet is closer to a mass of cells with glue of an adhesion molecule attached on the bottom surface, whereas the synthetic tissue of the present inventors is literally a "tissue" where an extracellular matrix wraps cells. Thus, the present invention is deemed significantly different from conventional techniques.

A cell sheet engineering technique, led by a group from Tokyo Women's Medical University, utilizing a temperature sensitive culture dish is a typical cell implanting method without a scaffold. Such a cell sheet engineering technique is internationally acclaimed due to its originality. However, a single sheet obtained by this technique is often fragile. Thus, when using this cell sheet technique, it was necessary to stack multiple sheets in order to obtain strength that can withstand surgical manipulation, such as implantation. However, such a problem is solved by the present invention. Although it is not desired to be constrained by theory, a synthetic tissue can be freely adjusted with respect to the three-dimensional thickness, width and the like. In addition, efficient regeneration of a single tissue such as a cartilage is possible. However, regeneration of a composite tissue, such as a bone/cartilage complex, by using only a synthetic tissue was in fact inefficient in terms of securing the number of cells. Efficient regeneration is enabled by formation of a composite tissue in the present invention.

A cell/matrix complex developed by the present study does not require a temperature sensitive culture dish unlike the cell sheet technique. Further, a cell/matrix complex can be readily formed into a multi-layer tissue. There is no technique in the world other than the present invention, which can produce a multi-layer complex having 10 or more layers without using so-called feeder cells, such as rodent stroma cells, in about three weeks. By adjusting conditions for matrix synthesis of synovial cell, it is possible to produce a complex having a strength which allows surgical manipulation, such as holding or transferring of a complex, without a special instrument. Therefore, the present invention has an effect that is an original, groundbreaking technique in the world for reliable and safe cell implantation. The synthetic tissue used in the present invention can be freely adjusted with respect to the three-dimensional thickness, width and the like, and efficient regeneration of a single tissue such as a cartilage is possible. However, regeneration of a composite tissue, such as a bone/cartilage complex, by using only a synthetic tissue was in fact inefficient. Efficient regeneration is made possible by formation of a composite tissue in the present invention.

### [Brief Description of Drawings]

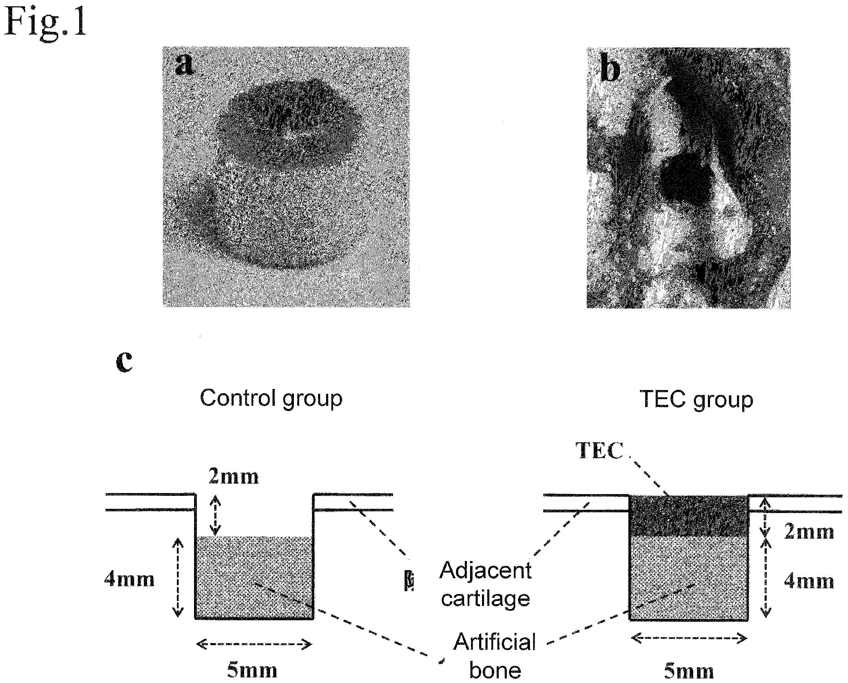

[Figure 1] Figure **1a** shows a hybrid graft consisting of a TEC and an artificial bone. Figure **1b** shows an osteochondral defect in a fossa of a femur of a rabbit. Figure **1c** shows a schematic diagram of an implant material in a control group and a TEC group.

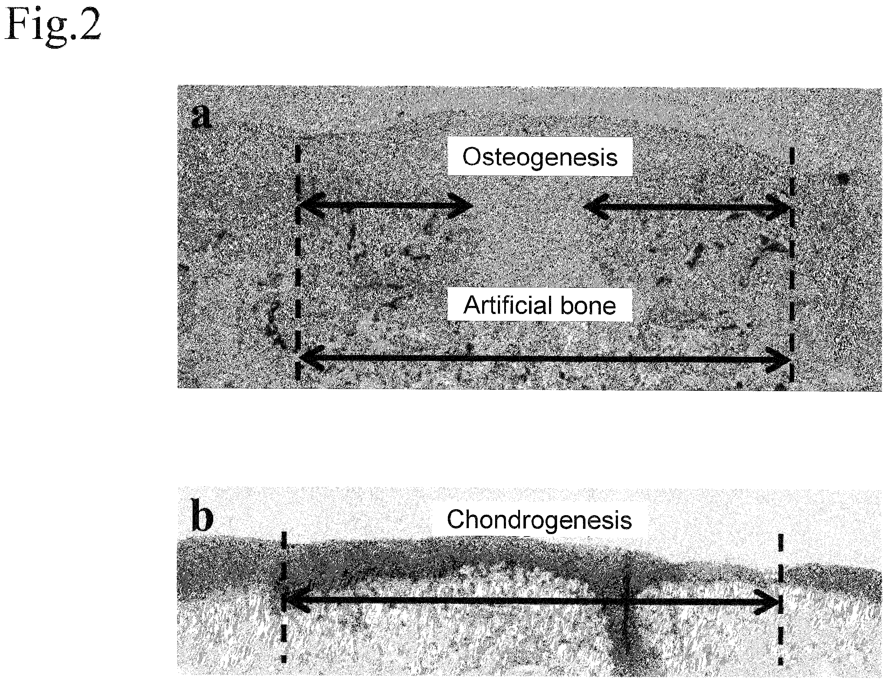

[Figure 2] Figure **2** shows quantification of repair of a subchondral bone and cartilage. (a) Bone formation ratios were calculated by dividing the length of a bone tissue after repair by the length of an artificial bone and the results are represented as a percentage. (b) Cartilage formation ratios were also calculated by the same method.

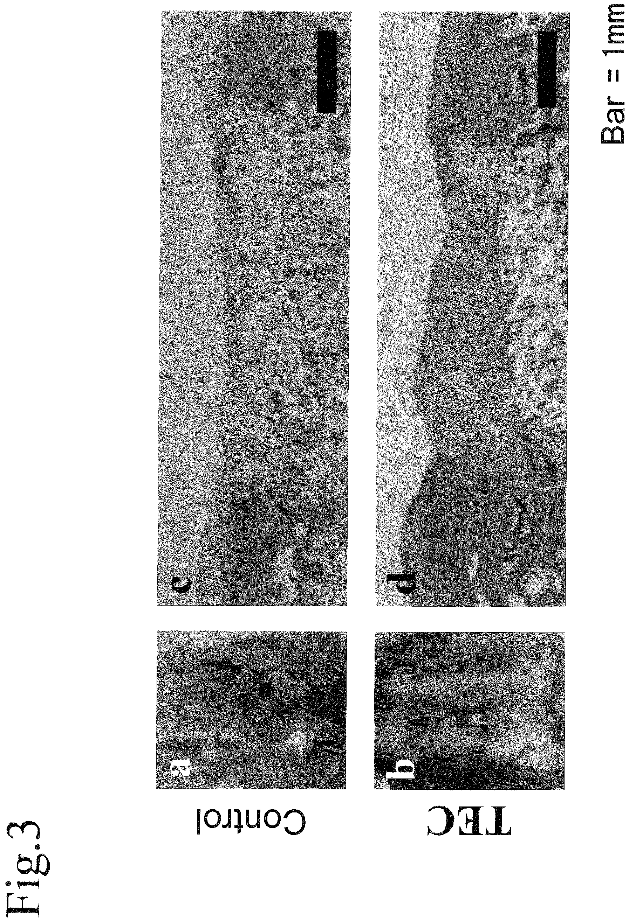

[Figure 3] Figures **3a****-b** show images seen by the naked eyes of a repair tissue after one month from a surgical operation using (a) only an artificial bone or (b) a hybrid graft. Figures **3c****-d** show H&E staining of a tissue after repair treated with (c) only an artificial bone or (d) a hybrid graft. A osteochondral defect that was treated with a hybrid graft was repaired with a thick, fiber-like tissue. Bar = 1mm

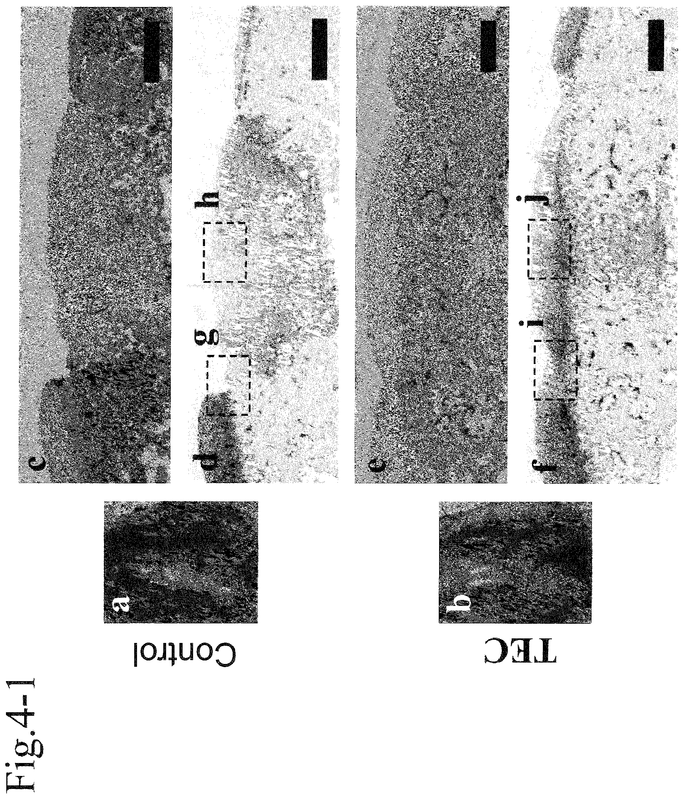

[Figure 4-1] Figures **4a-b** show images seen by the naked eyes of a repaired tissue after two months from a surgical operation, which is treated with (a) only an artificial bone or (b) a hybrid graft. Figures **4c-f** show H&E staining and toluidine blue staining of a tissue after repair, which is treated with (c, d) only an artificial bone or (e, f) a hybrid graft. Bar = 1mm.

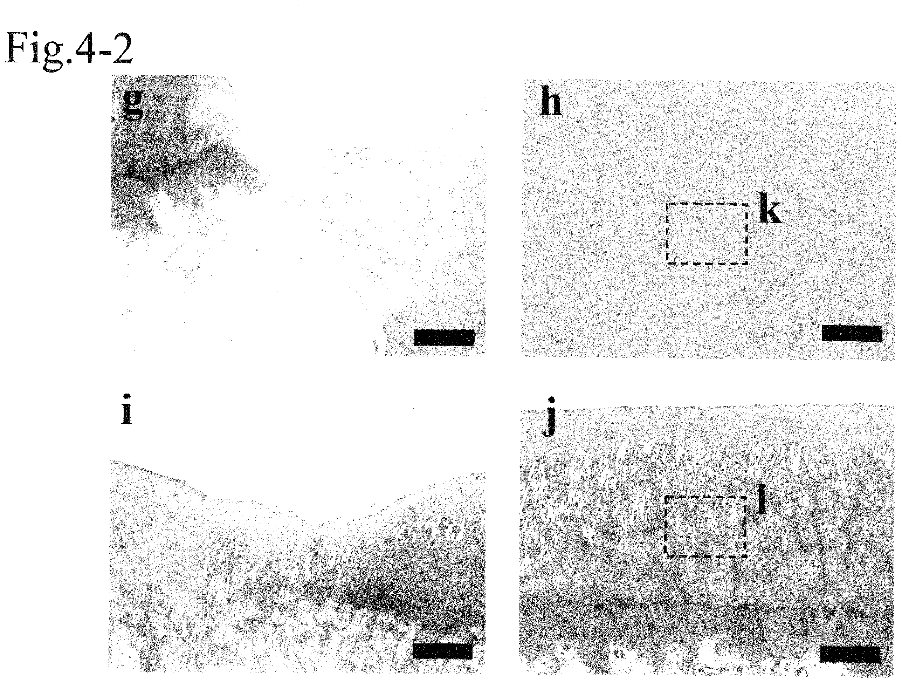

[Figure 4-2] Figures **4g-j** show low magnification images of peripheral (g, i) and central (h, j) regions of a repaired tissue. Bar = 1 µm. A defect treated with a hybrid graft was repaired with an osteochondral tissue and demonstrated excellent tissue integration with an adjacent receiving tissue.

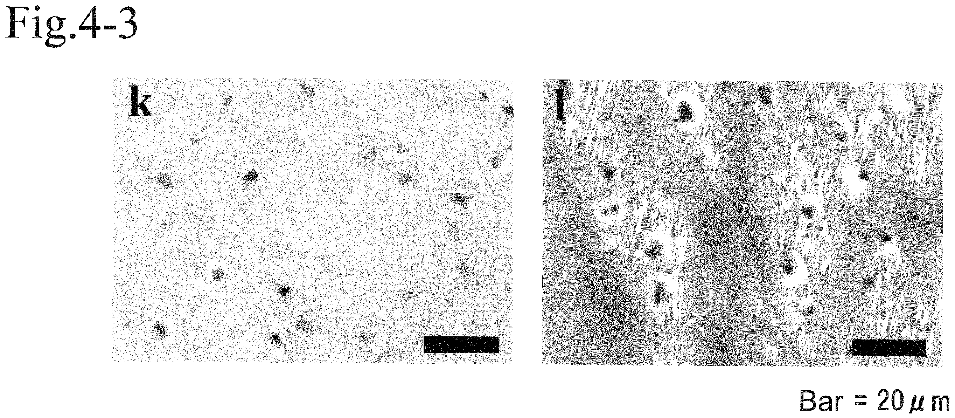

[Figure 4-3] Figures **4k-l** show high magnification images of a central regionof a repaired tissue. Bar = 20 µm The cells treated with a hybrid graft exhibited a round cell form in a small lacuna.

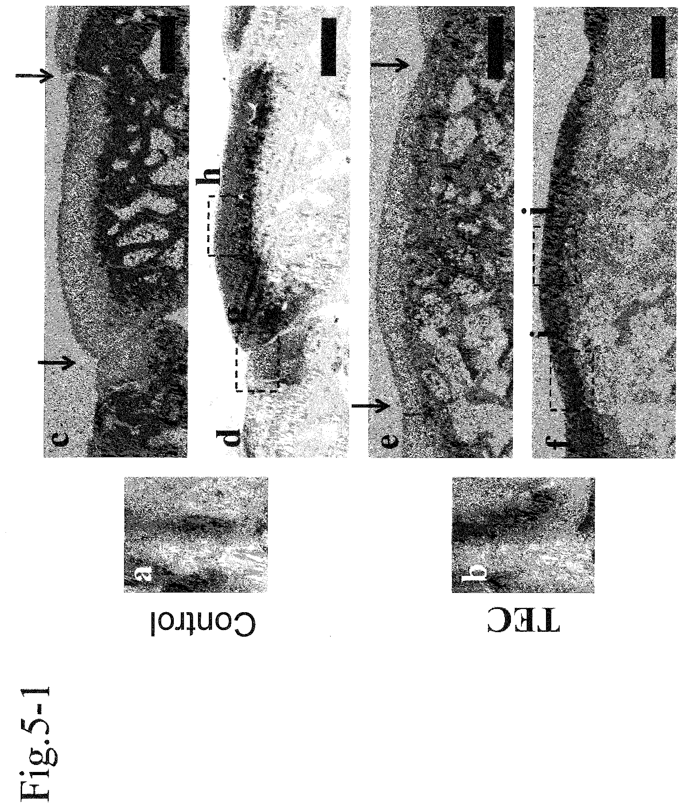

[Figure 5-1] Figures **5a-b** show images seen by the naked eyes of a repair tissue after six months from a surgical operation, which is treated with (a) only an artificial bone or (b) a hybrid graft. Figures **5c-f** show H&E staining and toluidine blue staining of a tissue after repair treated with (c, d) only an artificial bone or (e, f) a hybrid graft. Bar = 1mm

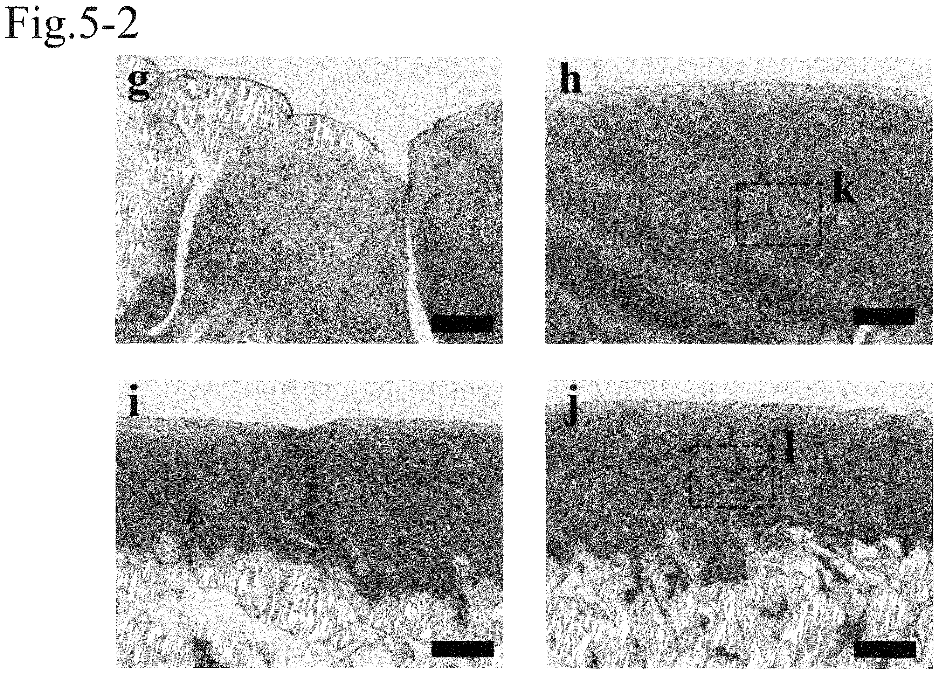

[Figure 5-2] Figures **5g-j** show low magnification images of peripheral (g, i, arrow) and central regions (h, j) of a repaired tissue. Bar = 100 µm. A repaired tissue treated with a hybrid graft maintained excellent biological tissue integration with an adjacent host tissue, but the tissue treated only with an artificial bone exhibited poor biological integration.

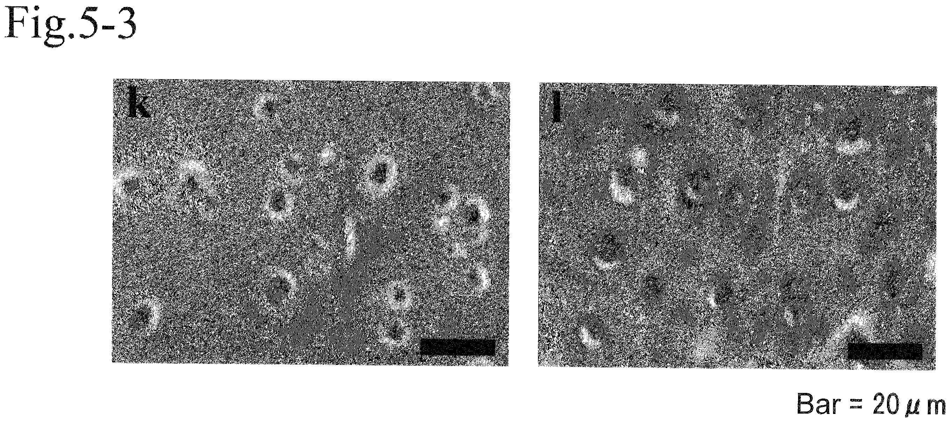

[Figure 5-3] Figures **5k-l** show high magnification images of a central regionof a repaired tissue. Bar = 20 µm. The cells treated with a hybrid graft exhibited a round cell form in a small lacuna. However, cells treated only with an artificial bone exhibited clusters of cells in a lacuna.

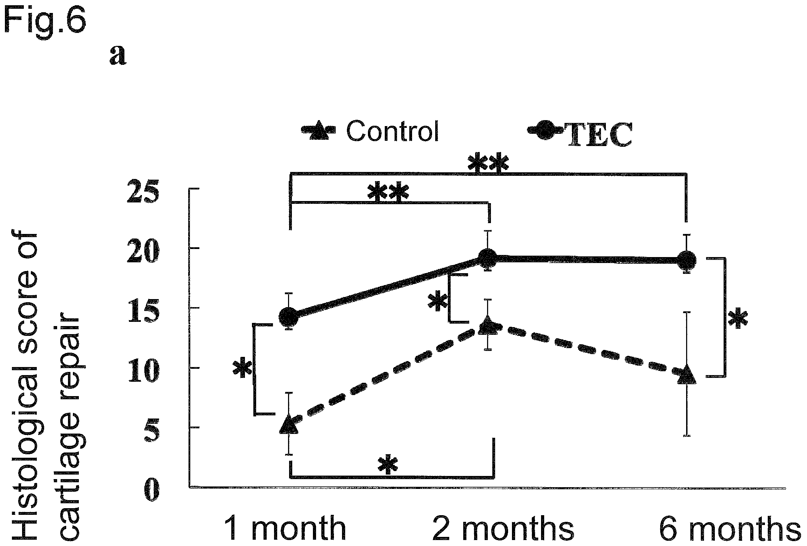

[Figure 6a] Figure **6a** shows histological scores in subchondral bone repair in a control group and a TEC group after one month, 2 months, and 6 months from a surgical operation [N=4, N=6, respectively]. *:p<0.05; **:p<0.01. It is particularly noteworthy that the scores were significantly higher in comparison to the control group up to six months after a surgical operation in cartilage repair when using the present invention.

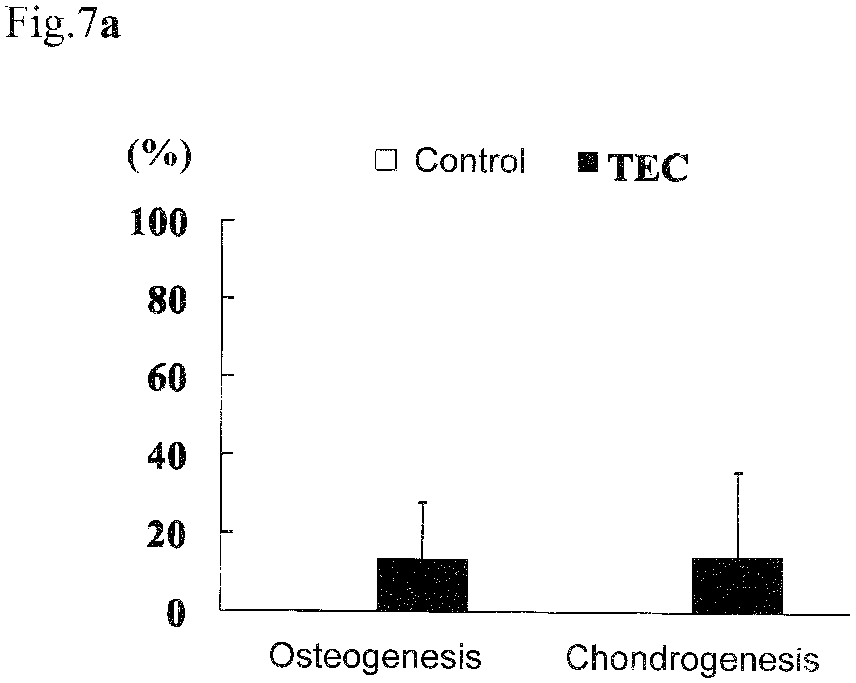

[Figure 7a] Figure **7a** shows formation of bone and cartilage in a control group and a TEC group after one month from a surgical operation [N=4, N=6, respectively].

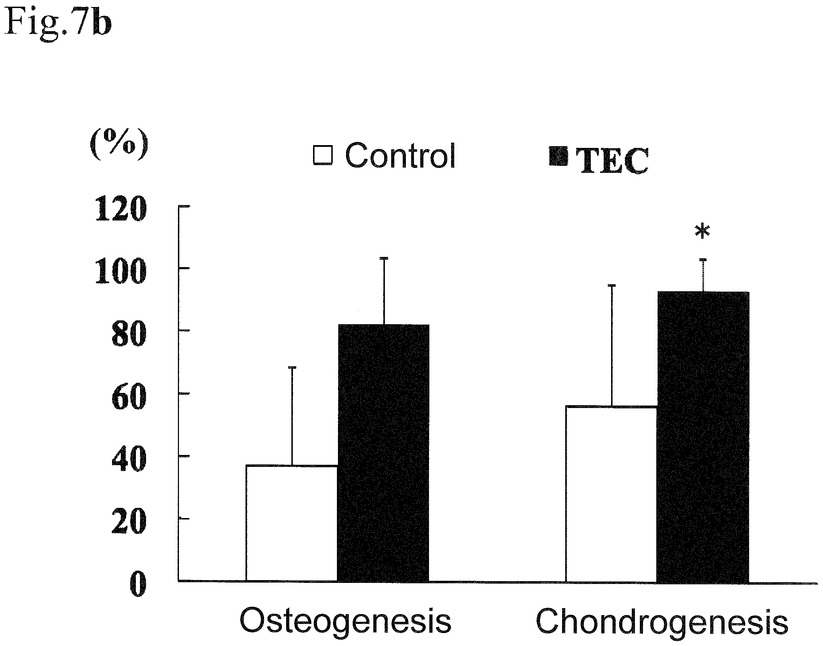

[Figure 7b] Figure **7b** shows formation of bone and cartilage in a control group and a TEC group after two months from a surgical operation [N=4, N=7, respectively]. *:p<0.05. Cartilage formation of the TEC group was significantly higher than that of the control group at two months after the surgical operation.

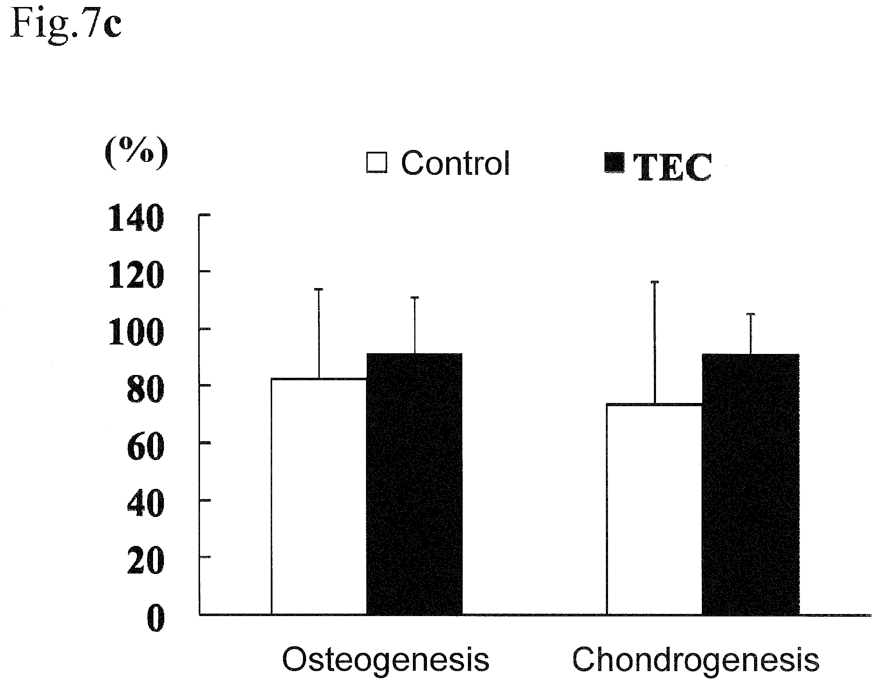

[Figure 7c] Figure **7c** shows formation of bone and cartilage in a control group and a TEC group after six months from a surgical operation [N=5, N=5, respectively].

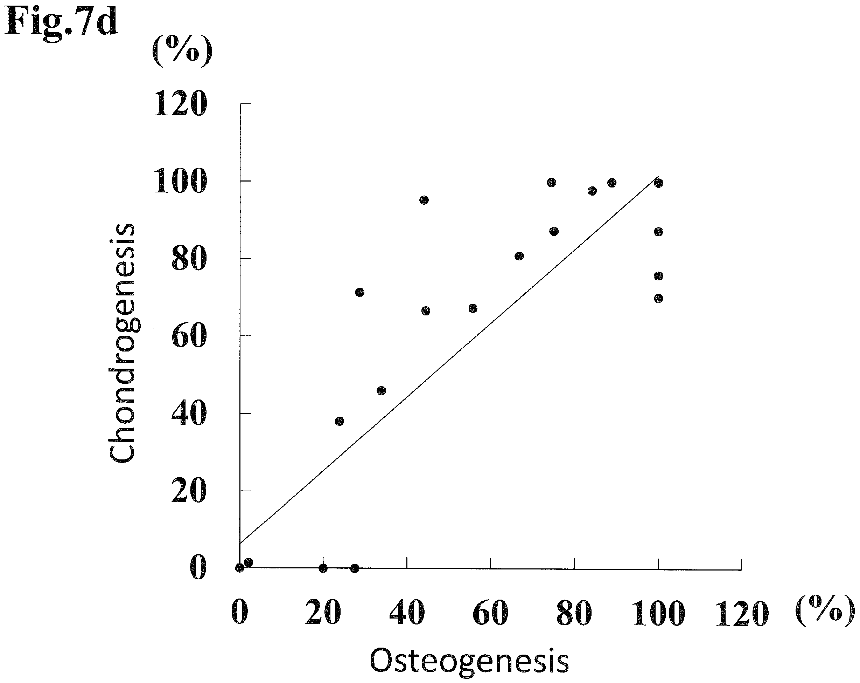

[Figure 7d] Figure **7d** shows that formation ratios of a bone and cartilage was significantly correlated (N = 31, r=0.8872, p<0.001).

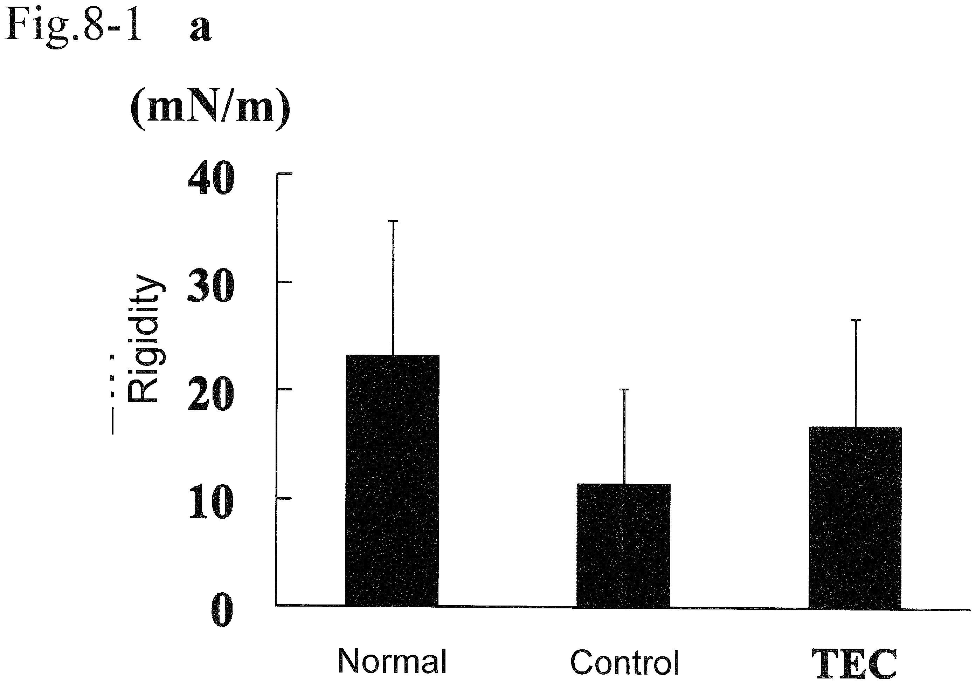

[Figure 8-1] Figure **8a** shows the rigidity of a repaired osteochondral tissue in untreated normal tissues (N=5) , a control group (N=5) , and a TEC group (N=5) . Rigidity equivalent to that in normal osteochondral tissues was restored in the repaired osteochondral tissue treated with a hybrid graft.

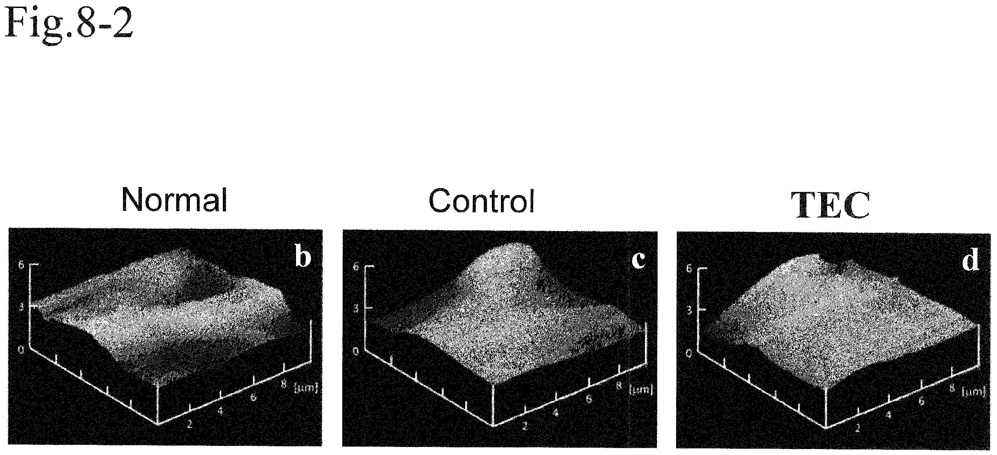

[Figure 8-2] Figures **8b-d** show digital images of repaired tissues in an untreated normal tissue (b), control group (c) and TEC group (d)

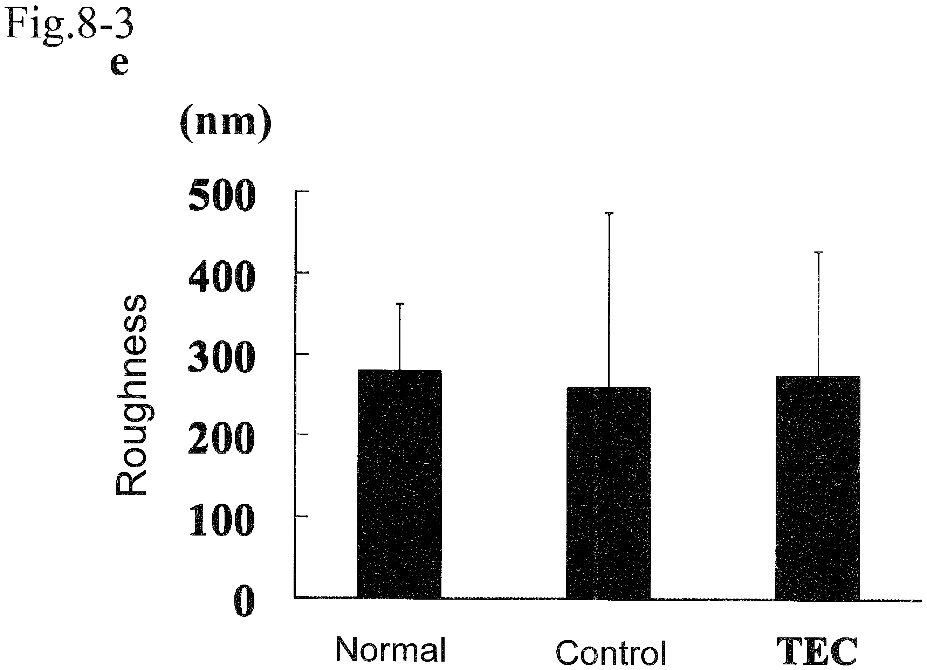

[Figure 8-3] Figure **8e** shows surface roughness calculated from the digital images of Figures **8b-d.** There was no significant difference among untreated normal tissue group (N=5), control group, and TEC group (N=3).

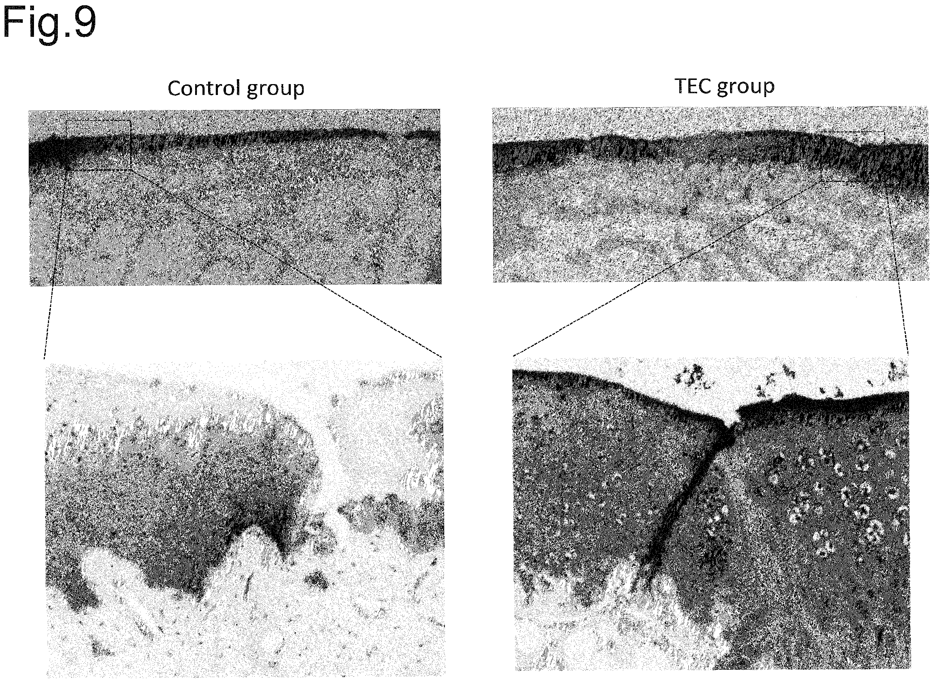

[Figure 9] Figure **9** is a diagram showing the results of Example 2 (toluidine blue staining) . The control group is shown in the left and the result using the TEC composite tissue of the present invention is shown in the right. Expanded views of the square portions on the top are shown below. As in NEOBONE, biological integration with an adjacent normal cartilage six months after an operation was not good in the control group. Further, the cartilage had been progressively thinning. On the other hand, the hybrid group had excellent biological integration with an adjacent normal cartilage.

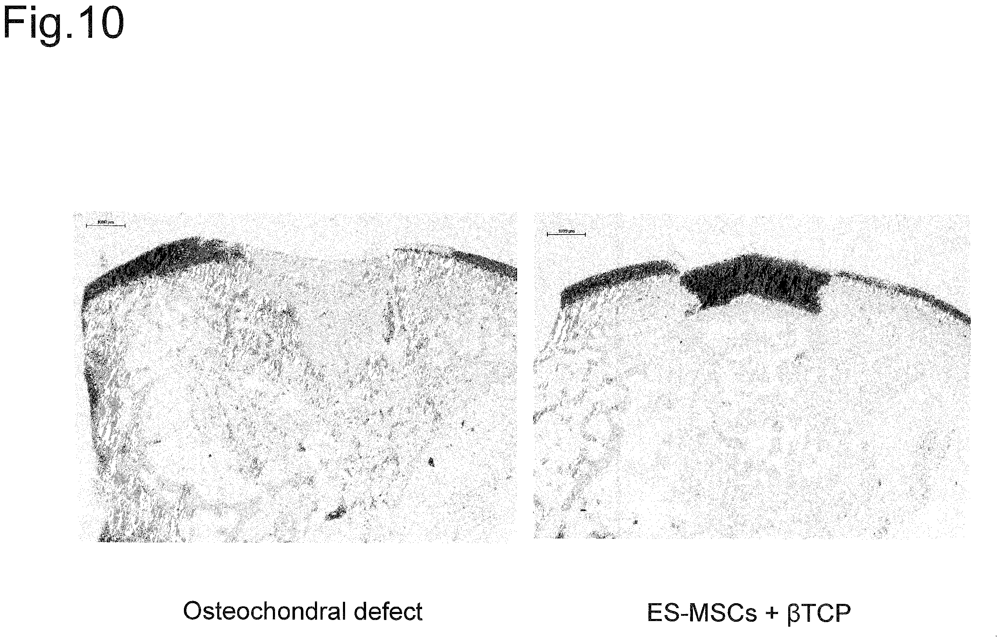

[Figure 10] Figure **10** demonstrates that the effects of the present invention can be obtained even when a mesenchymal stem cell, which is also called mesenchyme-like stem cell, is induced from rabbit ES cells to make a three-dimensional synthetic tissue therewith as in Example 7. The state of osteochondral defect is shown in the left and the state of healing one month after an operation with a composite tissue of βTCP and a TEC made with a mesenchymal stem cell, which is also called mesenchyme-like stem cell, from rabbit ES cells of the present invention is shown in the right. An inner cell mass was collected and cultured on a feeder cell (MEF) to induce ESCs. Next, an embryoid body (EB) was made and induced to differentiate into MSCs (ES-MSCs) in plate culture under controlled oxygen partial pressure for use. An integrated implant of a TEC made with ES-MSCs and an artificial bone with φ 5mm × height 4 mm was implanted in a φ 5mm × height 6 mm osteochondral defect in a rabbit knee joint. Obvious cartilage repair due to a TEC/artificial bone hybrid implant was observed in comparison to a knee with only a defect.

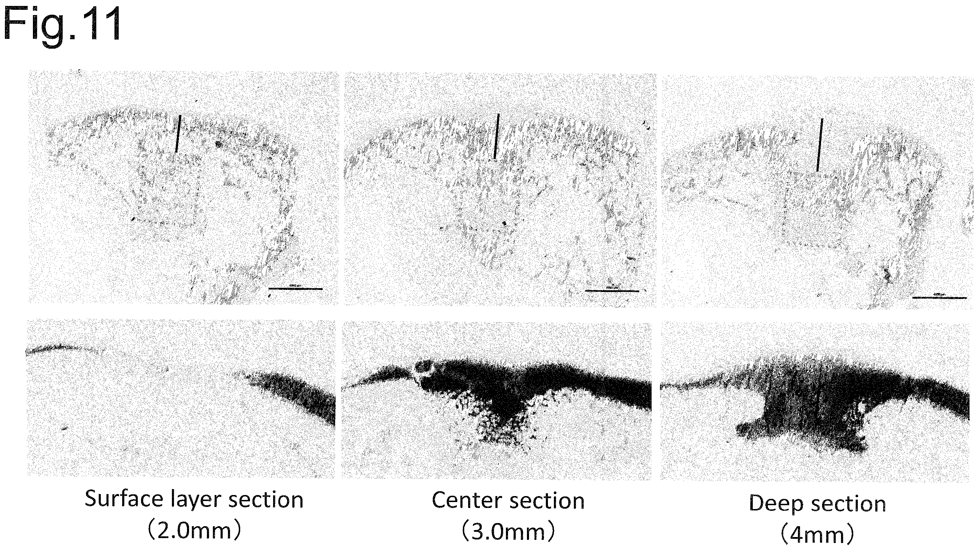

[Figure 11] Figure **11** shows the state after one month from implantation of a three dimensional synthetic tissue (TEC)/artificial bone complex (Example 5). Implantation that is 2.0 mm from the surface layer is shown in the left. Implantation that is 3.0 mm from the surface layer is shown in the middle. Implantation that is 4 mm from the surface layer is shown in the right. The top row shows repair of a subchondral bone section with hematoxylin-eosin staining and the bottom row shows repair of cartilage section with toluidine blue staining. As shown, regeneration differs depending on the depth of implantation of a complex. When shallow, a subchondral bone is repaired quickly, but a cartilage is poorly repaired. When deep, the cartilage is repaired well, but the repair of the subchondral bone is prolonged.

### [Description of Embodiments]

The present invention is described below. Throughout the entire specification, a singular expression shouldbe understood as encompassing the concept thereof in a plural form unless specifically noted otherwise. Thus, singular modifiers such as articles (e.g., "a", "an", "the" and the like in case of English) should be understood as encompassing the concept thereof in a plural form unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the terms commonly understood by those skilled in the art to which the present invention belongs. In case of a contradiction, the present specification (including the definitions) takes precedence.

### (Definition of terms)

The definitions of specific terms used herein are described below.

### (Regenerative medicine)

As used herein, the term "regeneration" refers to a phenomenon in which when an individual organism loses a portion of tissue, the remaining tissue grows and recovers. The extent and manner of regeneration vary depending on animal species or tissues in the same individual. Most human tissues have limited regeneration capability, and complete regeneration is not expected if a large portion of tissue is lost. In the case of severe damage, a tissue with strong proliferation capability different from that of a lost tissue may grow, resulting in incomplete regeneration where the damaged tissue is incompletely regenerated and the function of the tissue cannot be recovered. In this case, regenerative medicine is administered, wherein a structure made of a bioabsorbable material is used to prevent a tissue with strong proliferation capability from infiltrating the defect portion of the tissue so as to secure a space for proliferation of the damaged tissue, and a cell growth factor is supplemented to enhance the regeneration capability of the damaged tissue. Such a regeneration therapy is applied to cartilages, bones, hearts, and peripheral nerves, for example. It was believed until now that cartilages, nerve cells, and cardiac muscles have no or poor regeneration capability. There are reports of the presence of tissues (somatic stem cells), which have both the capability of differentiating into these tissues and self-proliferation capability. Some are about to be used in practice. Expectations are running high for regenerative medicine using tissue stem cells. Embryonic stem cells (ES cells) have the capability of differentiating into all tissues. Induced pluripotent stem (iPS) cells are stem cells that have the ability to differentiate into all tissues. IPS cells can be produced without the use of an embryo or a fetus. Somatic stem cells can be made from pluripotent cells such as ES cells and iPS cells (As references, see de Peppo et al., TISSUE ENGINEERING: Part A, 2010; 16; 3413-3426; Toh et al., Stem Cell Rev. and Rep., 2011; 7:544-559; Varga et al., Biochem.Biophys.Res.Commun., 2011; doi:10.1016/j.bbrc.2011.09.089; Barbet et al., Stem Cells International, 2011, doi:10.4061/2011/368192; Sanchez et al., STEM CELLS, 2011; 29:251-262; Simpsonetal., Biotechnol.Bioeng., 2011; doi:10.1002/bit.23301; Jung et al., STEM CELLS, 2011; doi:10.1002/stem.727).

As used herein, the term "cell" is defined in its broadest sense in the art, referring to a structural unit of a tissue of a multicellular organism or a lift form, which is surrounded by a membrane structure for separating the living body from the external environment, has self-regeneration capability inside, and has genetic information and a mechanism for expressing the information. In the method of the present invention, any cell can be used as a subject. The number of "cells" used in the present invention can be counted through an optical microscope. When counting with an optical microscope, counting is performed by counting the number of nuclei . For example, the tissues are sliced into tissue segments, which are then stained with hematoxylin-eosin (HE) to variegate nuclei derived from extracellular matrices (e.g., elastin or collagen) and cells with dye. These tissue segments can be observed under an optical microscope to count the number of cells by estimating the number of nuclei in a particular area (e.g., 200 µm × 200 µm) to be the number of cells. Cells used herein may be either naturally-occurring cells or artificially modified cells (e.g., fusion cells, genetically modified cells, etc.). Examples of a cell source include, a single-cell culture; the embryo, blood, or a body tissue of a normally-grown transgenic animal; and a cell mixture such as cells derived from normally-grown cell lines . Primary culture cells may be used as the cells. Alternatively, subculture cells may also be used. As used herein, cell density may be represented by the number of cells per unit area (e.g., cm²).

As used herein, the term "stem cell" refers to a cell that has self-replication capability and pluripotency. Typically, stem cells can regenerate a tissue when the tissue is injured. Stem cells used herein may be ES cells, iPS cells or tissue stem cells (also called tissular stem cell, tissue-specific stem cell, or somatic stem cell) . A stem cell may be an artificially produced cell as long as it can have the above-described capabilities. ES cells are pluripotent or totipotent stem cells derived from early embryos. An embryonic stem cell was first established in 1981, and has been applied to production of knockout mice since 1989. In 1998, a human ES cell was established, which is currently becoming available for regenerative medicine. Tissue stem cells have a relatively limited level of differentiation unlike ES cells. Tissue stem cells are present in specific location of tissues and have an undifferentiated intracellular structure. Thus, the level of pluripotency of tissue stem cells is low. Tissue stem cells have a higher nucleus/cytoplasm ratio and have few intracellular organelles. Most tissue stem cells have pluripotency, a long cell cycle, and proliferative ability maintained beyond the life of an individual. As used herein, stem cells may be preferably ES cells, but tissue stem cells may also be employed depending on the circumstance. Recently, iPS cells have also drawn attention. IPS cells also can be made by induction (initialization) using the so-called Yamanaka factor or the like from skin cells or the like. Induction from iPS cells into a mesenchymal stem cell, which is also called mesenchyme-like stem cell, can be carried out by referring to Jung et al, STEM CELLS, 2011; doi:10.1002/stem.727. Further, induction from ES cells into a mesenchymal stem cell, which is also called mesenchyme-like stem cell, can be carried out by referring to, for example, de Peppo et al., TISSUE ENGINEERING: Part A, 2010; 16; 3413-3426; Toh et al., Stem Cell Rev. and Rep., 2011; 7:544-559; Varga et al., Biochem.Biophys.Res.Commun., 2011; doi:10.1016/j.bbrc.2011.09.089; Barbet et al., Stem Cells International, 2011, doi:10.4061/2011/368192; Sanchez et al., STEM CELLS, 2011; 29:251-262; Simpsonetal., Biotechnol.Bioeng., 2011; doi:10.1002/bit.23301.

Historically, tissue stem cells are separated into categories of sites from which the cells are derived, such as the dermal system, the digestive system, the bone marrow system, and the nervous system. Tissue stem cells in the dermal system include epidermal stem cells, and hair follicle stem cells. Tissue stem cells in the digestive system include pancreatic (common) stem cells, and hepatic stem cells. Tissue stem cells in the bone marrow system include hematopoietic stem cells, mesenchymal stem cells (e.g., derived from fat or bone marrow), and the like. Tissue stem cells in the nervous system include neural stem cells, retinal stem cells, and the like. It is now possible to produce these tissue stem cells by differentiation from ES cells, iPS cells or the like. Thus, such classification by origin has recently been redefined in terms of differentiation capability of the stem cell as an index. Herein, stem cells having the same differentiation capability as a specific tissue stem cell (e.g., mesenchymal stem cell) are understood to be the same, regardless of whether the original is an ES cell, iPS cell or the like with differentiation capability of each stem cell as the index, because such cells are capable of achieving the objective of the present invention.

As used herein, the term "somatic cell" refers to any cell other than a germ cell, such as an egg or a sperm, which does not directly transfer its DNA to the next generation. Typically, somatic cells have limited or no pluripotency. Somatic cells used herein may be naturally-occurring or genetically modified.

Cells can be classified by the origin thereof into stem cells derived by the ectoderm, endoderm, or mesoderm. Cells of ectodermal origin, including neural stem cells, are mostly present in the brain. Cells of endodermal origin, including blood vessel stem cells, hematopoietic stem cells, and mesenchymal stem cells, are mostly present in bone marrow. Cells of mesoderm origin, including hepatic stem cells and pancreatic stem cells, are mostly present in organs. As used herein, somatic cells may be derived from any mesenchyme. As somatic cells, mesenchymal cell is preferably used, and cells including mesenchymal stem cell are more preferably used. Such mesenchymal stem cell can be made from a less differentiated stem cell such as ES cell or iPS cell. Thus, when used herein, "mesenchymal stem cell; MSC" refers to a somatic stem cell with the ability to differentiate into a mesenchymal cell. The differentiation capability includes differentiation into mesenchymal tissue such as bone, cartilage, blood vessel, and myocardium. Mesenchymal stem cells are applied to regenerative medicine such as reconstruction of such tissues. Representative mesenchymal stem cells include somatic stem cells from mesenchyme (e.g., marrow mesenchymal stem cells included in marrow stromal cells, mesenchymal stem cells included in synovial cells).

As used herein, the term "mesenchymal stem cell" refers to a stem cell found in mesenchyme. Mesenchyme refers to a population of free cells which have an asterodal-shaped or irregular projections and bridge gaps between epithelial tissues and which are recognized in each stage of development of multicellular animals. Mesenchyme also refers to a tissue formed with intracellular cement associated with the cells. Mesenchymal stem cells have proliferation capability and the capability to differentiate into osteocytes, chondrocytes, muscle cells, stroma cells, tendon cells, and adipocytes. Mesenchymal stem cells are employed in order to culture or grow bone marrow cells or the like collected from patients or to allow differentiation into chondrocytes or osteoblasts. Mesenchymal stem cells are also employed as reconstruction materials for alveolar bones; bones, cartilages or joints for arthropathy. There is a large demand for mesenchymal stem cells. Thus, the composite tissue comprising mesenchymal stem cells or differentiated mesenchymal stem cells of the present invention is particularly useful when a structure is required in these applications.

For example, differentiated cell or stem cell derived from the ectoderm, endoderm, or mesoderm described above can be used as a cell included in a three-dimensional construct constituting the composite tissue of the present invention. Such a cell includes mesenchymal cells. In a certain Embodiment, examples of such a cell that can be used include myoblasts (e.g., skeletal myoblasts), fibroblasts, synovial cells or the like. As such a cell, it is possible to directly use separated cells including stem cells and differentiated cells, directly use differentiated cells, or directly use stem cells. However, it is possible to use cells that are differentiated toward a desired direction from stem cells.

As used herein, the term "isolated" means that substances that are naturally accompanied in a normal circumstance are at least reduced, or preferably substantially eliminated. Therefore, an isolated cell, tissue or the like refers to a cell that is substantially free of other accompanying substances (e.g., other cells, proteins, nucleic acids, etc.) in normal circumstances. For tissues, isolated tissue refers to a tissue substantially free of substances other than that tissue (e.g., in the case of synthetic tissues or complexes, substances, scaffolds, sheets, coating, or the like that is used when the synthetic tissue is produced). As used herein, the term "isolated"preferably refers to a scaffold-free state. Therefore, it is understood that the synthetic tissue or complex of the present invention in an isolated state may contain components such as a medium used in the production thereof. The term "isolated" in relation to nucleic acids or polypeptides means that, for example, the nucleic acids or the polypeptides are substantially free of cellular substances or culture media when produced by recombinant DNA techniques or substantially free of precursory chemical substances or other chemical substances when chemically synthesized. Isolated nucleic acids are preferably free of sequences naturally flanking the nucleic acid within an organism from which the nucleic acid is derived (i.e., sequences positioned at the 5' terminus and the 3' terminus of the nucleic acid).

As used herein, the term "scaffold-free" indicates that a synthetic tissue is substantially free of a material (scaffold) which is conventionally used for production of a synthetic tissue. Examples of such a scaffold material include chemical polymeric compounds, ceramics, or biological formulations such as polysaccharides, collagens, gelatins, and hyaluronic acids. A scaffold is a material which is substantially solid and has strength which allows it to support cells or tissues.

As used herein, the term "established" in relation to cells refers to a state of a cell in which a particular property (e.g., pluripotency) is maintained and the cell undergoes stable proliferation under culture conditions. Therefore, established stem cells maintain pluripotency.

As used herein, the term "non-embryonic" refers to not being directly derived from early embryos. Therefore, the term "non-embryonic" refers to cells derived from parts of the body other than early embryos. Modified embryonic stem cells (e.g., genetically modified or fusion embryonic stem cells) are encompassed by non-embryonic cells.

As used herein, the term "differentiated cell" refers to a cell having a specialized function and form (e.g., muscle cells and neurons) . Unlike stem cells, differentiated cells have no or little pluripotency. Examples of differentiated cells include epidermic cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, adipocytes, osteocytes, and chondrocytes.

As used herein, the term "tissue" refers to a group of cells having the same function and form in cellular organisms. In multicellular organisms, constituent cells usually differentiate so that the cells have specialized functions, resulting in division of labor. Therefore, multicellular organisms are not simple cell aggregations, but instead constitute organic or social cell groups having a certain function and structure. Examples of tissues include integument tissue, connective tissue, muscular tissue, and nervous tissue. Tissues targeted by the present invention may be derived from any organ or part of an organism. In a preferable embodiment of the present invention, tissues targeted by the present invention include a bone, a cartilage, a tendon, a ligament, a meniscus, an intervertebral disk, a periosteum, and a dura mater.

As used herein, the term "cell sheet" refers to a structure made of a monolayer of cells. Such a cell sheet has at least two-dimensional biological integration. A sheet having biological integration is characterized in that after the sheet is produced, the connection between cells is not substantially destroyed even when the sheet is handled individually. Such biological integration includes intracellular integration via an extracellular matrix . Such a cell sheet may partially include a two- or three-layer structure.

As usedherein, the term "synthetic tissue" refers to a tissue in a state that is different from natural states. Typically, a synthetic tissue is herein prepared by cell culture. A tissue which is directly removed in an existing form from an organism is not referred to as a synthetic tissue. Therefore, a synthetic tissue may include materials derived from organisms and materials not derived from organisms. The synthetic tissue of the present invention typically is made of a cell and/or a biological material, and may comprise other materials . More preferably, the synthetic tissue of the present invention is substantially made of only of a cell and/or a biological material. Such a biological material is preferably a substance derived from cells constituting the tissue (e.g., extracellular matrix).

As used herein, the term "implantable synthetic tissue" refers to a synthetic tissue, which can be used for actual clinical implantation and can function as a tissue at an implantation site for at least a certain period of time after implantation. Implantable synthetic tissues typically have sufficient biocompatibility, sufficient affinity, and the like.

The sufficient strength of an implantable synthetic tissue varies depending on a part targeted by implantation. However, the strength can be appropriately determined by those skilled in the art. The strength is sufficient to provide self-supporting ability, and can be determined depending on the environment of implantation. Such strength can be measured by measuring stress or distortion characteristics or by conducting a creep characteristics indentation test as described below. The strength may also be evaluated by observing the maximum load.

The sufficient size of an implantable synthetic tissue varies depending on a part targeted by implantation. However, the size can be appropriately determined by those skilled in the art. The size can be determined depending on the environment of implantation.

However, an implantable synthetic tissue preferably has at least a certain size. Such a size, in terms of area, is at least 1 cm², preferably at least 2 cm², more preferably at least 3 cm², even more preferably at least 4 cm², at least 5 cm², at least 6 cm², at least 7 cm², at least 8 cm², at least 9 cm², at least 10 cm², at least 15 cm², or at least 20 cm². The area can be 1 cm² or less or 20 cm² or greater depending on the application. The essence of the present invention is understood such that a synthetic tissue of any size (area, volume) can be produced, i.e., the size is not particularly limited.

When the size is represented by volume, the size may be at least 2 mm³ or at least 40 mm³. It is understood that the size may be 2 mm³ or less or 40 mm³ or greater.

The sufficient thickness of an implantable synthetic tissue varies depending on a part targeted by implantation. However, the thickness can be appropriately determined by those skilled in the art. The thickness can be determined depending on the environment of implantation. The thickness may exceed 5 mm. For example, when an implantable synthetic tissue is applied to a bone, a cartilage, a ligament, a tendon, or the like, the tissue generally has a thickness of at least about 1 mm, e.g., at least about 2 mm, more preferably at least about 3 mm, at least about 4 mm, and even more preferably about 5 mm, or about 5 mm or greater or about 1 mm or less. The essence of the present invention is understood such that a tissue or complex of any thickness can be produced, i.e., the size is not particularly limited.

The sufficient biocompatibility of an implantable synthetic tissue varies depending on a part targeted by implantation. However, the degree of biocompatibility can be appropriately determined by those skilled in the art. Typically, a desired level of biocompatibility is, for example, such that biological integration to surrounding tissues is achieved without any inflammation or any immune reaction. In some cases (e.g., corneas, etc.), an immune reaction is less likely to occur. Therefore, an implantable synthetic tissue has biocompatibility for the object of the present invention even when an immune reaction is likely to occur in other organs. Examples of parameters indicating biocompatibility include the presence or absence of an extracellular matrix, the presence or absence of an immune reaction, and the degree of inflammation. Such biocompatibility can be determined by examining the compatibility of a synthetic tissue at an implantation site after implantation (e.g., confirming that an implanted synthetic tissue is not destroyed) (See "Hito Ishoku Zoki Kyozetsu Hanno no Byori Soshiki Shindan Kijyun Kanbetsu Shindan to Seiken Hyohon no Toriatsukai (Zufu) Jinzo Ishoku, Kanzo Ishoku Oyobi Shinzo Ishoku [Pathological Tissue Diagnosis Criterion for Human Transplanted Organ Rejection Reaction Handling of Differential Diagnosis and Biopsy Specimen (Illustrated Book) Kidney Transplantation, Liver Transplantation and Heart Transplantation]" The Japan Society for Transplantation and The Japanese Society for Pathology editors, Kanehara Shuppan Kabushiki Kaisha (1998)) . According to this document, biocompatibility is divided into Grade 0, 1A, 1B, 2, 3A, 3B, and 4. At Grade 0 (no acute rejection), no acute rejection reaction, cardiomyocyte failure, or the like is found in biopsy specimens. At Grade 1A (focal, mild acute rejection), there is focal infiltration of large lymphocytes around blood vessels or into interstitial tissue, while there is no damage to cardiomyocytes. This observation is obtained in one or a plurality of biopsy specimens. At Grade 1B (diffuse, mild acute rejection), there is diffuse infiltration of large lymphocytes around blood vessels or into interstitial tissue or both, while there is no damage to cardiomyocytes . At Grade 2 (focal, moderate acute rejection), there is a single observed infiltration focus of inflammatory cells clearly bordered from the surrounding portions. Inflammation cells are large activated lymphocytes and may include eosinophils . Damage to cardiomyocytes associated with modification of cardiac architecture is observed in lesions. At Grade 3A (multifocal, moderate acute rejection), there are multiple infiltration foci of inflammatory cells which are large activated lymphocytes and may include eosinophils. Two or more of the multiple inflammatory infiltration foci of inflammatory cells have damages to cardiomyocytes. In some cases, there is also rough infiltration of inflammatory cells into the endocardium. The infiltration foci are observed in one or a plurality of biopsy specimens. At Grade 3B (multifocal, borderline severe acute rejection), there are more confluent and diffuse infiltration foci of inflammatory cells found in more biopsy specimens than those observed at Grade 3A. There is infiltration of inflammatory cells including large lymphocytes and eosinophils, in some cases neutrophils, as well as damage to cardiomyocytes. There is no hemorrhage. At Grade 4 (severe acute rejection), there is diffuse infiltration of various inflammatory cells including activated lymphocytes, eosinophils, and neutrophils. There is always damage to cardiomyocytes and necrosis of cardiomyocytes. Edema, hemorrhage, and/or angitis are also typically observed. Infiltration of inflammatory cells into the endocardium, which is different from the "Quilty" effect, is typically observed. When a strong therapy is conducted using an immunosuppressant for a considerably long period of time, edema and hemorrhage may be more significant than cell infiltration.

Sufficient affinity of an implantable synthetic tissue varies depending on a part targeted by implantation. However, the degree of affinity can be appropriately determined by those skilled in the art. Examples of parameters for affinity include biological integration capability between an implanted synthetic tissue and its implantation site. Such affinity can be determined based on the presence of biological integration at an implantation site after implantation. Preferable affinity herein includes an implanted synthetic tissue having the same function as that of a site in which the tissue is implanted.

As used herein, the term "self-supporting ability" refers to a property of a synthetic tissue (e.g., a synthetic tissue), by which the synthetic tissue is not substantially destroyed when it is restrained on at least one point thereof. Self-supporting ability is herein observed if a tissue (e.g., a synthetic tissue) is picked up by using forceps with a tip having a thickness of 0.5 to 3.0 mm (preferably, tissue is picked up by using forceps with a tip having a thickness of 1 to 2 mm or 1 mm; the forceps preferably have a bent tip) and the tissue is not substantially destroyed. Such forceps are commercially available (e.g., from Natsume Seisakusho) . A force exerted for picking up a tissue is comparable to a force typically exerted by a medical practitioner handing a tissue. Therefore, the self-supporting ability can also be represented by a property, by which the tissue is not destroyed when it is picked up by hand. Examples of such forceps include a pair of curved fine forceps (e.g., No. A-11 (tip: 1.0 mm in thickness) and No . A-12-2 (tip: 0.5 mm in thickness) commercially available from Natsume Seisakusho) . A bent tip is more suitable for picking up a synthetic tissue. However, the forceps are not limited to a bent tip type.

For example, when a joint is treated, replacement is mainly performed. The strength of a synthetic tissue of the present invention required in such a case is sufficient at the minimum self-supporting ability described above. Cells contained in the synthetic tissue are subsequently replaced with cells in an affected portion. The replacing cells produce a matrix to enhance the mechanical strength, so that healing progresses. It is understood that the present invention may be used in conjunction with an artificial joint.

In the present invention, self-supporting ability plays an important role in evaluating the supporting ability of a synthetic tissue when actually produced. When a synthetic tissue of the present invention is produced, the synthetic tissue is formed in the shape of a cell sheet in a container. When the sheet is detached, with conventional techniques, the sheet is usually destroyed (due to lack of self-supporting ability). Therefore, in conventional techniques, an implantable synthetic tissue is practically unproduceable. Especially when a large-sized synthetic tissue is required, conventional techniques are not adequate. The synthetic tissue of the present invention is understood as applicable to substantially any situation because such a synthetic tissue already has sufficient strength, i.e., has self-supporting ability, to endure being separated from a container in a form of a monolayer sheet prior to detachment if the techniques of the present invention is used when producing a synthetic tissue. It is understood that the monolayer may partially include a two or three-layer structure. In addition, typically, after a synthetic tissue is produced and detached, the strength and self-supporting ability of the synthetic tissue increases, as is observed in the present invention. Therefore, in the present invention, it is understood that the self-supporting ability evaluated upon production may be an important aspect. Naturally, the strength upon implantation is also important in the present invention. Thus, it may also be important to evaluate the self-supporting ability of a synthetic tissue when a predetermined time has passed after the production of the tissue. Therefore, it is understood that those skilled in the art can determine the timing and strength at the time of transport by back-calculating the time the tissue is to be used based on the above-described relationship.

As used herein, the term "membranous tissue" refers to a tissue in the form of membrane and is also referred to as "planar tissue" . Examples of membranous tissues include tissues of organs such as periosteum, pericardium, dura mater, and cornea.

As used herein, the term "organ" refers to a structure, which is a specific part of an individual organism, where a certain function of the individual organism is locally performed and is morphologically independent. Generally, in multicellular organisms (e.g., animals and plants), organs are made of several tissues in specific spatial arrangement and tissue consists of a number of cells. Examples of such organs include skin, blood vessel, cornea, kidney, heart, liver, umbilical cord, intestine, nerve, lung, placenta, pancreas, brain, joint, bone, cartilage, peripheral limbs, and retina. Examples of such organs also include organs of the skin system, the parenchyma pancreas system, the pancreatic duct system, the hepatic system, the blood system, the myocardial system, the skeletal muscle system, the osteoblast system, the skeletal myoblast system, the nervous system, the blood vessel endothelial system, the pigment system, the smooth muscle system, the fat system, the bone system, and the cartilage system.

In one embodiment, the present invention targets organs including an intervertebral disk, a cartilage, a joint, a bone, a meniscus, a synovial membrane, a ligament, and a tendon. In another preferable embodiment, the present invention targets organs including bones and cartilages.

As used herein, the term "cover" or "wrap" in relation to wrapping a composite tissue or the like around a certain part (e.g., an injured site) means that the composite tissue or the like is arranged so as to cover the part (i.e., conceal an injury or the like) . The terms "wrap" and "arrange so as to cover" the part are used interchangeably. By observing the spatial arrangement between the part and the synthetic tissue, three-dimensional construct or the like, it can be determined whether the part is arranged to be covered by the synthetic tissue, three-dimensional construct or the like. In a preferable embodiment, in a wrapping step, a synthetic tissue or the like can be wrapped one turn around a certain site.

As used herein, the term "replace" means that a lesion (a site of an organism) is replaced, or cells which have originally been in a lesion are replaced with cells supplied by a synthetic tissue or a complex according to the present invention. Examples of a disease for which replacement is suitable include a ruptured site. The term "fill" may be used in place of the term "replace" in the present specification.

A "sufficient time required for biologically integration" between a "synthetic tissue" or "composite tissue" and a certain "part" herein varies depending on a combination of the part and the synthetic tissue, but can be appropriately determined by those skilled in the art based on the combination. Examples of such a time include1 week, 2 weeks, 1 month, 2 months, 3 months, 6 months, and 1 year after an operation. In the present invention, a synthetic tissue preferably comprises substantially only cells and materials derived from the cells. Hence, there is no particular material which needs to be extracted after an operation. Therefore, the lower limit of the sufficient time is not particularly important. Thus, in this case, a longer time is more preferable. If the time is essentially extremely long, reinforcement is regarded as substantially completed.

As used herein, the term "immune reaction" refers to a reaction due to the dysfunction of immunological tolerance between a graft and a host. Examples of immune reactions include a hyperacute rejection reaction (within several minutes after implantation) (immune reaction caused by antibodies, such as β-Gal), an acute rejection reaction (reaction caused by cellular immunity about 7 to 21 days after implantation), and a chronic rejection reaction (rejection reaction caused by cellular immunity 3 or more months after operation).

As used herein, the elicitation of an immune reaction can be confirmed by pathological and histological examination of the type, number, or the like of infiltration of (immunological) cells into an implanted tissue by using staining such as HE staining, immunological staining, or microscopic inspection of tissue sections.

As used herein, the term "calcification" refers to precipitation of calcareous substances in organisms.

"Calcification" *in vivo* can be determined herein by Alizarin Red staining and measuring calcium concentration. Specifically, quantification is possible by taking out an implanted tissue is taken out and dissolving a tissue section by acid treatment or the like to measure the atomic absorption of the solution by a trace element quantifying device.

As used herein, the term " (with) in organism (s) " or *"in vivo"* refers to the inner part of organism(s) . In a specific context, "within organism(s) " refers to a position of interest where a subject tissue or organ is to be placed.

As used herein, *"in vitro"* indicates that apart of an organism is extracted or released "outside the organism" (e.g., in a test tube) for various purposes of research. The term *in vitro* is in contrast to the term *in vivo.*

As used herein, the term *"ex vivo"* refers to a series of operations where target cells into which a gene will be introduced are extracted from a subject; a therapeutic gene is introduced *in vitro* into the cells; and the cells are returned into the same subject.

As used herein, the term "material derived from cell(s)" refers to any material originating from the cell (s), including, but not being limited to, materials constituting the cell(s), materials secreted by the cell(s), and materials metabolized by the cell(s). Representative examples of materials derived from cells include extracellular matrices, hormones, and cytokines . Materials derived from cells typically have no adverse effect on the cells and their hosts . Therefore, when the material is contained in a three-dimensional synthetic tissue or the like, the material typically has no adverse effect.

As used herein, the term "extracellular matrix" (ECM) refers to a substance existing between somatic cells regardless of whether the cells are epithelial cells or non-epithelial cells. Extracellular matrices are typically produced by cells, and are therefore biological materials. Extracellular matrices are involved in not only supporting tissue, but also in structuring an internal environment essential for survival of all somatic cells. Extracellular matrices are generally produced from connective tissue cells , but some extracellular matrices are secreted from cells with a basal membrane, such as epithelial cells or endothelial cells. Extracellular matrices are roughly divided into fibrous components and matrices filling there between. Fibrous components include collagen fibers and elastic fibers. A basic component of matrices is a glycosaminoglycan (acidic mucopolysaccharide), most of which is bound to a non-collagenous protein to form a polymer of a proteoglycan (acidic mucopolysaccharide-protein complex). In addition, matrices include glycoproteins, such as laminin of basal membrane, microfibrils around elastic fibers, fibers, and fibronectins on cell surfaces. Particularly differentiated tissues have the same basic structure. For example, in hyaline cartilage, chondroblasts characteristically produce a large amount of cartilage matrices including proteoglycans. In bones, osteoblasts produce bone matrices which cause calcification. Herein, examples of a typical extracellular matrix include collagen I, collagen III, collagen V, elastin, vitronectin, fibronectin, laminin, thrombospondin, and proteoglycans (for example, decolin, byglican, fibromodulin, lumican, hyaluronic acid, and aggrecan) . Various types of extracellular matrix may be utilized in the present invention as long as cell adhesion is achieved.

In one embodiment of the present invention, an extracellular matrix included in a three-dimensional synthetic tissue or the like comprised in the composite tissue of the present invention may be advantageously similar to the composition of an extracellular matrix (e.g., elastin, collagen (e.g., Type I, Type III, or Type IV), or laminin) of a site of an organ for which implantation is intended. In the present invention, extracellular matrices include cell adhesion molecules. As used herein, the terms "cell adhesion molecule" and "adhesion molecule" are used interchangeably to refer to a molecule for mediating the joining of two or more cells (cell adhesion) or adhesion between a substrate and a cell. In general, cell adhesion molecules are divided into two groups: molecules involved in cell-cell adhesion (intercellular adhesion) (cell-cell adhesion molecules) and molecules involved in cell-extracellular matrix adhesion (cell-substrate adhesion) (cell-substrate adhesion molecules). A three-dimensional synthetic tissue of the present invention typically comprises such a cell adhesion molecule. Therefore, cell adhesion molecules herein include a protein of a substrate and a protein of a cell (e.g., integrin) in cell-substrate adhesion. A molecule other than proteins falls within the concept of cell adhesion molecules herein as long as it can mediate cell adhesion.

A feature of the present invention is the synthetic tissue included in the composite tissue of the present invention comprising cells and an (autologous) extracellular matrix produced by the cell itself. Therefore, it is characterized in having a complicated composition with a mixture of collagen I, collagen III, collagen V, elastin, vitronectin, fibronectin, laminin, thrombospondin, proteoglycans (for example, decolin, byglican, fibromodulin, lumican, hyaluronic acid, and aggrecan) or the like. Conventionally, a synthetic tissue containing such cell-derived ingredients has not been provided. Practically, it is nearly impossible to obtain a synthetic tissue having such a composition when an artificial material is used. Thus, a composition containing such ingredients (particularly, collagen I and collagen III) is recognized to be a native composition.

More preferably, an extracellular matrix includes each of collagen (Types I, Type III, etc.), vitronectin, and fibronectin. A synthetic tissue containing vitronectin and/or fibronectin in particular has never been provided before. Therefore, the synthetic tissue and the complex according to the present invention are recognized to be novel in this regard.

As used herein, with regard to the synthetic tissue of the present invention, the term "provided" or "distributed" in relation to an extracellular matrix indicates that the extracellular matrix is present in the synthetic tissue. It is understood that such provision can be visualized and observed with stain by immunologically staining an extracellular matrix of interest.

As used herein, the term "in a diffused manner" or "diffusedly" in relation to the "distribution" of an extracellular matrix indicates that the extracellular matrix is not localized. Such diffusion of an extracellular matrix refers to diffusion with a ratio of the distribution densities of two arbitrary 1 cm² sections within a range of typically about 1:10 to about 10:1, and representatively about 1:3 to about 3:1, and preferably about 1:2 to about 2:1. More preferably, the ratio is substantially evenly distributed in any section the synthetic tissue. When an extracellular matrix is not localized, but is diffused over a surface of the synthetic tissue of the present invention, the synthetic tissue of the present invention has biological integration capability evenly with respect to the surrounding. Therefore, the synthetic tissue of the present invention achieves an excellent effect of recovery after implantation.

For cell-cell adhesion, cadherin, a number of molecules belonging to an immunoglobulin superfamily (NCAM, L1, ICAM, fasciclin II, III, etc.), and selectin are known, each of which is known to join cell membranes via a specific molecular reaction. Therefore, in one embodiment, the three-dimensional synthetic tissue or the like of the present invention preferably has substantially the same composition of cadherin, immunoglobulin superfamily molecules, or the like as that of a site for which implantation is intended.

In this manner, various molecules are involved in cell adhesion and have different functions. Thus, those skilled in the art can appropriately select a molecule to be contained in the three-dimensional synthetic tissue used in the present invention depending on the purpose. Techniques for cell adhesion other than those described above are also well known, as described in, for example, "Saibogaimatorikkusu -Rinsho heno Oyo-[Extracellular matrix -Clinical Applications-], Medical Review.

It can be determined whether a certain molecule is a cell adhesion molecule by an assay, such as biochemical quantification (an SDS-PAGE method, a labeled-collagen method, etc.), immunological quantification (an enzyme antibody method, a fluorescent antibody method, an immunohistological study, etc.), a PCR method, or a hybridization method, exhibiting a positive reaction. Examples of such a cell adhesion molecule include collagen, integrin, fibronectin, laminin, vitronectin, fibrinogen, an immunoglobulin superfamily member (e.g., CD2, CD4, CD8, ICM1, ICAM2, VCAM1), selectin, and cadherin. Most of these cell adhesion molecules transmit into a cell an auxiliary signal for cell activation due to intercellular interaction as well as cell adhesion. Therefore, an adhesion molecule for use in an implant of the present invention preferably transmits such an auxiliary signal for cell activation into a cell. This is because cell activation can promote growth of cells originally present or aggregating in a tissue or organ at an injured site after application of an implant thereto. It can be determined whether such an auxiliary signal can be transmitted into a cell by an assay, such as biochemical quantification (an SDS-PAGE method, a labeled-collagen method, etc.), immunological quantification (an enzyme antibody method, a fluorescent antibody method, an immunohistological study, etc.), aPCRmethod, or a hybridization method, exhibiting a positive reaction.

An example of a cell adhesion molecule is cadherin, which is widely known in cell systems capable of being fixed to a tissue. Cadherin can be used in a preferable embodiment of the present invention. Examples of a cell adhesion molecule in cells of blood and the immune system which are not fixed to a tissue include immunoglobulin superfamily molecules (LFA-3, CD2, CD4, CD8, ICAM-1, ICAM2, VCAM1, etc.); integrin family molecules (LFA-1, Mac-1, gpIIbIIIa, p150, p95, VLA1, VLA2, VLA3, VLA4, VLA5, VLA6, etc.); and selectin family molecules (L-selectin, E-selectin, P-selectin, etc.) . Therefore, such a molecule may be especially useful for the treatment of a tissue or organ of the blood and immune system.

Non-fixed cells need to adhere to a specific tissue in order to act on the tissue. In this case, it is believed that cell-cell adhesion is gradually enhanced via a first adhesion by a selectin molecule or the like which is constantly expressed and a second adhesion by a subsequently activated integrin molecule. Therefore, as cell adhesion molecules used in the present invention, it is possible to use a cell adhesion molecule for mediating the first adhesion and another cell adhesion molecule for mediating the second adhesion, or both.

As used herein, the term "actin regulatory agent" refers to a substance with a function of interacting directly or indirectly with actin in cells to change the form or state of the actin. It is understood that actin regulatory agents are categorized into two classes, actin depolymerizing agents and actin polymerizing agents, depending on the action on actin. Examples of actin depolymerizing agents include Slingshot, cofilin, CAP (cyclase associated protein), AIP1 (actin-interacting-protein 1), ADF (actin depolymerizing factor), destrin, depactin, actophorin, cytochalasin, and NGF (nerve growth factor). Examples of actin polymerizing agents include RhoA, mDi, profilin, Rac1, IRSp 53, WAVE2, ROCK, LIM kinase, cofilin, cdc42, N-WASP, Arp2/3, Drf3, Mena, LPA (lysophosphatidic acid), insulin, PDGFa, PDGFb, chemokine, and TGF-β. The above-described actin regulatory agents include substances which can be identified by the following assay. Interaction of an actin regulatory agent with respect to actin is assayed herein as follows . Actin is made visible with an actin staining reagent (Molecular Probes, Texas Red-X phalloidin) or the like. By observing actin aggregation or cell outgrowth under a microscope, the presence of the interaction is determined by confirming the aggregation and reconstruction of actin and/or an increase in the cell outgrowth rate. The determination may be performed quantitatively or qualitatively. The above-described actin regulatory agents are used in the present invention so as to promote the detachment or a multilayer structure of the synthetic tissue . When an actin regulatory agent used in the present invention may be derived from any organism, including mammalian species such as a human, mouse, or bovine.

The above-described agents involved in actin polymerization control actin polymerization in relation to Rho and examples of the agents include the following (see, for example, "Saibokokkaku/Undo ga wakaru (Understanding of cytoskeleton/movement)", (Ed./Hiroaki Miki), Yodo-sha).

Actin polymerization (see Takenaka T et al. J.Cell Sci., 114: 1801-1809, 2001)
RhoA -> mDi -> profilin ⇒ actin polymerization
RhoA → ROCK/Rho -> LIM kinase -> phosphorylation of cofilin (suppression) ⇒ actin polymerization
Rac1 -> IRSp53 →WAVE2 →profilin, Arp2/3 ⇒ actin polymerization cdc42 -> N-WASP -> profilin, Arp2/3 ⇒ actin polymerization cdc42 -> Drf3 -> IRSp53 -> Mena ⇒ actin polymerization
(In the above descriptions, -> indicates a signal transduction pathway such as phosphorylation.

In the present invention, any agent involved in such a pathway can be utilized.

Actin depolymerization
Slingshot -> dephosphorization of cofilin (activation) ⇒ actin depolymerization
Actin depolymerization is controlled by the balance between phosphorylation by LIM kinase activity of cofilin and dephosphorization by Slingshot. As another agent for activating cofilin, CAP (cyclase-associated protein) and AIPI (actin-interacting-protein 1) are identified. Any suitable agent is recognized as usable therefor.

LPA (lysophosphatidic acid) of any chain length can be used.

Any chemokine can be used. However, examples of preferable chemokine include interleukin 8, MIP-1, and SDF-1.

Any TGF-β can be used. However, examples of preferable TGF-β include TGF-β1 and TGF-β3. TGF-β1 and TGF-β3 have an extracellular matrix generation promoting activity. Thus, it is noted that TGF-β1 and TGF-β3 can be used in the present invention.

As used herein, the term "tissue strength" refers to a parameter which indicates a function of a tissue or organ and a physical strength of the tissue or organ. Tissue strength can be generally determined by measuring tensile strength (e.g., break strength, modulus of rigidity, and Young's modulus) . Such a general tensile test is well known. By analyzing data obtained by a general tensile test, various data, such as break strength, modulus of rigidity, and Young's modulus, can be obtained. These values can be used herein as indicators of tissue strength. Typically, tissue strength which allows clinical applications is herein required.

Herein, the tensile strength of a three-dimensional synthetic tissue or the like that is used in the present invention can be determined by measuring the stress and distortion characteristics thereof. Briefly, a load is applied to a sample; the resultant distortion and the load are input into respective A/D converters (e.g., ELK-5000) (e.g., 1 ch: distortion, 2 ch: load) ; and the stress and distortion characteristics are measured to determine the tensile strength. Tensile strength can also be determined by testing creep characteristics. A creep characteristics indentation test is a test to investigate how a sample extends over time while a constant load is applied to the sample. For small materials and thin materials an indentation test is conducted using, for example, a tetrahedronal indenter with a tip having a radius of about 0.1 µm to about 1 µm. Initially, the indenter is pushed into a test piece to apply a load. When the indenter reaches several tens of nanometers to several micrometers in depth into the test piece, the indenter is withdrawn to remove the load. Rigidity, Young's modulus, or the like can be obtained based on the behavior of the load and the push depth derived from the curve.

The tensile strength of the synthetic tissue of the present invention may be low. The tensile strength increases when the extracellular matrix in the cell to extracellular matrix ratio is increased, and decreases when the cell to extracellular matrix ratio is increased. The present invention is characterized in that the strength can be freely adjusted as necessary. The present invention is characterized in that the strength can be high or low relative to that of a tissue to be implanted. Therefore, it is recognized that the strength can be set to comply with any desired site.

As used herein, the term "physiologically active substance" refers to a substance capable of acting on a cell or tissue. Physiologically active substances include cytokines and growth factors. A physiologically active substance may be naturally-occurring or synthesized. Preferably, a cellular physiologically active substance is one that is produced by a cell or one that has a function similar thereto. As used herein, a physiologically active substance may be in the form of aprotein or a nucleic acid or in other forms. In actual practice, physiologically active substances typically refer to proteins. In the present invention, a physiologically active substance may be used to promote the affinity of an implanted synthetic tissue of the present invention.

As used herein, the term "cytokine" is defined in the broadest sense in the art and refers to a physiologically active substance which is produced from a cell and acts on the same or different cell. Cytokines are generally proteins or polypeptides having a function of controlling an immune response, regulating the endocrine system, regulating the nervous system, acting against a tumor, acting against a virus, regulating cell growth, regulating cell differentiation, or the like. Cytokines are in the form of a protein or a nucleic acid or in other forms herein. In actual practice, cytokines typically refer to proteins.

The terms "growth factor" or "cell growth factor" are used herein interchangeably and each refers to a substance which promotes or controls cell growth. Growth factors are also called "proliferation factors" or "development factors". Growth factors may be added to cell or tissue culture medium to replace the action of serum macromolecules. It has been revealed that a number of growth factors have a function of controlling differentiation in addition to a function of promoting cell growth.

Examples of representative cytokines include interleukins, chemokines, hematopoietic factors such as colony stimulating factors, a tumor necrosis factor, and interferons, and a platelet-derived growth factor (PDGFa, PDGFb), an epidermal growth factor (EGF), a fibroblast growth factor (FGF), a hepatocyte growth factor (HGF), and a vascular endothelial cell growth factor (VEGF) as growth factors having proliferative activity.

Physiologically active substances, such as cytokines and growth factors, typically have redundancy in function. Accordingly, cytokines or growth factors that are known by another name or function can be used in the present invention as long as they have the activity of a physiologically active substance for use in the present invention. Cytokines or growth factors can be used in a therapeutic method or pharmaceutical agent according to an embodiment of the present invention as long as they have preferable activity as described herein.

Therefore, in one embodiment of the present invention, it was revealed that when such a cytokine or growth factor (e.g., BMP-2) is provided to an implantation site (e.g., an injured site of a cartilage) concomitantly with a synthetic tissue or three-dimensional structure of the present invention, the affinity of the synthetic tissue or three-dimensional structure and an improvement in the function of the implantation site are observed. Thus, the present invention also provides such a combined therapy.

As used herein, the term "differentiation" refers to a developmental process of the state of parts of organisms, such as cells, tissues, or organs and a process in which a characteristic tissue or organ is formed. The term "differentiation" is mainly used in embryology and developmental biology. In organisms, various tissues and organs are formed from divisions of a fertilized ovum (a single cell) to be an adult. At early developmental stages (i.e., before cell division or after insufficient cell division), each cell or cell group has no morphological or functional feature and is thus indistinguishable. Such a state is referred to as "undifferentiated". "Differentiation" may occur at the level of organs. A cell constituting an organ develops into various cells or cell groups having different characteristics. This phenomenon is also referred to as differentiation within an organ in the formation of the organ. Therefore, a three-dimensional synthetic tissue that is used in the present invention may use a tissue including differentiated cells.

When differentiation is required to produce a three-dimensional synthetic tissue that is used in a composite tissue or the composite tissue of the present invention, the differentiation may be allowed to occur either before or after the organization of the cells.

As used herein, the terms "differentiation agent" and "differentiation promoting agent" are used interchangeably and refer to any agent which is known to promote differentiation into differentiated cells (e.g., chemical substances or temperature). Examples of such an agent include various environmental factors, such as temperature, humidity, pH, salt concentration, nutrients, metals, gas, organic solvent, pressure, chemical substances (e.g., steroids and antibiotics), and any combinations thereof. Representative examples of differentiation agents include cellular physiologically active substances. Representative examples of such cellular physiologically active substances include DNA demethylating agents (e.g., 5-azacytidine), histone deacetylating agents (e.g., trichosanthin), intranuclear receptor ligands (e.g., retinoic acid (ATRA), vitamin D₃, and T3), cell growth factors (e.g., activin, IGF-1, FGF, PDGFa, PDGFb, TGF-β, and BMP2/4), cytokines (e.g., LIF, IL-2, IL-6), hexamethylenebisacetoamides, dimethylacetoamides, dibutyl cAMPs, dimethylsulfoxides, iododeoxyuridines, hydroxyl ureas, cytosine arabinosides, mitomycin C, sodium lactate, aphydicolin, fluorodeoxyuridine, polybren and selenium.

Specific examples of differentiation agents are described below. These differentiation agents may be used alone or in combination.
1) Synovial cell: FGF, TGF-β (particularly, TGF-β1, TGF-β3);
2) Osteoblast: BMP (particularly, BMP-2, BMP-4, BMP-7), FGF;
3) Chondroblast: FGF, TGF-β (particularly, TGF-β1, TGF-β3), BMP (particularly, BMP-2, BMP-4, BMP-7), TNF-α, IGF;
4) Fat cell: insulin, IGF, LIF; and
5) Muscle cell: LIF, TNF-α, FGF.

As used herein, the term "osteogenesis" refers to making differentiate into an osteocyte. It is known that osteogenesis is promoted in the presence of dexamethasone, β-glycerophosphate, and ascorbic acid 2-phosphate. An osteogenic agent (BMP, (particularly, BMP-2, BMP-4, BMP-7)) may be added to promote osteogenesis.

As used herein, the term "chondrogenesis" refers to making any cell differentiate into a chondrocyte. It is known that chondrogenesis is promoted in the presence of pyrubic acid, dexamethasone, ascorbic acid 2-phosphate, insulin, transferrine, and selenious acid. A bone morphogenetic protein (BMP, (particularly, BMP-2, BMP-4, BMP-7)), TGF-β (particularly, TGF-β1 and TGF-β3), FGF, TNF-α or the like may be added to promote chondrogenesis.

As used herein, the term "adipogenesis" refers to making any cell differentiate into an adipocyte. It is known that adipogenesis is promoted in the presence of insulin, IGF, LIF, or ascorbic acid 2-phosphate.

As used herein, the terms "implant", "graft", and "tissue graft" are used interchangeably, referring to a homologous or heterologous tissue or a cell group which is inserted into a particular site of a body and thereafter forms a part of the body after insertion. Therefore, a three-dimensional synthetic tissue that is used in the present invention can be used as an implant. Examples of grafts include organs or portions of organs, dura mater, joint capsule, bone, cartilage, cornea, and tooth. Therefore, grafts encompass anything that is inserted into a defect part so as to compensate for the lost portion. Grafts include autografts, allografts, and xenografts, which depend on the type of their donor.

As used herein, the term "autograft" (a tissue, a cell, an organ, etc.) refers to a graft (a tissue, a cell, an organ, etc.) which is implanted into the same individual from which the graft is derived. As used herein, the term "autograft" (a tissue, a cell, an organ, etc.) may encompass a graft (a tissue, a cell, an organ, etc.) from another genetically identical individual (e.g. an identical twin) in a broad sense. As used herein, the terms "autologous" and "derived from a subject" are used interchangeably. Therefore, the term "not derived from a subj ect" is synonymous to the graft not being autologous (i.e., heterologous).

As used herein, the term "allograft (a tissue, a cell, an organ, etc.)" refers to a graft (a tissue, a cell, an organ, etc.) which is transplanted from a donor genetically different from, though of the same species as, the recipient. Since an allograft is genetically different from the recipient, the allograft (a tissue, a cell, an organ, etc.) may elicit an immune reaction in the recipient of implantation. Examples of such grafts (a tissue, a cell, an organ, etc.) include grafts derived from parents (a tissue, a cell, an organ, etc.) . The synthetic tissue of the present invention can be an allograft, which is noteworthy in terms of being demonstrated to have satisfactory therapeutic results.

As used herein, the term "xenograft" (a tissue, a cell, an organ, etc.) refers to a graft (a tissue, a cell, an organ, etc.) which is implanted from a different species. Therefore, for example, when a human is a recipient, a porcine-derived graft (a tissue, a cell, an organ, etc.) is called a xenograft (a tissue, a cell, an organ, etc.).

As used herein, "recipient" (acceptor) refers to an individual who receives a graft (a tissue, a cell, an organ, etc.) or implanted matter (a tissue, a cell, an organ, etc.) and is also called "host". In contrast, an individual providing a graft (a tissue, a cell, an organ, etc.) or implanted matter (a tissue, a cell, an organ, etc.) is called "donor" (provider).

When making a composite tissue of the present invention, a synthetic tissue derived from any cell can be used. This is because a synthetic tissue (e.g., membranous tissues or organs) that is used in a composite tissue formed by the method of the present invention can exhibit a desired function while the tissue injury rate is maintained at a level which does not interfere with the therapy of interest (i. e., a low level) . Conventionally, tissues or organs could only be directly used as grafts. In contrast to this state, the present invention enables the formation of a tissue that is three-dimensionally integrated from cells. Use of such a synthetic three-dimensional tissue and significant improvement in therapeutic results in comparison to prior art cannot be achieved by conventional techniques, which constitutes one significant effect of the present invention.

As used herein, the term "subject" refers to an organism to which treatment of the present invention is applied and is also referred to as "patient". A patient or subject may be preferably a human.

Cells comprised in a composite tissue of the present invention may be derived from a syngeneic origin (self origin), an allogenic origin (non-self origin), or a heterologous origin. In view of rejection reactions, syngeneic cells are preferable. If rejection reactions do not raise problems, allogenic cells may be employed. Cells which elicit rejection reactions can also be employed by optionally treating the cells in a manner that overcomes rejection reactions. Procedures for avoiding rejection reactions are known in the art, as described in, for example, "Shin Gekagaku Taikei, Dai 12 Kan, Zoki Ishoku (Shinzo Ishoku · Hai Ishoku Gijutsuteki, Rinriteki Seibi kara Jisshi ni Mukete [New Whole Surgery, Vol. 12, Organ Transplantation (Heart Transplantation · Lung Transplantation From Technical and Ethical Improvements to Practice)" (Revised 3rd ed.), Nakayama Shoten. Examples of such methods include a method using immunosuppressants or steroidal drugs. For example, there are currently the following immunosuppressants for preventing rejection reactions: "cyclosporine" (SANDIMMUNE/NEORAL); "tacrolimus" (PROGRAF); "azathioprine" (IMURAN); "steroid hormone" (prednine, methylprednine); and "T-cell antibodies" (OKT3, ATG, etc.) . Amethodwhich is used worldwide as a preventive immunosuppression therapy in many facilities, is the concurrent use of three drugs: cyclosporine, azathioprine, and steroid hormone. An immunosuppressant is desirably administered concurrently with a pharmaceutical agent of the present invention. An immunosuppressant may be administered before or after a regeneration/therapeutic method of the present invention as long as an immunosuppression effect can be achieved.

Examples of a combination of a target subj ect and a composite tissue of the present invention include dura mater implant at the time of brain surgery, a joint injury or denaturation; a osteochondral injury or denaturation; osteonecrosis; meniscus injury or denaturation; intervertebral disk denaturation; ligament injury or denaturation; a fracture; and implantation to a patient having a j oint, cartilage, or bone having bone defect.

Tissues targeted by the present invention may be any organ of an organism and may be derived from any animal. Examples of organisms targeted by the present invention include vertebrates . Preferably, organisms targeted by the present invention are mammals (e.g., primates or rodents) . More preferably, organisms targeted by the present invention are primates . Most preferably, organisms targeted by the present invention are humans.

As used herein, the term "flexibility" in relation to a synthetic tissue refers to an ability to resist physical stimuli from external environments (e.g., pressure) or the like. A synthetic tissue having flexibility is preferable when the implantation site moves or deforms autonomously or due to external effects.

As used herein, the term "extendibility and contractibility" in relation to a synthetic tissue refers to a property of having an ability to resist extending or contracting stimuli from external environments (e.g., pulsation). A synthetic tissue having extendibility and contractibility is preferable when the implantation site is subjected to extending or contracting stimuli. Examples of implantation sites, which are subjected to extending or contracting stimuli, include muscle, joint, cartilage, and tendon.

As used herein, the term "part" or "portion" refers to any part or portion, tissue, cell, or organ in the body. Examples of such parts, tissues, cells, and organs include a portion which can be treated with skeletal myoblasts, fibroblasts, synovial cells, or stem cells. A marker specific to a portion may be any parameter, such as a nucleic acid molecule (expression of mRNA), a protein, an extracellular matrix, a specific phenotype, or a shape of a cell. Therefore, any marker which is not specified herein may be used to identify a synthetic tissue of the present invention as long as the marker can indicate that cells are derived from said portion. Representative examples of such portions include portions containing mesenchymal stem cells or cells derived therefrom, other tissues, organs, myoblasts (e.g., skeletal myoblasts), fibroblasts, and synovial cells.

For observing a cartilage tissue or the like, following markers can be used as an index.

Sox9 (human: Accession No. NM_000346) is a marker specific to a chondrocyte . The marker can be confirmed mainly by observing the presence of mRNA (Kulyk WM, Franklin JL, Hoffman LM. Sox9 expression during chondrogenesis in micromass cultures of embryonic limb mesenchyme. Exp Cell Res. 2000 Mar 15, 255(2):327-32.).

Col2A1 (human: AccessionNo. NM_001844) is a marker specific to a chondrocyte . The marker can be confirmed mainly by observing the presence of mRNA (Kulyk WM, Franklin JL, Hoffman LM. Sox9 expression during chondrogenesis in micromass cultures of embryonic limb mesenchyme. Exp Cell Res. 2000 Mar 15; 255(2):327-32.).

Aggrecan (human: Accession No. NM_001135) is a marker specific to a chondrocyte. The marker can be confirmed mainly by observing the presence of mRNA (Kulyk WM, Franklin JL, Hoffman LM. Sox9 expression during chondrogenesis in micromass cultures of embryonic limb mesenchyme. Exp Cell Res. 2000 Mar 15; 255(2):327-32.).

Bone sialoprotein (human: Accession No. NM_004967) is a marker specific to an osteoblast. The marker can be confirmed mainly by observing the presence of mRNA (Haase HR, Ivanovski S, Waters MJ, Bartold PM. Growth hormone regulates osteogenic marker mRNA expression in human periodontal fibroblasts and alveolar bone-derived cells. J Periodontal Res. 2003 Aug; 38(4):366-74.).

Osteocalcin (human: Accession No. NM_199173) is a marker specific to an osteoblast. The marker can be confirmed mainly by observing the presence of mRNA (Haase HR, Ivanovski S, Waters MJ, Bartold PM. Growth hormone regulates osteogenic marker mRNA expression in human periodontal fibroblasts and alveolar bone-derived cells. JPeriodontalRes. 2003 Aug; 38 (4) :366-74.) .

GDF5 (human: Accession No. NM_000557) is a marker specific to a ligament cell. The marker can be confirmed mainly by observing the presence of mRNA (Wolfman NM, Hattersley G, Cox K, Celeste AJ, Nelson R, Yamaji N, Dube JL, DiBlasio-Smith E, Nove J, Song JJ, Wozney JM, Rosen V. Ectopic induction of tendon and ligament in rats by growth and differentiation factors 5, 6, and 7, members of the TGF-beta gene family. J Clin Invest. 1997 Jul 15; 100(2):321-30.).

Six1 (human: Accession No. NM_005982) is a marker specific to a ligament cell (Dreyer SD, Naruse T, Morello R, Zabel B, Winterpacht A, Johnson RL, Lee B, Oberg KC. Lmx1b expression during joint and tendon formation: localization and evaluation of potential downstream targets. Gene Expr Patterns. 2004 Jul; 4(4):397-405.). The marker can be confirmed mainly by observing the presence of mRNA.

Scleraxis (human: Accession No. BK000280) is a marker specific to a ligament cell (Brent AE, Schweitzer R, Tabin CJ. A somitic compartment of tendon progenitors. Cell. 2003 Apr 18; 113(2):235-48.). The marker can be confirmed mainly by observing the presence of mRNA.

CD56 (human: Accession No. U63041; SEQ ID NOs. 7 and 8) is a marker specific tomyoblasts. The marker can be confirmed mainly by observing the presence of mRNA.

MyoD (human: Accession No. X56677; SEQ ID NOs. 9 and 10) is a marker specific to myoblasts. This marker can be confirmed mainly by observing the presence of mRNA.

Myf5 (human: Accession No. NM_005593; SEQ ID NOs. 11 and 12) is a marker specific to myoblasts. The marker can be confirmed mainly by observing the presence of mRNA.

Myogenin (human: Accession No. BT007233; SEQ ID NOs. 13 and 14) is a marker specific to myoblasts . This marker can be confirmed mainly by observing the presence of mRNA.

In other embodiments, other markers specific to other tissues can be utilized. Examples of such markers include: Oct-3/4, SSEA-1, Rex-1, and Otx2 for embryonic stem cells; VE-cadherin, Flk-1, Tie-1, PECAM1, vWF, c-kit, CD34, Thy1, and Sca-1 for endothelial cells; skeletal muscle α actin in addition to the above-described markers for skeletal muscles; Nestin, Glu receptor, NMDA receptor, GFAP, and neuregulin-1 for nerve cells; and c-kit, CD34, Thy1, Sca-1, GATA-1, GATA-2, and FOG for hematopoietic cells.

As used herein, the term "derived" in relation to cells means that the cells are separated, isolated, or extracted from a cell mass, tissue, or organ in which the cells have been originally present, or that the cells are induced from stem cells.

As used herein, the term "three-dimensional synthetic tissue" refers to a synthetic tissue comprised in a composite tissue of the present invention, which is substantially made of a cell and an extracellular matrix from the cell. Such three-dimensional synthetic tissues typically constitute a three-dimensional structure. As used herein, the term "three-dimensional structure" refers to an object extending three-dimensionally, wherein the object comprises cells having intracellular integration or alignment and extends three-dimensionally and wherein matrices are oriented three-dimensionally and cells are arranged three-dimensionally. The extracellular matrix contains fibronectin, collagen I, collagen III, and vitronectin, and the extracellular matrix is diffusedly distributed in the tissue. The extracellular matrix are integrated (biologically integrated or has a biological integration) with the cell to form a three-dimensional structure together, have an ability to integrate with the surroundings when implanted, and have sufficient strength to provide a self-supporting ability.

As used herein, the term "artificial bone" refers to a medical device made of artificial material for filling a defect portion of a bone. Artificial bones are made of a material with high affinity to a normal human body. Preferably, an artificial bone uses components of actual bone. Such a material is typically made of a material selected from the group consisting of materials with high affinity to a human body such as ceramics such as hydroxyapatite (HA) and α-tricalcium phosphate or β-tricalcium phosphate, bioglass (silicon), bioceramics such as carbon, alumina, or zirconia, metals such as titanium or tungsten, and coral materials.

As usedherein, the term "composite tissue" refers to a tissue obtained by combining a three-dimensional tissue with another synthetic tissue such as an artificial bone. As used herein, the term "composite tissue" may be called "hybrid graft", but is used in the same meaning as "composite tissue". Thus, such a composite tissue can be used in the treatment of a plurality of tissues (bone/cartilage or the like). For example, such a composite tissue can be used in treating both a cartilage and a bone. The composite tissue of the present invention biologically integrates an implantable (three-dimensional) synthetic tissue and another synthetic tissue. Such integration can be achieved by allowing contact and optionally culturing two tissues. Such biological integration is mediated by an extracellular matrix. A three-dimensional synthetic tissue refers to an object extending three-dimensionally, wherein the object comprises cells having intracellular integration or alignment and extends three-dimensionally and wherein matrices are oriented three-dimensionally and cells are arranged three-dimensionally.

As used herein, the term "biological integration" in relation to the relationship between biological entities means that there is certain biological interaction between the biological entities (it is understood that integration and union can be interchangeably used). For body tissues such as bones and cartilages, biological union or biological integration is referred to as "integration", which is used herein in the same meaning. Examples of such interaction include interaction via biological molecules (e.g., extracellular matrix), interaction via signal transduction, and electrical interaction (electrical integration, such as synchronization of electrical signals). Biological integration includes biological integration in a synthetic tissue and biological integration of a synthetic tissue with its surroundings (e.g., surrounding tissues and cells after implantation). In order to confirm interactions, an assay appropriate for a characteristic of the interaction is employed. In order to confirm physical interactions via biological molecules, the strength of a three-dimensional synthetic tissue or the like is measured (e.g., a tensile test) . In order to confirm interaction via signal transduction, gene expression or the like is investigated for the present of signal transduction. In order to confirm electrical interactions, the electric potential of a three-dimensional synthetic tissue or the like is measured to determine whether the electric potential is propagated with constant waves. In the present invention, biological integration is typically provided in all three dimensional directions. Preferably, there is biological integration substantially uniformly in all directions in a three-dimensional space. However, in another embodiment, the three-dimensional synthetic tissue or the like, which has substantially uniform two-dimensional biological integration and slightly weaker biological integration in three- dimensional directions, may be employed. Alternatively, biological integration via an extracellular matrix can be confirmed based on the degree of staining by staining the extracellular matrix. As a method for observing biological integration *in vivo,* there is an integration experiment using a cartilage. In this experiment, a surface of the cartilage is removed and digested with chondroitinase ABC (Hunziker E.B. et al., J. Bone Joint Surg. Am., 1996May; 78 (5) : 721-33) . Thereafter, a tissue of interest is implanted onto the cut surface, followed by culturing for about 7 days. The subsequent integration is histologically observed. It is understood that a capability to adhere to surrounding cells and/or extracellular matrix can be determined with the above-described experiment using a cartilage.

A composite tissue or the like of the present invention may be provided using known preparation methods, as a pharmaceutical product or alternatively as medical instrument, an animal drug, a quasi-drug, a marine drug, a cosmetic product or the like.

Animals targeted by the present invention include any organism, as long as it has organs (e.g., animals (e.g., vertebrates)). Preferably, the animal is a vertebrate (e.g., mammalian), more preferably mammal (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, or lagomorpha). Illustrative examples of a subject include animals, such as cattle, pigs, horses, chickens, cats, and dogs. More preferably, primates (e.g., chimpanzee, Japanese monkey, or human) are used. Most preferably, a human is used. This is because there is limitation to implantation therapies.

When the present invention is used as a pharmaceutical agent, it may further comprise a pharmaceutically acceptable carrier or the like. A pharmaceutically acceptable carrier contained in a pharmaceutical agent of the present invention includes any material known in the art.

Examples of such a suitable formulation material or pharmaceutically acceptable carrier include antioxidants, preservatives, colorants, flavoring agents, diluents, emulsifiers, suspending agents, solvents, fillers, bulking agents, buffers, delivery vehicles, diluents, excipient and/or pharmaceutical adjuvants.

The amount of a pharmaceutical agent (e.g., a composite tissue, a pharmaceutical compound used in conjunction therewith, etc.) used in the treatment method of the present invention can be readily determined by those skilled in the art while considering the purpose of use, a target disease (type, severity, and the like), the patient's age, weight, sex, case history, the form or type of the cell, or the like. The frequency of the treatment method of the present invention applied to a subject (or patient) is also readily determined by those skilled in the art while considering the purpose of use, target disease (type, severity, and the like), the patient's age, weight, sex, case history, the progression of the therapy, or the like. Examples of the frequency include once, as many cases are healed after one treatment. Needless to say, treatment of two or more times is also contemplated while considering the results.

As used herein, the term "administer", in relation to a composite tissue or the like of the present invention or a pharmaceutical agent comprising it, means that it is administered alone or in combination with another therapeutic agent. A composite tissue of the present invention may be introduced into therapy sites (e.g., osteochondral defect) by the following methods, in the following forms, and in the following amounts. Specifically, administration methods of a composite tissue of the present invention include direction insertion into an impaired site of osteoarthritis, or the like. Combinations may be administered either concomitantly as an admixture, separately but simultaneously or concurrently; or sequentially. This includes presentations in which the combined agents are administered together as a therapeutic mixture, and also procedures in which the combined agents are administered separately but simultaneously (e.g., a composite tissue or the like is directly provided by operation, while other pharmaceutical agents are provided by intravenous injection). "Combination" administration further includes the separate administration of one of the compounds or agents given first, followed by the second.

As used herein, the term "reinforcement" means that the function of a targeted part of an organism is improved.

As used herein, the term "instructions" refers to an article describing how to handle a composite tissue, reagents and the like, usage, a preparation method, a method of producing a synthetic tissue, a contraction method, amethodof administering a pharmaceutical agent of the present invention, a method for diagnosis, or the like for persons who administer or are administered with the pharmaceutical agent or the like or persons who diagnose (e.g., may be the patients). The instructions describe a statement for instructing the procedure for administering a diagnostic or pharmaceutical agent or the like of the present invention. The instructions are prepared in accordance with a format defined by an authority of the country in which the present invention is practiced (e.g., Health, Labor and Welfare Ministry in Japan or Food and Drug Administration (FDA) in the U.S.), explicitly describing that the instructions are approved by the authority. The instructions are so-called package insert and are typically provided in paper media. The instructions may also be provided in the form of electronic media (e.g., web sites, electronic mails, or the like provided on the Internet).

As used herein, the term "extracellular matrix synthesis promoting agent" or "ECM synthesis promoting agent" refers to any agent which promotes the production of an extracellular matrix of a cell. In the present invention, when an extracellular matrix synthesis promoting agent is added to a cell sheet, an environment which promotes detachment of the cell sheet from a culture container is provided and such a sheet biologically integrates in three-dimensional directions, where biological integration includes integration of extracellular matrix and cells in a tissue and integration of extracellular matrix with each other. Here, three-dimensionalization is further promoted by self-contraction. Representative examples of such an agent include agents capable of promoting the secretion of an extracellular matrix (e.g., TGF-β1 and TGF-β3). Representative examples of an ECM synthesis promoting agent include TGF-β1, TGF-β3, ascorbic acid, ascorbic acid 2-phosphate, and a derivative and salt thereof. Preferably, an ECM synthesis promoting agent may be preferably a component of an extracellular matrix of a part targeted by application and/or a component (s) capable of promoting the secretion of an extracellular matrix in an amount similar thereto . When such an ECM synthesis promoting agent comprises a plurality of components, such components may be components of an extracellular matrix of a part targeted by application and/or components in an amount similar thereto.

As used herein, the term "ascorbic acid or a derivative thereof" includes ascorbic acid and an analog thereof (e.g., ascorbic acid 2-phosphate), and a salt thereof (e.g., sodium salt and magnesium salt). Ascorbic acid is preferably an L-isomer.

### (Description of the Preferred Embodiments)

Hereinafter, preferable embodiments of the present invention will be described.

### (Composite tissue)

In one aspect, the present invention provides a composite tissue for treating or preventing a disease, disorder, or condition associated with an osteochondral defect, comprising a three-dimensional synthetic tissue and an artificial bone. The composite tissue of the present invention has successfully healed a disease, disorder, or condition associated with an osteochondral defect at a level that was impossible with conventional techniques. Thus, the present invention achieves a significant effect in terms of drastic improvement in therapeutic results.

In general, in one embodiment, a three-dimensional synthetic tissue used in the present invention is substantially made of a cell and an extracellular matrix derived from the cell. Preferably, a three-dimensional synthetic tissue used in the present invention is substantially made of a cell or a substance derived from the cell. Since the synthetic tissue is composed substantially of only cells and a cell-derived material (e.g., extra cellular matrix), the synthetic tissue can have an increased level of biocompatibility and affinity. As used herein, the term "substantially made of ..." is defined such that cells and substances derived from the cells are included, and also any other substance may be included as long as it does not cause any harmful effect (herein, mainly, adverse effect on implantation), and should understood as such herein. Such substances which do not cause any harmful effect are known to those skilled in the art or can be confirmed by conducting a simple test. Typically, such substances are any additives approved by the Health, Labor and Welfare Ministry (or PMDA), FDA, or the like, and ingredients involved in cell culture. The cell-derived material representatively includes extracellular matrices. Particularly, the three-dimensional synthetic tissue of the present invention preferably comprises a cell and an extracellular matrix at an appropriate ratio. Examples of such an appropriate ratio of a cell and an extracellular matrix include 1:3 to 20:1. The strength of a tissue is adjusted by the ratio between a cell and an extracellular matrix. Thus, the ratio between a cell and an extracellular matrix can be adjusted for use in accordance with application of cell implantation and physical environment at the implantation site. The preferable ratio varies depending on the intended treatment. Such a variation is apparent to those skilled in the art and can be estimated by investigating the ratio of a cell in an organ which is a target and an extracellular matrix.

In a preferred embodiment, an extracellular matrix comprised in a three-dimensional synthetic tissue used in the present invention contains fibronectin, collagen I, collagen III, and vitronectin. Preferably, various such extracellular matrices contain all of those listed above. It is advantageous that they are integrated and mixed. In another preferred embodiment, the extracellular matrix is diffusedly distributed in the tissue. Alternatively, extracellular matrices are preferably scattered across the entire tissue. Such a distribution achieves a significant effect of improving compatibility and affinity with an environment when implanted. In the preferred embodiment, regarding extracellular matrices that are diffusedly distributed on the three-dimensional synthetic tissue used in the present invention, distribution densities in any two section of 1 cm² , when compared, are preferably within the range of about 1:2 to 2:1 and more preferably about 1.5:1-1.5:1. The diffusion of distribution of extracellular matrices is advantageously uniform. Preferably, extracellular matrix is dispersed substantially uniform. The three-dimensional synthetic tissue used in present invention is known to be characterized in that adhesion to intercellular matrix which promotes cell adhesion to a matrix, cell extension, and cell chemotaxis is especially promoted by including collagen (Types I, III), vitronectin, and fibronectin. In one embodiment, an extracellular matrix distributed in a three-dimensional synthetic tissue used in the present invention may include collagen I, collagen III, vitronectin, fibronectin or the like. However, a synthetic tissue which includes integrated collagen (Types I, III), vitronectin and fibronectin has not been provided. Although it is not desired to be constrained by theory, but collagen (Types I, III), vitronectin and fibronectin are contemplated to have a function in exercising the biological integration capability with the surrounding. Therefore, in the preferable embodiment, it is advantageous that vitronectin are diffusedly distributed on a surface of the three-dimensional synthetic tissue used in the present invention. This is because it is considered that adhesion, affinity, and stability after implantation would be significantly different.

It is preferable that fibronectin is also diffusedly distributed in the three-dimensional synthetic tissue used in the present invention. It is known that fibronectin has a function in cell adhesion, in cell shape regulation, and in adjustment in cell migration. However, a synthetic tissue in which fibronectin is expressed has not been provided. Although it is not desired to be constrained by theory, fibronectin is also believed to have a function in exercising the biological integration capability with the surrounding. Therefore, in the preferable embodiment, it is advantageous that fibronectin is also diffusedly distributed on a surface of three-dimensional synthetic tissue used in the present invention. This is because it is considered that adhesion, affinity, and stability after implantation would be significantly different.

Since the three-dimensional synthetic tissue used in the present invention includes an abundance of adhesion molecules such as extracellular matrix including collagen (types I, III, etc.), vironectin, and fibronectin, the tissue is accepted by the surrounding tissue. Thus, implanted cells can be stably accepted by the implantation site. In conventional cell implantation, it was difficult for cells to be stably accepted by the implantation site not only in cells implantation without a scaffold, but also in cell implantation using an additional stabilizing treatment (sewing of a patch, scaffold, etc.). However, use of the present invention facilitates stabilization. When only cells are used, reinforcement by another tissue, fixing scaffold, or the like is necessary. However, if the composite tissue comprising a three-dimensional synthetic tissue of the present invention is used, without requiring such means, cells which may have pluripotency included in the three-dimensional synthetic tissue can be stably accepted by the implantation portion without an additional fixing means.

In another preferred embodiment, the extracellular matrix and the cell integrate to form a three-dimensional structure together. In another preferred embodiment, the extracellular matrix and the cell have an ability to integrate to the surroundings when implanted and have sufficient strength to provide a self-supporting ability. Preferably, the three-dimensional synthetic tissue to be used is substantially made of a cell selected from the group consisting of myoblasts, mesenchymal stem cells, adipocytes, synovial cells, and bone marrow cells and an extracellular matrix derived from the cell. The extracellular matrix contains collagen I and/or collagen III, and there are more of the collagen I and/or collagen III than collagen II. The extracellular matrix is diffusedly distributed in the tissue. Such a three-dimensional synthetic tissue is implantable and has tissue strength capable of being used in clinical applications. Such a three-dimensional synthetic tissue is also characterized in being scaffold-free. When mesenchymal stem cells are used in the present invention, mesenchymal stem cells to be used may be obtained from an actual tissue. It is also possible to use less differentiated stem cells such as those differentiated from ES cells or iPS cells.

In an alternative embodiment, the three-dimensional synthetic tissue of the present invent ion may employ heterologous cells, allogenic cells, isogenic cells or autologous cells. In the present invention, it is found that even when allogenic cells, and particularly mesenchymal cells are used, no adverse reactions, such as immune rejection reactions, is generated. Thus, the present invention lends to the development of the treatment of ex *vivo,* and also a therapy which produces a synthetic tissue using cells of others and utilizes the tissue without using an immune rejection suppressor or the like.

In one preferred embodiment, cells included in the three-dimensional synthetic tissue used in the present invention may be stem cells, differentiation cells, or include both. In a preferred embodiment, cells included the three-dimensional synthetic tissue used in the present invention are mesenchymal cells. Although it is not desired to be constrained by theory, the mesenchymal cells are preferably used because the mesenchymal cells are highly compatible with organs such as bones, and may have capability to differentiate into various organs such as a tissue. Such mesenchymal cells may be mesenchymal stem cells, or may be mesenchymal differentiation cells. Cells from another system having the features of mesenchymal or those from undifferentiated cells (ES cells or iPS cells) may be used.

Examples of mesenchymal cells used in the present invention include bone marrow cells, adipocyte, synovial cell, myoblasts, and skeletal muscle cells . Examples of mesenchymal cells as used herein include stem cells derived from an adipose tissue, stem cells derived from a bone marrow, and stem cells differentiated from ES cells or iPS cells.

In the preferred embodiment, it is advantageous that cells used in the three-dimensional synthetic tissue used in the present invention are cells derived from the subject to which the composite tissue of the present invention is applied. In such a case, cells as used herein also are referred to as autologous cells. Immune rejection reactions can be prevented or reduced by using autologous cells. Alternatively, in another embodiment, cells used in the three-dimensional synthetic tissue as used herein may not be cells derived from a subject to which the composite tissue of the present invention is applied. In such a case, it is preferable that measures are taken to prevent immune rejection reactions.

In a preferable embodiment, the three-dimensional synthetic tissue used in the present invention has sufficient tissue strength for clinical applications. The sufficient tissue strength for clinical applications varies depending on a site to which the synthetic tissue is applied. Such strength can be determined by those skilled in the art by referring to the disclosure of the specification and techniques well known in the art. The tensile strength of the three-dimensional synthetic tissue used in the present invention may be low. The tensile strength becomes higher when the matrix concentration is increased, and becomes lower when the cell ratio is increased in the cell/extracellular matrix ratio. The present invention is characterized in that the strength can be freely adjusted as necessary. The present invention is also characterized in that the strength can set to be relatively high or low to approximate that of a tissue to be implanted. Therefore, it is understood that the goal can be set to comply with any site.

In another embodiment, it is preferable that strength of the three-dimensional synthetic tissue used in the present invention is sufficient for having a self-supporting ability. Conventional synthetic tissues do not have a self-supporting ability after production. Therefore, when synthetic tissues other than those from the technique of the present invention are transferred, at least a part of them are injured. However, when the technique of the present invention is used, a synthetic tissue having aself-supporting ability is provided. Preferable self-supporting ability is such that, when a tissue is picked up with a tweezers having tips with thickness of 0.5 to 3 mm (preferably, tips with thickness of 1 to 2mm, and more preferably, tips with thickness of 1 mm), the tissue is not substantially destroyed. Herein, whether the tissue is substantially destroyed can be confirmed by eyes, but can also be confirmed by performing, for example, a water leakage test after the tissue is picked up in the above-described conditions and confirming that water does not leak. Alternatively, the self-supporting ability as described above can also be confirmed by not being destroyed when picked up by fingers instead of tweezers. In a particular embodiment of the present invention, portions to which clinical application is intended include a bone, a joint, a cartilage, a meniscus, a tendon, and a ligament. The origin of cells contained in the synthetic tissue of the present invention is not affected by clinical applications. Further, when a site of defect is a cartilage portion, the attachment ability of the synthetic tissue can be tested by determining whether the synthetic tissue remains attached without an artificial fixation procedure when the synthetic tissue is implanted into a defect portion of the intra-articular tissue (e.g., 2, 3 minutes later).

The three-dimensional synthetic tissue used in the present invention is an implantable synthetic tissue. Attempts have been heretofore made to produce synthetic tissues by cell culture. However, there were no synthetic tissues suitable for implantation in terms of size, strength, physical injuries when it is detached from a culture container, or the like. The three-dimensional synthetic tissue used in the present invention is provided by utilizing a tissue culture method as described above as an implantable synthetic tissue. The three-dimensional synthetic tissue used in the present invention provides a complex comprising a cell and a component derived from the cell. Herein, it is understood that, preferably, the complex substantially is substantially made of cells and the components derived from the cells. Herein, the complex of the present invention is provided for reinforcing, repairing, or regenerating a part of an organism. As used herein, the term "complex" means that cells and other components are integrated into a complex by some kind of interaction. Therefore, the complex of the present invention often has an appearance like a synthetic tissue, and it is understood that the meaning of the term "complex" overlaps with what is referred to by a synthetic tissue. It should be noted that "complex" itself is a synthetic tissue, which is different from a "composite tissue". A "complex" can be one component of a "composite tissue" of the present invention.

In another embodiment, the three-dimensional synthetic tissue used in the present invention is preferably isolated. In this case, the term "isolate" means that the three-dimensional synthetic tissue is separated from a scaffold, a support, and a culture medium used in culture and separated from anything other than an artificial bone used in a composite tissue. The three-dimensional synthetic tissue used in the present invention is substantially free of materials such as a scaffold so that it is possible to suppress adverse reactions after implantation, such as immune rejection reactions or inflammation reactions. The base area of the composite tissue of the present invention and thus the three-dimensional synthetic tissue included therein may be, for example, 1 cm² to 20 cm². However, the area may be less than 1cm² or greater than 20cm². It is understood that the essential feature of the present invention is that a tissue of any size (area, volume) can be produced, and it is not limited in the size.

In a preferable embodiment, the three-dimensional synthetic tissue used in the present invention is thick. The term "thick" typically means that the three-dimensional synthetic tissue has a thickness which provides strength sufficient to cover a site to which the synthetic tissue is implanted. Such a thickness is, for example, at least about 50 µm, more preferably at least about 100 µm, at least about 200 µm, at least about 300 µm, even more preferably at least about 400 µm, still more preferably at least about 500 µm, and still even more preferably about 1 mm. It is understood that, in some cases, a tissue having a thickness of 3 mm or greater and a tissue having a thickness of 5 mm or greater can also be produced. Alternatively, such a thickness may be less than 1 mm. It is understood that an essential feature of the present invention is that a tissue or a complex having any thickness can produced, and the tissue or complex is not limited in size.

The three-dimensional synthetic tissue used in the present invention provides a scaffold-free synthetic tissue. By providing such a scaffold-free synthetic tissue, a therapeutic method and a therapeutic agent for providing an excellent condition after implantation can be obtained. The three-dimensional synthetic tissue used in the present invention solves a long outstanding problem with biological formulations, which is attributed to contamination of the scaffold itself by utilizing a scaffold-free synthetic tissue. Despite the absence of a scaffold, the therapeutic effect is comparable with or more satisfactory than conventional techniques. In addition, when a scaffold is used, the alignment of implanted cells in the scaffold, the cell-to-cell adhesion, in vivo alteration of the scaffold itself (eliciting inflammation), and acceptance of the scaffold to recipient tissue become problematic. However, these problems can be solved by the present invention. In particular, in the present invention, usefulness of use of a synthetic tissue in cartilage regeneration is directly passed on to use of a proven composite tissue having an artificial bone with usefulness and safety that are already proven as bone regenerating implant in terms of preferably using an actual bone component and being free of biological formulation and synthetic polymer. This is recognized as an advantageous point in comparison to conventional synthetic tissue and composite tissue. The three-dimensional synthetic tissue used in the present invention is also different from methods using conventional cell therapy in that the three-dimensional synthetic tissue is self-organized and biologically integrated inside. It is easy to form a three-dimensional structure with the three-dimensional synthetic tissue used in the present invention, and thus it is easy to design it into a desired form. The versatility thereof should be noted. Further, treatment of sites that could not be considered for implantation treatment with conventional synthetic products is made possible. The synthetic tissue of the present invention has biological integration within tissues and with the environment and actually works in implantation therapies. The composite tissue of the present invention has biological integration capability with surrounding tissues and cells (preferably by extracellular matrix). Therefore, the post-operational acceptance is satisfactory. Thus, the composite tissue of the present invention provides medical treatment which provides a therapeutic effect by filling, replacing, and/or covering an affected portion.

The three-dimensional synthetic tissue used in the present invention has biological integration with the environment after implantation, such as surrounding tissues and cells. Therefore, excellent results are achieved such as the post-operational acceptance is satisfactory and cells are reliably supplied. An effect of the present invention is that the satisfactory biological integration allows the formation of a composite tissue with another synthetic tissue or the like, thus enabling a more complex therapy. Another effect of the three-dimensional synthetic tissue used in the present invention is that differentiation can be induced after a tissue is provided as a three-dimensional synthetic tissue. Alternatively, differentiation is induced so that such a three-dimensional synthetic tissue can be formed before providing a three-dimensional synthetic tissue. Another effect of the three-dimensional synthetic tissue used in the present invention is that the cell implantation achieves an effect such as satisfactory replacement ability and a comprehensive supply of cells, compared to conventional cell-only implantation and sheet implantation. The composite tissue of the present invention provides an implantable synthetic tissue that has biological integration capability by a three-dimensional synthetic tissue used in the present invention. The above-described features and effects of such a tissue make it possible to treat a site which could be considered as an implantation site for conventional synthetic products. The three-dimensional synthetic tissue used in the present invention has biological integration in tissues and between other tissues and actually works in implantation therapies. The synthetic tissue is not provided by conventional techniques and is first provided by the present invention. The three-dimensional synthetic tissue used in the present invention has biological integration capability with surrounding tissues and cells after implantation, thus having excellent post-operation results. A synthetic tissue having such biological integration capability does not exist outside of the method used in the present invention. Thus, the present invention achieves a therapeutic effect that could not be accomplished with a synthetic tissue by a method other than this method. The composite tissue of the present invention provides medical treatment which provides a therapeutic effect by filling, replacing, and/or covering an affected portion.

In a preferred embodiment, a three-dimensional synthetic tissue used in the present invention is biologically integrated in third dimensional directions. The three-dimensional synthetic tissue is in an adhering state in the integration with an artificial bone, which is essentially recognized as a close adhesion. In this regard, biological integration is explained in other portions of the present specification. For example, biological integration includes physical integration by an extracellular matrix and electric integration. It is particularly important that an extracellular matrix within a tissue is biologically integrated. A synthetic tissue in such a biologically integrated state is not provided by a method other than the method used by the present invention. Furthermore, in a preferred embodiment of having biological integration capability with the surrounding, a synthetic tissue is recognized as achieving a significant effect in terms providing a composite tissue comprising a synthetic tissue capable of constituting a part of a living body even after implantation. The present invention can provide a composite tissue comprising a synthetic tissue which is first frozen to kill cells so that cells are not really included. Such a tissue is still unique in that there is property to adhere to the surrounding even in such a case.

An extracellular matrix or a cell adhesion molecule, such as fibronectin or vitronectin, is distributed throughout the three-dimensional synthetic tissue used in the present invention. In the cell sheet engineering, a cell adhesion molecule is in contrast localized on a surface of culture cells which is attached to a Petri dish. In the sheet provided by the cell sheet engineering, cells are the major component of the sheet, which is the biggest difference therebetween. The sheet is recognized as a mass of cells with glue of an adhesion molecule attached on the bottom surface. The synthetic tissue of the present invention, however, is literally a "tissue" such that an extracellular matrix surrounds cells. Thus, the present invention is significantly distinguished from conventional techniques. The present invention achieves improvement in acceptance of another synthetic tissue such as an artificial bone.

In one embodiment, the three-dimensional synthetic tissue used in the present invention is different from conventional synthetic tissues in that the former comprises a cell. Particularly, it should be noted that cells can be included at a high density, i.e., a maximum cell density of 5×10⁶/cm². The present invention is noteworthy in that it is suitable for implanting cells rather than implanting a tissue.

A representative cell implanting method without a scaffold is a cell sheet engineering technique described in Non Patent Document 27 using a temperature sensitive culture dish , which is internationally acclaimed due to its originality. However, a single sheet obtained by such a sheet engineering technique is fragile in many cases. In order to obtain the strength that can withstand surgical manipulation, such as implantation, a plurality of sheets need to be stacked, for example. Furthermore, there were portions where integration condition is poor and hardly can be recognized as complete recovery when progress after the operation is observed. The composite tissue of the present invention solves such an issue.

A three-dimensional synthetic tissue used in the present invention does not require a temperature sensitive culture dish unlike the cell sheet technique. It is characterized in being easy for the cell/matrix complex to form multilayers. There is no technique that can produce a multi-layer complex having 10 or more layers without using so-called feeder cells, such as rodent stroma cells, in about three weeks. By adjusting conditions for matrix synthesis of material cells such as synovial cells, it is possible to produce a complex having a strength which allows surgical manipulation, such as holding or transferring the complex, without a special instrument. Therefore, the composite tissue of the present invention is innovative in enabling a custom-made therapy that can be varied depending on the circumstances.

Recovery has been observed (bone and cartilage formation has been observed) in rabbits one month from a surgical operation, and significant degree of repair was observed at two months in the present invention. Thus, it is possible to achieve a quick and more complete heeling, which was not possible in the convention therapeutic methods. That is, the characteristic effect of the present invention is the speed of integration and heeling (data for 2 months) in comparison to conventional methods. In a rabbit model, natural healing is observed after about 6 months. However, there was a significant difference in the level and quality of healing such as integration at 6 months. Thus, the present invention is recognized as capable of achieving a quick and more complete healing that could not be achieved by conventional therapeutic methods. Since the present invention is proven in rabbits, said rabbits are established models for other animals such as humans. For humans, those skilled in the art can understand that a similar effect is achieved in "mammals" in general because the above results are proven examples with rabbits, which are established models in osteochondral defect treatment. The following references can be referred for such animal models, constituting the common general knowledge in the art.

### <Examples of references of conventional techniques in animal models>

*F. Berenbaum, The OARSI histopathology initiative - the tasks and limitations, Osteoarthritis and Cartilage 18 (2010) S1
*Trattnig S, Winalski CS, Marlovits S, Jurvelin JS, Welsch GH, Potter HG. Magnetic resonance imaging of cartilage repair: a review. Cartilage. 2011; 2:5-26.
*Mithoefer K, Saris DBF, Farr J, Kon E, Zaslav K, Cole B, Ranstam J, Yao J, Shive MS, Brittberg M. Guidelines for the design and conduct of clinical studies in knee articular cartilage repair: International Cartilage Repair Society recommendations based on current scientific evidence and standards of clinical care. Cartilage. 2011; 2: 100-121.
*Roos EM, Engelhart L, Ranstam J, Anderson AF, Irrgang JJ, Marx R, Tegner Y, Davis AM. Patient-reported outcome instruments for use in patients with articular cartilage injuries. Cartilage. 2011; 2:122-136.
*Hurtig M, Buschmann MD, Fortier L, Hoemann CD, Hunziker EB, Jurvelin JS, Mainil-Varlet P, McIlwraith W, Sah RL, Whiteside RA. Preclinical studies for cartilage repair: recommendations from the International Cartilage Repair Society. Cartilage. 2011; 2:137-153.
*Hoemann CD, Kandel R, Roberts S, Saris D, Creemers L, Manil-Varlet P, Methot S, Hollander A, BuschmannMD. Recommended guidelines for histological endpoints for cartilage repair studies in animal models and clinical trials. Cartilage. 2011; 2:154-173.
*C. Wayne McIlwraith and David D. Frisbie, Microfracture: Basic Science Studies in the Horse, Cartilage 2010 1: 87-95
*F. Berenbaum, The OARSI histopathology initiative - the tasks and limitations Osteoarthritis and Cartilage 18 (2010) S1
*T. Aigner, J. L. Cook, N. Gerwin x, S.S. Glasson k, S. Laverty, C.B. Little, W. McIlwraith, V.B. Kraus, Histopathology atlas of animal model systems e overview of guiding principles Osteoarthritis and Cartilage 18 (2010) S2-S6
*K.P.H. Pritzker, T. Aigner, Terminology of osteoarthritis cartilage and bone histopathology - a proposal for a consensus Osteoarthritis and Cartilage 18 (2010) S7-S9
*R. Poole, S. Blake, M. Buschmann, S. Goldring, S. Laverty S. Lockwood, J. Matyas, J. McDougall, K. Pritzker, K. Rudolphi, W. van den Berg, T. Yaksh, Recommendations for the use of preclinical models in the study and treatment of osteoarthritis, Osteoarthritis and Cartilage 18 (2010) S10-S16
*S.S. Glasson, M.G. Chambers, W.B. Van Den Berg, C.B. Little, The OARSI histopathology initiative e recommendations for histological assessments of osteoarthritis in the mouse, Osteoarthritis and Cartilage 18 (2010) S17-S23
*N. Gerwin, A.M. Bendele, S. Glasson, C.S. Carlson, The OARSI histopathology initiative - recommendations for histological assessments of osteoarthritis in the rat, Osteoarthritis and Cartilage 18 (2010) S24-S34
*V.B. Kraus, J.L. Huebner, J. DeGroot, A. Bendele, The OARSI histopathology initiative - recommendations for histological assessments of osteoarthritis in the guinea pig, Osteoarthritis and Cartilage 18 (2010) S35-S52
*S. Laverty, C.A. Girard, J.M. Williams, E.B. Hunziker, K.P.H. Pritzker, The OARSI histopathology initiative - recommendations for histological assessments of osteoarthritis in the rabbit, Osteoarthritis and Cartilage 18 (2010) S53-S65
*J.L. Cook, K. Kuroki, D. Visco, J.-P. Pelletier, L. Schulz, F.P.J.G Lafeber, The OARSI histopathology initiative - recommendations for histological assessments of osteoarthritis in the dog, Osteoarthritis and Cartilage 18 (2010) S66-S79
*C.B. Little, M.M. Smith, M.A. Cake, R.A. Read, M.J. Murphy, F.P. Barry, The OARSI histopathology initiative - recommendations for histological assessments of osteoarthritis in sheep and goats, Osteoarthritis and Cartilage 18 (2010) S80-S92
*C.W. McIlwraith, D.D. Frisbie, C.E. Kawcak, C.J. Fuller, M. Hurtig, A. Cruz, The OARSI histopathology initiative - recommendations for histological assessments of osteoarthritis in the horse, Osteoarthritis and Cartilage 18 (2010) S93-S105
*P.C Pastoureau, E.BHunziker, J.-P. Pelletier, Cartilage, bone and synovial histomorphometry in animal models of osteoarthritis, Osteoarthritis and Cartilage 18 (2010) S106-S112
*N. Schmitz, S. Laverty, V.B. Kraus, T. Aigner, Basic methods in histopathology of joint tissues, Osteoarthritis and Cartilage 18 (2010) S113-S116
*G.L. Pearce, D.D. Frisbie, Statistical evaluation of biomedical studies, Osteoarthritis and Cartilage 18 (2010) S117-S122

In a preferable embodiment, the three-dimensional synthetic tissue used in the present invention has a biological integration capability to the surroundings. As used herein, the term surroundings refers to the implanted environment, and typical examples thereof include tissues and cells. The biological integration capability of surrounding tissues and cells can be confirmed by photomicrograph, physical test, or staining of a biological marker. Conventional synthetic tissues have a low affinity for tissues in implanted environment. It was not even assumed that conventional synthetic tissues can exhibit the biological integration capability. Conventional synthetic tissues depend on a regeneration capability of an organism, and serves as a temporary solution until autologous cells or the like gather and regenerate. Thus, these conventional synthetic tissues are not intended for a permanent use. Therefore, the composite tissue of the present invention should be deemed capable of constituting an implantation treatment in the true sense. The biological integration capability mentioned in the present invention preferably includes an adhesion capability to surrounding cells and/or extracellular matrices. Such an adhesion capability can be measured by an *in vitro* culturing assay with a tissue section (e.g., a cartilage section). In addition, it is demonstrated that sufficient biological integration capability is exerted and a therapeutic effect with excellent integration condition was achieved at a level that could not be achieved in conventional art by using a composite tissue of the present invention. In a preferred embodiment, the synthetic tissue or complex of the present invention has biological integration in all three dimensional directions. There are some synthetic tissues prepared by conventional methods, which have biological integration in two dimensional directions to some degree. However, no tissue having biological integration in all three dimensional directions is prepared by conventional methods. Therefore, since the three-dimensional synthetic tissue used in the present invention has biological integration in all three dimensional directions in this manner, the three-dimensional synthetic tissue is provided with a property of being substantially implantable in any application. Examples of biological integration to be an index in the present invention include interconnection of extracellular matrices, electrical integration, and the presence of intracellular signal transduction. The interaction of extracellular matrices can be observed with a microscope by staining intracellular adhesion as appropriate. Electrical integration can be observed by measuring electric potential.

In one embodiment, an artificial bone that can be used in a composite tissue of the present invention may be made of a material selected from the group consisting of hydroxyapatite and β-tricalcium phosphate.

In a preferred embodiment, an artificial bone that is included in a composite tissue of the present invention is preferably smaller in size than a depth of a defect of a bone section in the osteochondral defect. Although it is not desired to be constrained by theory, since it was found that this size creates a margin for a cartilage to form in a defect portion so that a bone/cartilage undergoes smooth biological integration, it is believed that it may be preferable to specify the size in such relation of the depth of the defect for improvement in therapeutic results. As a representative example, when an osteochondral defect is about 6 mm in a small animal such as a rabbit, a cartilage portion would be about 300-400 pm. Thus, the conditions would be met if the depth of an artificial bone is about 4 mm and a three-dimensional synthetic tissue (TEC) is about 0.5-2 mm. The thickness of a cartilage varies by the animal. In humans, it is understood to be about 1-5 mm depending on the site. Thus, when a cartilage portion is about 3 mm, a composite tissue can be produced by adding a three-dimensional synthetic tissue (TEC) deeper than about 3 mm, e.g., about 4 mm from the depth of an osteochondral defect (mm) to an artificial bone with a length that is about 4mm less than the depth of a defect. Alternatively, as another embodiment, it is possible to implant at a constant depth irrespective of the thickness of a cartilage. In one embodiment, the artificial bone is smaller in size than a depth of a defect of a bone section in the osteochondral defect by about l mm or greater. In another embodiment, the artificial bone is smaller in size than a depth of a defect of a bone section in the osteochondral defect by twice the thickness of a cartilage or less. In yet another embodiment, the artificial bone is smaller in size than a depth of a defect of a bone section in the osteochondral defect by about 1 mm or greater and twice the thickness of a cartilage or less. Thus, for example, a boundary surface of a TEC/artificial bone complex is preferably at a position that has an addition 1-6 mm in depth from a native bone/cartilage boundary in humans. Preferably, the artificial bone is advantageously smaller than a depth of a defect of a bone section in the osteochondral defect by about 2 mm to about 4 mm. When shallow as in about 2 mm, a subchondral bone is repaired quickly, but a cartilage is poorly repaired. When deep as in about 4 mm, a cartilage is repaired well, but the repair of subchondral bone is prolonged. Thus, although it depends of the case, in one embodiment, it is preferable to be about 3mm from the surface layer of a cartilage. Forexample, a cartilage portion is 1-5 mm in humans, and typically 3 mm. Thus, in a typical case, an artificial bone is preferably about 1 mm as the minimum in terms of depth from the bone/cartilage boundary. Thus, it is preferable to use a depth of about 1 mm as the lower limit and about 6 mm, which is twice the cartilage portion, as the upper limit. In this case, if a defect of a bone portion is presumed to be about 10 mm, a portion of an artificial bone that is used would be about 9 mmm to about 4 mm. Thus, since the thickness of an adjacent cartilage is approximately constant by the animal in this embodiment, a composite tissue can be suitably sized from the depth of a bone portion depending on the target animal. For example, it is known that the thickness of a joint cartilage is about 1 mm to about 5mm for the largest patella cartilage in humans. In addition, it is known to vary by site. Thus, the thickness of a cartilage can be determined in accordance with the site. The cartilage thickness is known in the art for animals other than humans and rabbits. Thus, it is possible to determine the cartilage thickness by referring to references described in <Examples of references of conventional techniques in animal models> and other well-known references. A composite tissue of the present invention can be structured in accordance with such thickness.

In another preferred embodiment, it is preferred that the total of depths of the artificial bone and the three-dimensional synthetic tissue is nearly the same as a depth of the osteochondral defect. A tolerance in the total depth (length) can be allowed. For example, a tolerance of about 1 mm can be deemed as approximately the same. However, it is preferable that the total depth is no longer than the depth of an osteochondral defect (e.g., the total of depths of an artificial bone and a three-dimensional synthetic tissue is the same or about 1 mm shorter than the depth of the osteochondral defect) . Although it is not desired to be constrained by theory, since it was found that this size creates a margin for a cartilage to form in a defect portion so that a bone/cartilage undergoes smooth biological integration, it is believed that it may be preferable to specify the size in such relation of the depth of the defect for improvement in therapeutic results. More preferably, the artificial bone is smaller in size than a depth of a defect of a bone section in the osteochondral defect, and the total of depths of the artificial bone and the three-dimensional synthetic tissue is nearly the same as the depth of the osteochondral defect. This is because both of the advantages are utilized by combining both of these features.

In a preferred embodiment, a three-dimensional synthetic tissue and an artificial bone are diphasic in a composite tissue of the present invention. That is, in a preferred embodiment, these two components are preferably unmixed and are present as substantially separate constituent elements in a composite tissue of the present invention. Thus, as used herein, the term "biphasic" refers to two or more components being unmixed and present as substantially separate constituent elements. Although it is not desired to be constrained by theory, it is demonstrated that subchondral bone formation is promoted thereby in a three-dimensional synthetic tissue.

In one embodiment, the three dimensional synthetic tissue and the artificial bone are attached to each other. The three-dimensional synthetic tissue used in the present invention can adhere or closely adhere to another synthetic tissue such as an artificial bone (including, for example, those made of calcium phosphate, hydroxy apatite or the like) and provided in an integrated form as a composite tissue. In this manner, it is understood that the composite tissue of the present invention comprises a feature that is helpful in improving treatment results after implantation.

It is understood that a composite tissue of the present invention can be used in any animal having an osteochondral defect in an osteochondral tissue. In one embodiment, an example of such an animal is a mammal. In particular, it was difficult for an osteochondral defect of primates including humans to completely heel. Thus, it should be noted that the present invention can achieve a significant effect in terms of achieving a complete recovery or a condition close thereto.

Anything that is known as a bone substitute can be used as an artificial bone used in the present invention. For example, it is possible to use a material that has affinity to bones in a body, such as hydroxyapatite, β-tricalcium phosphate, silicon, bioceramics such as carbon, alumina, or zirconia, metals such as titanium or tungsten, and materials made of materials such as coral materials. A representative examples thereof include porous β-tricalcium phosphate (β-TCP) (Olympus), hydroxyapatite synthetic bone substitute material NEOBONE^{®} (MMT Co., Ltd.), Apacerum, Superpore, Cellyard, Biopex-R, Bonetite, and Bonefill (each from Hoya Pentax) .

It is understood that the present invention targets any disease, disorder or condition associated with osteochondral disorders for treatment or prevention. Examples of such a disease, disorder and condition especially include any disease involving osteochondral degeneration, necrosis or injury, including osteoarthritis, osteochondral injury, osteonecrosis, intractable fracture, bone tumor and other similar diseases (bone cyst), osteochondral lesion, bone necrosis, rheumatoid arthritis, cartilage injury (cartilage full thickness injury, cartilage partial injury, osteochondral injury), meniscus injury, ligament injury, conditions requiring ligament repair (chronic injury, degenerative tear, biological augmentation for reconstructive surgery, etc.), tendon injury, conditions requiring tendon (including Achilles' heel) repair (chronic injury, degenerative tear, biological augmentation for reconstructive surgery, etc.), cartilage degeneration, meniscus degeneration, intervertebral disk denaturation, ligament degeneration, or tendon degeneration, delayed union; nonunion; skeletal muscle repair/regeneration and any other diseases with injury to tissue or faulty union of a bone and body inserts such as artificial joint.

As used herein, the term "prophylaxis" or "prevention" in relation to a certain disease or disorder refers to a treatment which keeps such a condition from happening before the condition is induced, or causes the condition to occur at a reduced level or to be delayed.

As used herein, the term "therapy" in relation to a certain disease or disorder means that when such a condition occurs, such a disease or disorder is prevented from deteriorating, preferably is retained as it is, more preferably is diminished, and even more preferably extinguished. As used herein, the term "radical therapy" refers to a therapy which eradicates the root or cause of a pathological process. Therefore, when a radical therapy is performed for a disease, there is no recurrence of the disease in principle.

As used herein, the term "prognosis" is also referred to as "prognostic treatment". The term "prognosis" in relation to a certain disease or disorder refers to a diagnosis or treatment of such a condition after a therapy.

The composite tissue of the present invention may be provided as a drug. Alternatively, the composite tissue of the present invention may be prepared by a physician in clinical settings, or a physician may first prepare the cells, and then a third party may culture the cells for preparation as a three-dimensional synthetic tissue to attach to an artificial bone for use in a surgery. In such a case, culturing cells is not necessarily performed by a physician, but can be performed instead by those skilled in the art of cell culture. Thus, those skilled in the art can determine culturing conditions in accordance with the type of cells and intended implantation site after reading the disclosure herein.

In terms of another perspective, the present invention provides a composite tissue comprising a scaffold-free three dimensional synthetic tissue. By providing such a composite tissue comprising a scaffold-free synthetic tissue, a therapeutic method and a therapeutic agent for providing an excellent condition after implantation can be obtained.

In terms of another perspective, a composite tissue comprising a scaffold-free synthetic tissue solves a long outstanding problem with biological formulations, which is attributed to contamination of the scaffold itself. Despite the absence of a scaffold, the therapeutic effect is comparable with, or more satisfactory than conventional techniques. When a scaffold is used, the alignment of implanted cells in the scaffold, the cell-to-cell adhesion, in vivo alteration of the scaffold itself (eliciting inflammation), and the integration of the scaffold to recipient tissue become problematic. However, these problems can be solved by the present invention. The three-dimensional synthetic tissue used in the present invention is also self-organized and have biological integration inside thereof. Thus, the present invention is distinguished from conventional cell therapies in terms of these points. It is easy to form a three-dimensional structure with the three-dimensional synthetic tissue used in the present invention. In addition, it is easy to design it into a desired form. Thus, the versatility of the three-dimensional synthetic tissue used in the present invention is noteworthy. The three-dimensional synthetic tissue used in the present invention has biological integration with the post-implantation environment, such as adjacent tissues and cells. Therefore, excellent effects are achieved, e.g., the post-operational stability is satisfactory and cells are reliably supplied. The satisfactory biological integration capability from use of the present invention results in very satisfactory adhesion upon the formation of a composite tissue with another synthetic tissue such as an artificial bone, thus enabling in a complicated therapy by using a composite tissue of the present invention. Another effect of the composite tissue of the present invention is that differentiation can be induced after providing as a composite tissue of the present invention. Alternatively, differentiation can be induced before providing as a composite tissue of the present invention to form such a composite tissue of the present invention. In terms of cell implant, the present invention provides effects such as satisfactory replacement ability and a comprehensive supply of cells by filling or covering, compared to conventional cell-only implantation and sheet implantation.

The three-dimensional synthetic tissue used in the present invention is free of injury caused by a protein degradation enzyme, such as, representatively, dispase, trypsin, or the like, during culture. Therefore, the three-dimensional synthetic tissue can be recovered as a cell mass with strength for holding proteins between cells, between cell and extracellular matrix, and extracellular matrices. The three-dimensional synthetic tissue also retains functions intact as a cell-extracellular matrix complex as a three-dimensional structure. When typical protein degradation enzymes such as trypsin are used, substantially no cell-to-cell link or cell-to-extracellular matrix link are retained, so that cells are individually separated. Among these protein degradation enzymes, dispase destroys most of basement membrane-like proteins between cells and base materials. In this case, the resultant synthetic tissue has weak strength.

The composite tissue of the present invention achieves a level of histological score related to cartilage repair and histological score related to subchondral bones (e.g., "O'Driscoll score related to bone layer reconstruction", "surface", "matrix", "exposure of subchondral bone", "alignment of subchondral bone", "biological integration of bone" (also referred to as "integration"), "bone infiltration into defect region", "hardening of cartilage" and "cell form" (cell distribution, survival rate of cell population)) that could not be achieved conventionally (see O' Driscoll SW, Keeley FW, Salter RB.,J Bone Joint Surg Am 1988; 70:595-606; Mrosek EH, Schagemann JC, Chung HW, Fitzsimmons JS, Yaszemski MJ, Mardones RM, et al., J Orthop Res 2010; 28:141-148;Olivos-Meza A, Fitzsimmons JS, Casper ME, Chen Q, An KN, Ruesink TJ, et al., Osteoarthritis Cartilage 2010; 18:1183-1191, Examples). Items that can be examined may be as follows. These items can be examined as to whether improvement is observed in comparison to prior art.

O'Driscoll score related to cartilage layer
Cell form
Matrix
Dye affinity of tissue
Continuity of surface layer
Continuity of tissue
Thickness of repaired tissue
Integration with host tissue
Cell density, Survival rate
Cartilage cell clustering ratio
Host tissue metamorphism

O'Driscoll score related to bone layer reconstruction
Surface
Matrix
Exposure of subchondral bone
Alignment of subchondral bone
Biological integration of bone
Bone infiltration into defect region
Cartilage calcification (tidemark formation)
Cell form
Cell distribution
Survival rate of cell population
Exposure of subchondral bone

### <Production method of composite tissue for therapy application>

In one aspect, the present invention provides a method for producing a composite tissue of the present invention, comprising positioning the three-dimensional synthetic tissue and the artificial bone so that the three-dimensional synthetic tissue and the artificial bone are in contact. The method of the present invention is advantageous in that a synthetic tissue, when positioned on an artificial bone, would immediately adhere thereto and become inseparable, whereby an inconvenient step as in a conventional composite tissue is not be required. It is understood that any form as described in (Composite tissue), (Production of three-dimensional synthetic tissue) and (Production kit of a composite tissue for therapeutic applications) described below can be used herein as a three-dimensional synthetic tissue and artificial bone. It is also understood that any form as described in (Composite tissue) can be used in the treatment or prevention of a disease, disorder, or condition associated with an osteochondral defect.

### (Production of three-dimensional synthetic tissue)

When producing a three-dimensional synthetic tissue used in the present invention, the period of time required for culture may be determined depending on the purpose of use of the composite tissue of the present invention. In order to detach and recover a cultured three-dimensional synthetic tissue from a support material, the cultured cell sheet or three-dimensional synthetic tissue can be detached directly or with a macromolecular membrane attached thereto. The three-dimensional synthetic tissue may be detached in culture medium in which cells have been cultured, or in other isotonic solutions. Such solutions may be selected depending on the purpose . When a monolayer cell sheet is prepared, examples of the macromolecular membrane, which is optionally attached to the cell sheet or three-dimensional synthetic tissue, include hydrophilized polyvinylidene difluoride (PVDF), polypropylene, polyethylene, cellulose and derivatives thereof, chitin, chitosan, collagen, paper (e.g., Japan paper), urethane, and net-like or stockinette-like macromolecular materials (e.g., spandex) . When a net-like or stockinette-like macromolecular material is employed, the composite tissue of the present invention has a higher degree of freedom, so that the contraction/relaxation function thereof can be further increased. A method for producing the three-dimensional structure of the present invention is not particularly limited. For example, the three-dimensional structure of the present invention can be produced by utilizing the above-described cultured cell sheet attached to a macromolecular membrane. A synthetic tissue that is substantially made of a cell and extracellular matrix derived from the cell cannot be produced by another method. Thus, the present invention is recognized as providing a composite tissue with a significant feature with respect to this point.

In a preferred embodiment, it is understood that placement of an extracellular matrix used in a three-dimensional synthetic tissue used in the present invention in a three-dimensional synthetic tissue used in the present invention can be readily achieved by a specific production method utilized in the present invention.

In order to detach and recover the three-dimensional synthetic tissue with a high yield from the cell culture support when producing the three-dimensional synthetic tissue used in the present invention, the cell culture support is tapped or shaken, or the medium is stirred with a pipette. These procedures may be performed alone or in combination. In addition, the three-dimensional synthetic tissue may be detached and recovered by deforming the base of the culture container or rinsing the container with isotonic solution or the like as needed. By stretching the three-dimensional synthetic tissue in a specific direction after being detached from the base material, the three-dimensional synthetic tissue is provided with alignment. Stretching may be performed by using a tensile device (e.g., Tensilon), or simply forceps, but the stretching method is not particularly limited. By providing alignment, it is possible to confer directionality to the motion of the three-dimensional synthetic tissue itself. This, for example, allows the three-dimensional synthetic tissue to move in accordance with the motion of a specific organ. The three-dimensional synthetic tissue can be efficiently applied to organs.

The methods disclosed in Japanese Patent NO. 4522994 can be appropriately referred with regard to the methods for producing a three-dimensional synthetic tissue used in the present invention. Although described in detail below, the present invention can utilize techniques besides those described below.

A (three-dimensional) synthetic tissue used in the present invention can be produced as follows. In summary, the producing method comprises the steps of: A) providing a cell; B) positioning the cell in a container containing a cell culture medium including an ECM synthesis promoting agent, wherein the container has a base with an area sufficient to accommodate a desired size of the synthetic tissue; and C) culturing the cell in the container for a period of time sufficient to form the synthetic tissue having the desired size.

The above-described cell may be any cell. A method for providing a cell is well known in the art. For example, a tissue is extracted and cells are isolated from the tissue. Alternatively, cells are isolated from body fluid containing blood cells or the like. Alternatively, a cell line is prepared in an artificial culture . Cells used herein may be any stem cells or differentiated cells, particularly myoblasts, mesenchymal stem cells, adipocytes, synovial cells, and bone marrow cells. Examples of mesenchymal stem cells used herein include adipose tissue-derived stem cells, bone marrow-derived stem cells, cells differentiated from iPS.

The method for producing a three-dimensional synthetic tissue used in the present invention employs a cell culture medium containing an ECM synthesis promoting agent. Examples of such an ECM synthesis promoting agent include ascorbic acid or a derivative thereof, ascorbic acid 2-phosphate, and L-ascorbic acid.

The cell culture medium used in the production method used in the present invention may be any medium which allows a cell of interest to grow. Examples of such a medium include DMEM, MEM, F12, DME, RPMI1640, MCDB104, 199, MCDB153, L15, SkBM, and Basal medium which are supplemented with glucose, FBS (fetal bovine serum) or human serum, antibiotics (penicillin, streptomycin, etc.).

The container used in production method used in the present invention may be any container typically used in the art which has a base with an area sufficient to accommodate a desired size of a synthetic tissue. Examples of such a container include petri dishes, flasks, and mold containers, and preferably containers having a large area of the base (e.g., at least 1 cm²). The material of the container may be any material including glass, plastic (e.g., polystyrene and polycarbonate, etc.), and silicone.

In a preferable embodiment, the production method used in the present invention may comprise detaching a produced (three-dimensional) synthetic tissue. As used herein, the term "detach" indicates that after a synthetic tissue of the present invention is formed in a container, the synthetic tissue is removed from the container. The detachment can be achieved by, for example, physical means (e.g., pipetting of medium) and chemical means (addition of a substance). In the present invention, a synthetic tissue can be detached by providing a stimulus around the synthetic tissue by physical means or chemical means, but not by aggressive means (e.g., treatment with a protein degradation enzyme) to the synthetic tissue. Thus, it should be noted that the present invention provides ease of handling, which cannot be conventionally achieved, and the resulting synthetic tissue is substantially intact, resulting in a high-performance implant.

In a preferable embodiment, the production method used in the present invention further comprises detaching cells which construct a synthetic tissue. In a more preferable embodiment, the detaching step can apply a stimulus for contracting a synthetic tissue, including a physical stimulus (e.g., pipetting). Such a physical stimulus is not directly applied to the produced synthetic tissue. This is a preferred feature of the present invention. This is because it is possible to suppress damage to the synthetic tissue by not directly applying a physical stimulus to a synthetic tissue. Alternatively, the detaching step includes chemical means, such as adding an actin regulatory agent. Such an actin regulatory agent includes a chemical substance selected from the group consisting of actin depolymerizing agents and actin polymerizing agents. Examples of actin depolymerizing agents include Slingshot, cofilin, CAP (cyclase associated protein), AIP1 (actin-interacting-protein 1), ADF (actin depolymerizing factor), destrin, depactin, actophorin, cytochalasin, and NGF (nerve growth factor). Examples of actin polymerizing agents include RhoA, mDi, profilin, Rac1, IRSp53, WAVE2, ROCK, LIM kinase, cofilin, cdc42, N-WASP, Arp2/3, Drf3, Mena, LPA (lysophosphatidic acid), insulin, PDGFa, PDGFb, chemokine, and TGF-β. Although it is not desired to be constrained by theory, these actin regulatory agents cause actomyocin-based cytoskeleton to contract or extend. It is believed that by regulating contraction and extension of a cell itself, as a result, a three-dimensional synthetic tissue itself may be promoted to or inhibited from being detached from the base of a container.

In another embodiment, the production method utilized in of the present invention is characterized in that they are produced from cells which are cultured in monolayer culture. Despite the cells being cultured in monolayer culture, synthetic tissues having various thicknesses can be constructed as a result. This is a significant effect. Conventionally, for example, a thick tissue cannot be constructed without using a multilayer structure when a temperature responsive sheet or the like is used. No other method can achieve a method for constructing a three-dimensional structure, which does not require feeder cells and can construct multilayer cells including ten or more layers . A typical cell implantation method which does not employ a scaffold is a cell sheet engineering technique utilizing a temperature sensitive culture dish in Non Patent Literature 27. The technique has won international recognition as an original technique. However, when using this cell sheet technique, a single sheet is weak in many cases, and requires modification such as layering sheets for obtaining the strength resistant to a surgical operation such as implantation.

A three-dimensional synthetic tissue used in a composite tissue of the present invention is a cell/matrix complex that does not require a temperature sensitive culture dish unlike the cell sheet technique. The cell/matrix complex is characterized in that it is readily formed into a multilayer tissue. There is no other technique is found, which can produce a multilayer complex having 10 or more layers without using so-called feeder cells, such as rodent stroma cells, in approximately three weeks. By adjusting conditions for matrix production of material cells such as synovial cell, it is possible to produce a complex having strength which allows surgical manipulation, such as holding or transferring the complex, without a special instrument. Therefore, the present invention is an original, ground-breaking technique for reliably and safely performing cell implantation.

In a preferable embodiment, the ECM synthesis promoting agent used in the production method used in the present invention includes ascorbic acid 2-phosphate (see Hata R., Senoo H., J. Cell Physiol., 1989, 138 (1) :8-16). In the present invention, by adding a certain amount or more of ascorbic acid 2-phosphate, it is possible to promote production of an extracellular matrix, so that the resultant three-dimensional synthetic tissue is hardened for easy detachment. Thereafter, self contraction is elicited by applying a stimulus for detachment. Hata et al. do not report that a tissue becomes strong and obtains a property of being readily detachable after adding such an ascorbic acid and culturing. Although it is not desired to be constrained by theory, a significant difference is that Hata et al. used a significantly different cell density. Hata et al. does not suggest an effect of making a tissue hard. Such an effect of the tissue being hardened, an effect of contraction, and an effect of the tissue becoming readily detachable are found in no other method. The synthetic tissue used in the composite tissue of the present invention can be recognized as totally different from the synthetic tissue which has been fabricated conventionally at least on the point that it is produced through the process of hardening, contraction, and detachment. Contraction when the culture is detached and promotion in constructing a three-dimensional structure, a multilayer tissue, and the like are surprising effects. Such effects have not been reported in any other method.

In a preferable embodiment, ascorbic acid 2-phosphate used in the production method utilized in the present invention typically has a concentration of at least about 0.01 mM, preferably at least about 0.05 mM, more preferably at least about 0.1 mM, even more preferably at least about 0.2 mM, still more preferably about 0.5 mM, and still even more preferably about 1.0 mM. Herein, any concentration of about 0.1 mM or higher may be employed. However, there may be an aspect in which a concentration of about 10 mM or lower is desired. In a certain preferable embodiment, the ECM synthesis promoting agent used in the production method utilized in the present invention includes ascorbic acid 2-phosphate or a salt thereof, and L-ascorbic acid or a salt thereof.

In a preferable embodiment, after the culturing step, the production method of the present invention further comprises D) detaching the synthetic tissue and allowing the synthetic tissue to perform self contraction. The detachment can be accelerated by applying a physical stimulus (e.g., application of physical stimulus (shear stress, pipetting, deformation of the container, etc.) to a corner of a container with a stick or the like). Self-contraction naturally takes place when a stimulus is applied after the detachment. When a chemical stimulus is applied, self-contraction and detachment occurs simultaneously. By self-contraction, biological integration is accelerated, particularly in the third dimensional directions (direction perpendicular to the two-dimensional directions in the case of tissue on a sheet). Therefore, a synthetic tissue of the present invention may take a form of a three-dimensional structure due to being produced in such a manner. In a production method utilized in the present invention, sufficient time preferably means at least 3 days, though it varies depending on the application of a synthetic tissue of interest. An exemplary period of time is 3 to 7 days.

In another embodiment, the production method utilized in the present invention may further comprise causing a synthetic tissue to differentiate. This is because a synthetic tissue can have a form closer to that of a desired tissue by differentiation. An example of such differentiation includes chondrogenesis and osteogenesis. In a preferable embodiment, osteogenesis may be performed in medium containing dexamethasone, β-glycerophosphate, and ascorbic acid 2-phosphate. More preferably, bone morphogenetic proteins (BMPs) are added. This is because such BMP-2, BMP-4, and BMP-7 promote osteogenesis.

In another embodiment, the production method utilized in the present invention is a process of differentiating a synthetic tissue. Examples of a form of differentiation include chondrogenesis. In the preferable embodiment, chondrogenesis may take place in a medium including pyruvic acid, dexamethasone, ascorbic acid 2-phosphate, insulin, transferrin, and selenious acid. More preferably, bone morphogenetic proteins (such as BMP-2, BMP-4, BMP-7, TGF-β1, or TGF-β3) are added. This is because such BMPs promote further chondrogenesis.

An important point in the production method utilized in the present invention is that it is possible to fabricate a tissue having a pluripotency into various differentiated cells such as a bone and cartilage. Conventionally, differentiation into a cartilage tissue is difficult in other synthetic tissues which are scaffold-free. If a certain size is required, in any other method, it was necessary to coculture with a scaffold, construct a three-dimensional structure, and add a chondrogenesis medium. Conventionally, scaffold-free differentiation into cartilage was difficult. The present invention enables differentiation into a cartilage in a synthetic tissue. This is an effect which has not been obtained in a method other than the methods utilizing the present invention, and is a characteristic effect of the present invention. In a cell treatment which aims to regenerate a tissue, a method for performing a treatment efficiently and safely by using a tissue of sufficient size without a scaffold was difficult. The present invention achieves a significant effect on this point. Particularly, the present invention is significant on the point that it becomes possible to easily manipulate differentiated cells such as cartilage, which has been impossible conventionally. In methods other than the methods of the present invention, for example, cells can be collected in a pellet shape and the aggregation of cells can be differentiated to obtain a tissue of about 2 mm³. For obtaining a tissue larger than this size, however, it was necessary to use a scaffold.

The differentiation step in the production method utilized in the present invention may be performed before or after providing the cells.

Primary culture cells can be used as cells in the production method utilized in the present invention. Subcultured cells (e.g., three or more passages) can also be used. Preferably, it is advantageous when subculture cells are used that the cells are preferably of four passages or more, more preferably of 5 passages or more, and even more preferably of 6 passages or more . It is believed that since the upper limit of cell density increases with an increase in the number of passages within a certain range, a denser synthetic tissue can be produced. It seems that a certain range of passages (e.g., 3 to 8 passages) are appropriate.

In the production method utilized in the present invention, cells are preferably provided at a cell density of 5.0×10⁴/cm² or more. This is because a synthetic tissue with greater strength can be provided by sufficiently raising the cell density. It is understood that the lower limit of the cell density may be lower than the above-described density. It is also understood that those skilled in the art can define the lower limit based on the present specification.

In one embodiment, for example, a myoblast, a synovial cell, an adipocyte, and a mesenchymal stem sell (e.g., derived from adipose tissue or bone marrow or ES cell or iPS cell) can be used. These cells can be applied to, for example, a bone, a cartilage, a tendon, a ligament, a joint, or a meniscus.

Another aspect of the production of a three-dimensional synthetic tissue utilized in the present invention can utilize a method for producing a three-dimensional synthetic tissue having a desired thickness. This method comprises: A) providing cells; B) positioning the cells in a container having a base area sufficient for accommodating a desired three-dimensional synthetic tissue having the desired size, which contains a cell culture medium containing anECM synthesis promoting agent (e.g., ascorbic acids, TGF-β1, or TGF-β3); C) culturing the cells in the container with the cell culture medium containing the ECM synthesis promoting agent for a time sufficient for forming the three-dimensional synthetic tissue having the desired size to convert the cells into a three-dimensional synthetic tissue; and D) adjusting the thickness of the three-dimensional synthetic tissue to obtain a desired thickness by a physical stimulation or a chemical stimulation. Herein, the steps of providing the cells, positioning the cells, stimulating and converting into the synthetic tissue or complex are described in detail in the specification such as in (Composite tissue) or the present section, and it is understood that any embodiment can be employed.

Next, examples of the physical or chemical stimulation to be used may include pipetting and use of actin interacting substance. Pipetting may be preferable because operation is easy and no harmful substance is produced. Alternatively, examples of the chemical stimulation to be used include actin depolymerizing factors and actin polymerizing factor. Examples of such an actin depolymerizing factor include ADF (actin depolymerizing factor), destrin, depactin, actophorin, cytochalasin, and NGF (nerve growth factor). Examples of the actin polymerizing factor include LPA (lysophosphatidic acid), insulin, PDGFa, PDGFb, chemokine, and TGFb. The polymerization or depolymerization of actin can be observed by checking the activity on actin. It is possible to test any substance whether it has such an activity. It is understood that a substance which is tested as such and identified can be used for achieving the desired thickness in production of the synthetic tissue of the present invention. For example, in the present invention, adjustment of the desired thickness can be achieved by adjusting the ratio of actin depolymerizing factor to actin polymerizing factor.

### (Production kit of composite tissue for therapeutic applications)

In one aspect, the present invention provides a kit for treating or preventing a disease, disorder, or condition associated with an osteochondral defect, comprising a three-dimensional synthetic tissue and an artificial bone. It is understood that any form as described in (Composite tissue) or (Production of three-dimensional tissue) can be used as a three-dimensional synthetic tissue and an artificial bone. It is understood that any form as described in (Composite tissue) can be used in the treatment or prevention of a disease, disorder or condition associated with an osteochondral defect.

In another aspect, a kit of the present invention can comprise a cell culture composition for producing a three-dimensional synthetic tissue from a cell instead of the three-dimensional synthetic tissue itself, i.e., the present invention is a kit for treating or preventing a disease, disorder, or condition associated with an osteochondral defect, comprising a cell culture composition for producing a three-dimensional synthetic tissue and an artificial bone. This is because it is possible to producing a three-dimensional synthetic tissue by using a cell culture composition so that an artificial bone attaches thereto by a kit of the present invention. The cell culture composition contains an ingredient (e.g., commercially available medium) for maintaining or growing the cell, and an ECM synthesis promoting agent. The ECM synthesis promoting agent has been described in detail in the above description of production method. Therefore, the ECM synthesis promoting agent includes ascorbic acid or a derivative thereof (e.g., TGF-β1, TGF-β3, ascorbic acid 1-phosphate or a salt thereof, ascorbic acid 2-phosphate or a salt thereof, or L-ascorbic acid or a salt thereof). The culture composition of the present invention contains ascorbic acid 2-phosphate or a salt thereof at a concentration of at least 0.1 mM. Alternatively, in the case of a condensed culture composition, the condensed culture composition contains ascorbic acid 2-phosphate or a salt thereof at a concentration which becomes at least 0.1 mM at preparation. It appears that 0.1 mM or greater that the effect of ascorbic acids barely changes at 0.1 mM or greater. Thus, 0.1 mM can be said to be sufficient. For TGF-β1 and TGF-β3, 1 ng/ml or more, or representatively 10 ng/ml, may be sufficient. The present invention may provide a composition for producing a three-dimensional synthetic tissue, comprising such an ECM synthesis promoting agent.

An ECM synthesis promoting agent used in the cell culture composition used in the present invention includes ascorbic acid 2-phosphate (see Hata R., Senoo H., J. Cell Physiol., 1989, 138(1):8-16). In the present invention, by adding at least a predetermined amount of ascorbic acid 2-phosphate, the production of an extracellular matrix is promoted. As a result, the resultant synthetic tissue or complex is hardened, and therefore, becomes readily detachable. Thereafter, the tissue undergoes self-contraction in response to a stimulus for detachment. Hata et al. do not report that the culture supplemented with ascorbic acid causes the tissue to harden and thus confers to the tissue a property of being easily detachable . Though not wishing to be bound by any theory, a significant difference between the present invention and Hata et al. is present in cell density used. Also, Hata et al. does not suggest the effect of hardening. In terms of such an effect of hardening and contraction and being readily detachable, the synthetic tissue produced by a kit of the present invention can be recognized as completely different from conventionally-produced synthetic tissues, since the synthetic tissue produced by a kit of the present invention is produced via the procedures of hardening, contraction and detachment.

In a preferable embodiment, ascorbic acid 2-phosphate used in a kit of the present invention is typically present at a concentration of at least 0.01 mM, preferably at least 0.05 mM, more preferably at least 0.1 mM, even more preferably at least 0.2 mM, and still more preferably at least 0.5 mM. Still even more preferably, the minimum concentration is 1.0 mM.

In one embodiment of the present invention, the cell density is, but is not particularly limited to, 5×10⁴ to 5×10⁶ cells per 1 cm². These conditions may be applied, for example, to myoblast. In this case, the ECM synthesis promoting agent is preferably provided ascorbic acids at a concentration of at least 0.1 mM. This is because a thick synthetic tissue can be produced. In this case, if the concentration is increased, a synthetic tissue having a dense extracellular matrix is produced. If the concentration is low, the amount of an extracellular matrix is decreased but the self-supporting ability is maintained.

### (Cartilaginous/osteochondral regeneration application)

In another aspect, the present invention provides a composite tissue for regenerating a cartilage, comprising a three-dimensional synthetic tissue and an artificial bone. It is understood that any form as described in (Composite tissue) or (Production of three-dimensional synthetic tissue) can be used as a three-dimensional synthetic tissue and an artificial bone. It is also understood that any form described in (Composite tissue) can be used for cartilage regeneration as needed.

Cartilage regeneration is recognized as a significant effect that could not be achieved by convention therapeutic methods in that a synthetic tissue alone can treat effectively.

In another aspect, the present invention provides a composite tissue for regenerating an osteochondral system, comprising a three-dimensional synthetic tissue and an artificial bone. It is understood that any form as described in (Composite tissue) or (Production of three-dimensional synthetic tissue) can be used as a three-dimensional synthetic tissue and an artificial bone. It is also understood that any form described in (Composite tissue) can be used for osteochondral system regeneration as needed.

An example of a significant point of osteochondral system regeneration is that implantation of a complex is better in comparison to that of a synthetic tissue alone. Further, it is preferable that an artificial bone remains little below the osteochondral boundary surface. Although it is not desired to be constrained by theory, this is because it was found that regeneration of a subchondral bone is promoted between the space between the osteochondral boundary and the surface of an artificial bone to yield significant therapeutic results in biological integration of cartilaginous portion.

In another aspect, the present invention provides a composite tissue for regenerating a subchondral bone, comprising a three-dimensional synthetic tissue and an artificial bone. It is understood that any form as described in (Composite tissue) or (Production of three-dimensional synthetic tissue) can be used as a three-dimensional synthetic tissue and an artificial bone. It is also understood that any form described in (Composite tissue) can be used for subchondral bone regeneration as needed.

Preferably, one of the features of the regeneration of cartilaginous or osteochondral system of the present invention is the cartilage integrating with an existing cartilage after regeneration. In a conventional therapy with only an artificial bone, integration with a cartilage after regeneration does not occur to regeneration in a form of a population of substantially different tissue fragments (bone fragments or cartilage fragments) . Thus, the present invention is significant in that the regeneration for substantial recovery to a condition prior to a defect is possible.

With regard to subchondral bone regeneration, it should be noted that a subchondral bone is efficiently formed directly above an artificial bone by implantation of the artificial bone and cartilage differentiation of undifferentiated cells in a synthetic tissue is subsequently promoted. Although it is not desired to be constrained by theory, this is a significant point because it is known that formation of cartilage bone significantly correlates with the extent of cartilage tissue formation within a synthetic tissue.

### (Therapy using composite tissue)

The references to the methods of treatment by therapy in this description are to be interpreted as references to compositions of the present invention for use in those methods".

In another aspect, the present invention provides a method of treating or preventing a disease, disorder, or condition associated with an osteochondral defect. The method comprises the steps of: A) positioning a composite tissue to replace or cover the defect; and B) holding the synthetic tissue or complex for a time sufficient for biological integration of the portion and the synthetic tissue of complex. It is understood that any form as described in (Composite tissue), (Production of three-dimensional synthetic tissue) or the like herein can be used as a "composite tissue" used in the present invention. It is also understood that any form described in (Composite tissue) or the like herein can be used for a disease, disorder, orcondition associated with osteochondral defect. Herein, to position a portion for replacement typically means to perform debridement or curetage of an affected portion as needed to position the composite tissue of the present invention on the affected portion, and to allow it to stand so as to promote replacement. An objective of such replacement is to fill with cells. Techniques known in the art can be combined and used. The step of positioning the synthetic tissue to cover a portion can be carried out using a technique well known in the art. The sufficient time varies depending on a combination of the portion and synthetic tissue, but can be easily determined as appropriate by those skilled in the art depending on the combination. Examples of such a time include 1 week, 2 weeks, 1 month, 2 months, 3 months, 6 months, and 1 year after an operation. In the present invention, a synthetic tissue preferably comprises substantially only cell (s) and material (s) derived from the cell. Therefore, there is no particular material which needs to be extracted after an operation. The lower limit of the sufficient time is not particularly important. Thus, it can be said that a longer time is preferable in this case. If the time is extremely long, it can be said that reinforcement is substantially completed. Therefore, there is no particularly need for a limit. The synthetic tissue of the present invention is also characterized in that it is easily handled, is not destroyed during an actual treatment, and facilitates a surgery due to its self-supporting ability.

Alternatively, the above-described portion may include a bone or cartilage. Examples of such portions include meniscus, ligament, and tendon. The method of the present invention may be utilized for treating, preventing or reinforcing a disease, injury, or condition of a bone, cartilage, ligament, tendon, or meniscus.

In another preferred embodiment, the reinforcement method of the present invention includes biological integration (e.g., interconnection of extracellular matrices, electrical integration, and intracellular signal transduction) that is mentioned with respect to a composite tissue of the present invention. The biological integration is preferably provided in all three dimensional directions.

In another preferred embodiment, the method of the present invention further comprises culturing a cell in the presence of an ECM synthesis promoting agent to form a composite tissue of the present invention. Such an implantation/regeneration technique which comprises the step of culturing in the presence of an ECM synthesis promoting agent had not been provided by conventional techniques. The method enables a therapy for diseases (e.g., cartilage injury or intractable bone fracture), which cannot be achieved by conventional therapies.

In a preferred embodiment, in the method of the present invention, the cell used in the composite tissue of the present invention is derived from an animal to which the composite tissue is to be implanted (i.e., an autologous cell). By using an autologous cell, side effects such as immune rejection reactions can be avoided.

Examples of targets of the therapeutic methods of the present invention include: cartilage full thickness injury, cartilage partial injury; osteochondral injury; osteonecrosis; osteoarthritis; meniscus injury; ligament injury (chronic injury, degenerative tear, biological augmentation for reconstruction surgery, etc.); tendon (includingAchillesheel) injury (chronic injury, degenerative tear, biological augmentation for reconstruction surgery, etc.); rotator cuff (particularly, chronic injury, degenerative tear, etc.); delayed union; nonunion; faulty union of a bone and body inserts such as artificial joint; and skeletal muscle repair/regeneration; cardiac muscle repair.

For some organs, it is said that it is difficult to radically treat a specific disease, disorder, or condition thereof for diseases caused by an osteochondral defect or the like. However, the present invention provides the above-described effect, thereby making it possible a treatment which cannot be achieved by conventional techniques. It has been clarified that the present invention can be applied to radical therapy. Therefore, the present invention has usefulness which cannot be achieved by conventional pharmaceutical agents.

Such an improvement in the condition by the method of the present invention can be determined in accordance with the function of the portion to be treated. In the case of a bone, for example, an improvement in the condition can be determined by measuring its strength or by evaluating bone marrow and/or a bone quality by using MRI. If a cartilage or meniscus should be treated, a surface of a joint can be observed by an arthroscopy. Further, it is possible to determine an improvement in condition by performing a biomechanical inspection under arthroscopy. It is also possible to determine an improvement in the condition by confirming a repairing state by using MRI. For ligaments, it is possible to determine by confirming the presence of lability by a joint stability inspection. Further, an improvement of the condition can be determined by confirming a continuousness of a tissue by an MRI. In the case of any tissue, it is possible to determine whether the condition is improved by performing a biopsy of the tissue and making a histological evaluation.

In a preferred embodiment, the treatment treats, prevents, or enhances a disease, injury, or condition of a bone, cartilage, ligament, tendon, or meniscus. Preferably, the composite tissue has a self-supporting ability. For such a composite tissue, those skilled in the art can use a composite tissue of any form described above herein or a variant thereof.

### (Combined therapy)

In another aspect, the present invention provides a regeneration therapy which uses a cytokine, such as BMP (e.g., BMP-2, BMP-4, and BMP-7), TGF-β1, TGF-β3, HGF, FGF, or IGF, in combination with a composite tissue of the present invention. It is understood that any form as described in the section of (Composite tissue) or the like herein can be used as a composite tissue or the like to be used.

Some cytokines used in the present invention are already commercially available (e.g., BMP (Yamanouchi Pharmaceutical), bFGF2 (Kaken Pharmaceutical), TGF-β1 (for research only), IGF (Fujisawa pharmaceutical), and HGF-101 (ToyoBoseki)). However, cytokines prepared by various methods can be used in the present invention if they are purified to an extent which allows them to be used as a medicament. A certain cytokine can be obtained as follows: primary cultured cells or an established cell line capable of producing the cytokine is cultured; and the cytokine is separated from the culture supernatant or the like, followed by purification. Alternatively, a gene encoding the cytokine is incorporated into an appropriate vector by a genetic engineering technique; the vector is inserted into an appropriate host to transform the host; a recombinant cytokine of interest is be obtained from the supernatant of the transformed host culture (see, Nature, 342, 440(1989); Japanese Laid-Open Publication No. 5-111383; Biochem-Biophys. Res. Commun., 163, 967 (1989), etc.). The above-described host cell is not particularly limited and examples thereof can include various host cells conventionally used in genetic engineering techniques, such as, *Escherichia coli,* yeast, and animal cells. The cytokine obtained in such a manner may have one or more amino acid substitutions, deletions and/or additions in the amino acid sequence as long as it has substantially the same action as that of naturally-occurring cytokine. Examples of a method for introducing the cytokine into patients in the present invention include a Sendai virus (HVJ) liposome method with high safety and efficiency (Molecular Medicine, 30, 1440-1448 (1993) ; Jikken Igaku (Experimental Medicine), 12, 1822-1826 (1994)), an electrical gene introduction method, a shotgun gene introduction method, and an ultrasonic gene introduction method. In another preferable embodiment, the above-described cytokines can be administered in the form of proteins.

Hereinafter, the present invention will be described by way of examples. Examples described below are provided only for illustrative purposes.

### [Examples]

In the present Examples, all procedures in this study were carried out in accordance with the Declaration of Helsinki . When applicable, experiments were conducted while handling animals in compliance with the rules set forth by the Osaka University.

### (Production Example 1: Production of three-dimensional synthetic tissue using synovial cells)

In this example, various synovial cells were used to produce a three-dimensional synthetic tissue as follows.

### <Preparation of cells>

Synovial cells were collected from a knee joint of a pig (LWD ternary hybrid, 2-3 months old upon removal of cells), followed by treatment with collagenase. The cells were cultured and subcultured in 10% fetal bovine serum+High Glucose-DMEM medium (FBS was obtained from HyClone, DMEM was obtained from GIBCO). It has been reported that 10th passage synovial cells still have pluripotency. Although cells of 10 or less passages were used in this example, it is understood that cells of more than 10 passages may be used depending on the application. Autotransplantation was performed for actual human implant, but it was necessary that a sufficient number of cells were secured and the cells were cultured for a short period of time so as to reduce the risk of infection or the like.

Considering these points, cells of various passages were used. Actually, primary culture cells, first passage cells, second passage cells, third passage cells, fourth passage cells, fifth passage cells, sixth passage cells, eighth passage cells, and tenth passage cells were used in experiments. These cells were used for use of synthetic tissues.

### <Preparation of synthetic tissue>

Synovial cells (4.0×10⁵) were cultured in 0.1-0.2 ml/cm² of 10% FBS-DMEM medium in a 35-mm dish, a 60-mm dish, 100-mm dish , 150-mm dish, 500-m dish, 6-well culture dish, 12-well culture dish, or 24-well culture dish (BD Biosciences, cell culture dish and multiwell cell culture plate). In this case, ascorbic acid was added. The dishes, the ascorbic acid and cell concentrations are described below.
*Dishes: BD Biosciences, cell culture dishes and multiwell cell culture plates
*Ascorbic acid 2-phosphate: 0 mM, 0.1 mM, 0.5 mM, 1 mM, 2 mM, and 5 mM
*The number of cells: 5×10⁴ cells/cm², 1×10⁵ cells/cm², 2.5×10⁵ cells/cm², 4.0×10⁵ cells/cm², 5×10⁵ cells/cm², 7.5x10⁵ cells/cm², 1×10⁶ cells/cm², 5×10⁶ cells/cm², and 1×10⁷ cells/cm²

Medium was exchanged two times per week until the end of a predetermined culture period. At the end of the culture period, a cell sheet was detached from the dish by pipetting circumferentially around the dish using a 100-µl pipette. After detachment, the cell sheet was made as flat as possible by lightly shaking the dish. Thereafter, 1 ml of medium was added to completely suspend the cell sheet. The cell sheet was allowed to stand for two hours, resulting in the contraction of the cell sheet into a three-dimensional form. In this manner, a synthetic tissue was obtained.

### <Hematoxylin-Eosin (HE) Staining>

The acceptance or vanishment of support in cells was observed by HE staining. The procedure is described as follows. A sample is optionally deparaffinized (e.g. , with pure ethanol), followed by washing with water. The sample is immersed in Omni's hematoxylin for 10 min. Thereafter, the sample is washed with running water, followed by color development with ammonia in water for 30 sec. Thereafter, the sample is washed with running water for 5 min and is stained with eosin hydrochloride solution (10 × diluent) for 2 min, followed by dehydration, clearing, and mounting.

### (Various extracellular matrix)

1. Make 5 µm thick sections from frozen block.
2. Fix sections in acetone at -20°C for 5-10 mins (paraffin blocks should be deparaffinized and rehydrated).
3. Endogenous peroxide activity is blocked in 0.3% H₂O₂ in methanol for 20 mins at RT (1 ml 30% H₂O₂ + 99 ml methanol)
4. Wash with PBS (3 × 5 mins).
5. Incubate with primary monoclonal antibody (a mouse or rabbit antibody against various extracellular matrix proteins) in a moist chamber at 4°C overnight (1 µl antibody + 200 µl PBS per slide).
6. Next day, wash with PBS (3 × 5 mins).
7. Apply anti mouse and anti rabbit no. 1 Biotynalated link for 30 mins to 1 hr at RT (apply 3 drops directly on slide).
8. Wash with PBS (3 × 5 mins).
9. Apply Streptavidin HRP no. 2 for LSAB and soak for 10-15 mins.
10. Wash with PBS (3 × 5 mins).
11. Apply DAB (5 ml DAB+5 µl H₂O₂).
12. Observe under microscope for brownish color.
13. Dip in water for 5 mins.
14. Apply HE for 30 sec-1 min.
15. Wash several times.
16. Wash once with ion exchange water.
17. wash for 1 min with 80% ethanol.
18. Wash for 1 min with 90% ethanol.
19. Wash for 1 min with 100% ethanol (3 times).
20. Wash for 1 min with Xylene (3 times) and apply coverslip.
21. Examine color development.

As a result, when ascorbic acid 2-phosphate was added as an ECM synthesis promoting agent, a multilayer structure of the cells was only slightly observed. On the other hand, by detaching the sheet-like cells from the base of the culture dish and allowing the cells to self-organize, the cells were promoted to be layered and to form a three-dimensional structure. Large tissue without a hole was also produced when synovial cells were used. This tissue was thick and rich in extracellular matrix. When observing synthetic tissues with ascorbic acid 2-phosphate concentration of 0 mM, 0.1 mM, 1 mM and 5 mM, it can be seen that the formation of an extracellular matrix was promoted when ascorbic acid 2-phosphate was added at a concentration of 0.1 mM or more. If synthetic tissues on Day 3, 7, 14, and 21 of culture were observed, after 3 days of culture, it can be seen that the tissue was already so rigid that it can be detached. As the number of culture days is increased, the density of the extracellular matrix fluctuates and increases.

The tissue was detached from the base of the culture dish and self-contracted. The synthetic tissue was prepared in a sheet form. When the sheet was detached from the dish and was allowed to stand, the sheet self contracted into a three-dimensional structure. It can be seen from the tissue that a number of layers of cells exist in the tissue.

Next, various markers including extracellular matrix were stained.

If the result of staining extracellular matrices is studied, it can be seen that various extracellular matrix components (collagen I, II, III, IV, fibronectin, vitronectin, etc.) were present. When immunostaining was conducted, collagen I and III were strongly stained while collagen II staining was limited to a portion. By being strongly magnified, it can be confirmed that collagen was stained at a site slightly away from the nuclei, and collagen was extracellular matrix. On the other hand, fibronectin and vitronectin, which are deemed important cell adhesion molecules, when strongly magnified, it can be confirmed that fibronectin and vitronectin were stained at a region close to nuclei unlike collagen, and fibronectin and vitronectin were present around the cells.

It seems that cells of at least 3 to 8 passages are preferable for production of a synthetic tissue, but cells with any number of passages can be used.

For comparison, an example is shown in which a normal tissue and a collagen sponge (CMI, Amgen, USA) were stained. If the normal tissue (normal synovial membrane tissue, tendon tissue, cartilage tissue, skin, and meniscus tissue) was compared to the commercially-available stained collagen sponge used as the comparative example, the conventional synthetic tissue was not stained with fibronectin or vitronectin. Therefore, the synthetic tissue of the present invention is different from conventional synthetic tissues. Existing collagen scaffolds do not contain adhesion agents fibronectin and vitronectin. In view of this, the originality of the tissue of the present invention is clearly understood. Stains are not found in any extracellular matrix. When the synthetic tissue of this production example was compared with normal tissue, it is confirmed that manner of integration of the synthetic tissue is close to the natural manner.

Further, when the synthetic tissue of the present invention was contacted with a filter paper in order to remove moisture, the filter adhered to the synthetic tissue, and it was difficult to manually detach the synthetic tissue.

In order to determine the collagen concentration, the collagen content was measured. As a result, the amount of hydroxyproline clearly indicates that the production of collagen was significantly promoted when 0.1 mM or more ascorbic acid 2-phosphate was added. The amount of produced collagen is substantially proportional to the time period of culture.

### (Production Example 2: Production of three-dimensional synthetic tissue using cells from adipose-derived tissue)

Next, cells derived from adipose tissue were used to produce a synthetic tissue.

A) Cells were collected as follows.
1) A specimen was removed from the fat-pad of a knee joint.
2) The specimen was washed with PBS.
3) The specimen was cut into as many pieces as possible using scissors.
4) 10 ml of collagenase (0.1%) was added to the specimen, followed by shaking for one hour in a water bath at 37°C.
5) An equal amount of DMEM (supplement with 10% FBS) was added, followed by filtration using a 70 µl filter (available from Millipore or the like).
6) Cells which passed through the filter and residues which remained on the filter were placed in a 25-cm² flask (available from Falcon or the like) containing 5 ml of DMEM supplemented with 10% FBS.
7) Cells attached to the bottom of the flask (including mesenchymal stem cells) were removed and subjected to the production of a synthetic tissue as follows.

### B) Production of synthetic tissue

Next, the above-described adipose-derived cells were used to produce a synthetic tissue. The concentrations of ascorbic acid 2-phosphate were 0 mM (absent), 0.1 mM, 0.5 mM, 1.0 mM, and 5.0 mM. The synthetic tissue was produced in accordance with the above-described method of producing synovial cells (described in Example 1). Cells were inoculated at an initial concentration of 5×10⁴ cells/cm². The cells were cultured for 14 days. A synthetic tissue was also produced from an adipose tissue-derived cell and such a tissue had fibronectin and vitronectin as much as the synovial cell-derived synthetic tissue. Collagen I and III were similarly expressed in abundance.

Ascorbic acid 2-phosphate 0 mM: tangent tensile modulus (Young's modulus) 0.28
Ascorbic acid 2-phosphate 1.0 mM: tangent tensile modulus (Young's modulus) 1.33

### C) Implantation experiment

Next, the above-described synthetic tissue can be used to produce a composite tissue described in the following Example. As a result, it is demonstrated that adipose-derived synthetic tissue has repairing capability similar to that of a composite tissue made of a three-dimensional synthetic tissue from synovial cells.

### D) Differentiation induction of adipose-derived synthetic tissue into bone/cartilage

The synthetic tissue of a bone or cartilage made in this example can be induced to differentiate into a cartilage or a bone. The synthetic tissue was confirmed to have a positive reaction to Alizarin Red in an osteogenesis induction medium. Thus, osteogenesis was confirmed. In a chondrogenesis induction experiment, the synthetic tissue was confirmed to differentiate by a stimulus due to chondrogenesis induction medium+BMP-2 into a cartilage-like tissue which was Alcian blue positive. Thus, it is confirmed that the adipose-derived synthetic tissue also has the ability to differentiate into a bone or a cartilage as with a synovial cell-derived synthetic tissue (see Japanese Patent No. 4522994).

### (Production Example 3: Production example with human synovial cells)

Next, a synovial cell is collected from a patient having an injured meniscus, and it is determined whether the synovial cell can be used to produce a synthetic tissue.

### (Collection of synovial cell)

A human patient, who is diagnosed by an imaging technique as having cartilage injury or meniscus injury, is subjected to arthroscopy under lumber anesthesia or general anesthesia. In this case, several tens of milligrams of synovial membrane are collected. The collected synovial membrane is transferred to a 50-ml centrifuge tube (manufactured by Falcon) and washed with phosphate buffered saline (PBS). Thereafter, the sample is transferred to a 10-cm diameter culture dish (Falcon) and is cut into small pieces using a sterilized blade. Thereafter, 10 ml of 0.1% collagenase (Sigma) is added to the cut pieces. The dish is shaken in a constant temperature bath at 37°C for 1 hour and 30 minutes. To the solution, 10 ml of medium (DMEM, Gibco) containing self-serum previously collected or bovine serum (FBS) is added to inactivate the collagenase, followed by centrifugation at 1500 rpm for 5 minutes to pellet the cells. Thereafter, 5 ml of the serum-containing medium is added again. The culture medium is passed through a 70-µl filter (Falcon). The collected cells are transferred to a 25 cm² flask (Falcon), followed by culture in a CO₂ incubator at 37°C.

### (Subculture of synovial cell)

During primary culture, medium is exchanged twice a week. When cells become confluent, the cells are subcultured. For initial subculture, the medium is suctioned and thereafter the cells are washed with PBS. Trypsin-EDTA (Gibco) is added to the cells which are in turn allowed to stand for 5 minutes . Thereafter, the serum-containing medium is added and the resultant mixture is transferred to a 50-ml centrifuge tube (Falcon), followed by centrifugation at 1500 rpm for 5 minutes. Thereafter, 15 ml of the serum-containing medium is added to the pellet. The cells are placed in a 150-cm² culture dish (Falcon). Subsequent subculture is performed so that the cell ratio is 1:3. The same procedure is repeated up to 4 to 5 passages.

### (Production of synthetic tissue)

The synovial cell of 4 to 5 passages is treated with trypsin-EDTA. The synovial cells (4.0×10⁶) are dispersed in 2 ml of medium containing 0.2 mM ascorbic acid 2-phosphate on a 35-ml culture dish (Falcon), followed by culture in a CO₂ incubator at 37°C for 7 days. As a result, a culture cell-extracellular matrix complex is formed. The complex is mechanically detached from the culture dish by pipetting the periphery thereof two or more hours before an implantation operation. After detachment, the culture cell-extracellular matrix complex contracts into a three-dimensional tissue having a diameter of about 15 mm and a thickness of about 0.1 mm.

### (Production Example 4: Production of a synthetic tissue from a human adipocyte)

A collection-intended site (e.g., around a knee joint) from a patient under local anesthesia is resected. Several tens of milligrams of adipocytes are collected. For example, the collected adipocytes are treated in a manner similar to that of the synovial cells as in the above-described production example. As a result, a three-dimensional synthetic tissue can be produced.

### (Production Example 5: Study on timing of differentiation for production of a synthetic tissue in the case of human cells)

Next, a synthetic tissue was produced using cells derived from adipose tissue.

### A) The cells were collected as follows.

1) A specimen was collected from a fat-pad of a knee joint.
2) The specimen was washed with PBS.
3) The specimen was cut into as many pieces as possible with a pair of scissors.
4) 10 ml of collagenase (0.1%) was added, followed by shaking in 37°C water bath for one hour.
5) An equal amount of DMEM (supplemented with 10% FBS) was added. The resultant mixture was passed through a 70-µl filter (available from Millipore, etc.).
6) Cells passing through the filter and cells remaining on the filter were cultured in 25-cm² flask (available from FALCON or the like) containing 5 ml of DMEM medium supplemented with 10% FBS.
7) The cells (including a mesenchymal stem cell) attached to the base of the flask were removed to produce a synthetic tissue as follows.

### B) Production method of synthetic tissue

Next, the adipose-derived cells were used to produce a synthetic tissue. Ascorbic acid 2-phosphate was used at a concentration of 0 mM (absence), 0.1 mM, 0.5 mM, 1.0 mM, or 5.0 mM. The production was conducted in accordance with the method for producing a synthetic tissue from synovial cells (as described in Example 1). The cells were inoculated at an initial density of 5×10⁴ cells/cm².

The cells were used to study the important of differentiation period by using various conditions.

As a result, it was revealed that differentiation period does not especially affect the synthetic tissue of the present invention, similarly to those derived from collected adipocytes (see Japanese Patent No. 4522994).

### (Production Example 6: Production of three-dimensional synthetic tissue by myoblasts)

Next, an effect on production of a synthetic tissue by ascorbic acid or a derivative thereof was studied when myoblasts were used. Production of a synthetic tissue was conducted in accordance with Production Example 1.

After the myoblast was sufficient grown, 5×10⁶myoblast cells were cultured. For culture, a medium called SkBM Basal Medium (Clonetics (Cambrex)) was used. Next, ascorbic acid 2-phosphate (0.5 mM), a magnesium salt of ascorbic acid 1-phosphate (0.1 mM), and L-ascorbic acid Na (0.1 mM) were added. After four days from the start of culture, the tissue was detached. As a control, a synthetic tissue was produced by culturing in a culture system without ascorbic acids.

### (Results)

When ascorbic acids were added, the synthetic tissue was more readily detached as compared to the synthetic tissue from the ascorbic acid-free culture system was used. In addition, in the ascorbic acid-free culture system, the tissue was cultured only to a size of about several millimeters. When the tissue exceeded such a level, a crack or the like occurred to inhibit growth. In addition, it was substantially difficult to detach the tissue. Thus, no implantable synthetic tissue could be provided. In contrast, the synthetic tissue cultured in a medium supplemented with ascorbic acid of the present invention grew to an implantable size and was readily detachable. It was found that interaction with extracellular matrix significantly progressed in view of biological integration.

In this manner, a three-dimensional synthetic tissue used in the present invention was able to be produced by using various cells.

### (Example 1)

### (Collection of synovial tissues and isolation of cells)

All animal experimentations were approved by Osaka University Faculty of Medicine animal experiment facility. A synovial membrane of rabbits was aseptically collected from a knee joint of a rabbit with a mature (24 weeks of age) skeleton within 12 hours post mortem. The protocol for cell isolation was substantially the same as that reported previously with regard to isolation of MSC derived from human synovial cells [Ando W, Tateishi K, Katakai D, Hart DA, Higuchi C, Nakata K, et al., Tissue Eng Part A 2008; 14:2041-2049] . Briefly stated, synovial membrane samples were rinsed with phosphate buffered saline (PBS), cut into small pieces, and treated with 0.4% collagenase XI (Sigma-Aldrich, St. Louis, MO, USA) for 2 hours at 37°C. After the collagenase was neutralized in a growth medium containing a high-glucose Dulbecco's modified eagle's medium (HG-DMEM; Wako, Osaka, Japan) added with 10% fetal bovine serum (FBS; HyClone, Logan, UT, USA) and 1% penicillin/streptomycin (Gibco BRL, Life Technologies Inc., Carlsbad, CA, USA), cells were collected by centrifugation, washed with PBS, resuspended in a growth medium, and plated on a culture dish. The characteristics of rabbit mesenchymal stem cells were similar to human synovial membrane-derived MSC in terms of form, growth characteristics, and pluripotency (into bone system, cartilage system and fat system) [Ando W, Tateishi K, Katakai D, Hart DA, Higuchi C, Nakata K, et al., Tissue Eng Part A 2008; 14:2041-2049; Tateishi K, Higuchi C, Ando W, Nakata K, Hashimoto J, Hart DA, et al., Osteoarthritis Cartilage 2007; 15: 709-718] . For cell growth, these cells were grown in a growth medium at 37°C in a humidified atmosphere of 5% CO₂. The medium was changed once a week. When cells reached confluence 7-10 days after primary culture, the cells were washed twice with PBS, collected after treatment with trypsin-EDTA (0.25% trypsin and 1mM EDTA: Gibco BRL, Life Technologies Inc., Carlsbad, CA, USA), and diluted so that the cell concentration would be 1:3. The cells were again plated. When culture cells substantially reached confluence, the cells were similarly diluted to 1: 3 ratio to continue passage of cells. The present application used cells of 3-7 passages.

### (Production of three-dimensional tissue (TEC))

Synovial membrane MSCs were plated on a 6-well plate (9.6 cm²) at a density of 4.0 × 10⁵ cells/cm² in a growth medium containing 0.2 mM of ascorbate-2-phosphate (Asc-2P), which is the optimal concentration from previous studies [Ando W, Tateishi K, Katakai D, Hart DA, Higuchi C, Nakata K, et al., Tissue Eng Part A 2008; 14:2041-2049; Ando W, Tateishi K, Hart DA, Katakai D, Tanaka Y, Nakata K, et al., Biomaterials 2007; 28:5462-5470; Shimomura K, Ando W, Tateishi K, Nansai R, Fujie H, Hart DA, et al., Biomaterials 2010; 31:8004-8011]. The cells reached confluence within a day. Furthermore, after continuous culturing for 7-14 days, a complex of culture cells and ECM synthesized by said cells was detached from the lower layer by applying shearing stress by using a light pipette. The detached single layer complex was maintained in a suspension to allow formation of a three-dimensional structure by self-tissue contraction. This tissue was called a three-dimensional TEC that is not dependent on a scaffold (also called scaffold-free).

### (Production of composite tissue (hybrid graft) consisting of TEC and artificial bone)

Synthetic HA (diameter 5 mm, height 4 mm (NEOBONE^{®}; MMT Co. Ltd., Osaka, Japan)) with an interconnected, porous structure was used as an artificial bone. A TEC was detached from a culture dish immediately prior to an implant surgery and connected to the artificial bone without using an adhesive to produce a bone/cartilage hybrid (Figure 1a). Once a TEC integrates with an artificial bone, the integration is so strong that separation would be difficult.

### (Implantation of hybrid graft to osteochondral defect)

41 New Zealand white rabbits with a mature skeleton (24 weeks of age or older) were maintained in a cage while freely providing food and water. The rabbits were administered with anesthesia by an intravenous injection of 1 ml of pentobarbital [50 mg/ml (Nembutal^{®}, Dainippon Pharmaceutical Co. Ltd., Osaka, Japan)] and 1ml of xylazine hydrochloride (25 mg/ml (Seractal^{®}, Bayer, Germany)). The rabbits were shaved and disinfected. After covering with a sterilized fabric, a straight 3 cm incision was made on the side of the inside patella of the right knee. The patella was moved outward to expose the femoral fossa. A joint osteochondral defect through the entire thickness of 5 mm diameter and 6 mm depth was mechanically made in the femoral fossa of the right distal femur (Figure 1b). A hybrid of a TEC and artificial bone was formed immediately prior to implantation. Then, a diphasic graft was implanted within the defect of the right knee of 23 rabbits (TEC group) . In a control group, only an artificial bone was implanted in a defect of the right knee of 18 rabbits (Figures **1c****-d)** . The right limbs of all the animals were stabilized in a cast for 7 days. The rabbits were then euthanized under anesthesia at one month, 2 months, and 6 months after the surgical operation. The implantation site (18 samples in the TEC group and 13 samples in the control group) were secured for use in the following paraffin section production and histological analysis. Other implantation sites (5 samples in the TEC group and 5 samples in the control group) were subjected to biodynamic tests. The present inventors prepared left knees of 5 rabbits as untreated normal controls for the biodynamic tests.

### (Histological evaluation of repaired tissue)

For histological evaluation, tissues were fixed with 10% formalin neutral buffer, decalcified with K-CX (Falma, Tokyo, Japan) and embedded in paraffin to produce a 4 mm section. The section was stained with hematoxylin-eosin (H&E) and toluidine blue.

At one, two, and six months, histology of repaired tissue was evaluated with improved "O'Driscoll score" for cartilage and subchondral bones [O'Driscoll SW, Keeley FW, Salter RB., J Bone Joint Surg Am 1988; 70:595-606; Mrosek EH, Schagemann JC, Chung HW, Fitzsimmons JS, Yaszemski MJ, Mardones RM, et al. , J Orthop Res 2010; 28:141-148; Olivos-Meza A, Fitzsimmons JS, Casper ME, Chen Q, An KN, Ruesink TJ, et al., Osteoarthritis Cartilage 2010;18:1183-1191]. In the improved version, standard classification "toluidine blue stain" was changed to "safranin O stain". For subchondral bone repair, new standard classifications "Cell form" and "Exposure of subchondral bone" were established. For "Cell form", normal subchondral repair has a score of 2, a tissue repaired with cartilage-like tissue mixed therein has a score of 1, and a tissue repaired with fibrous tissue mixed therein has a score of 0. For "Exposure of subchondral bone", no exposure of a subchondral bone has a score of 2, exposure of subchondral bone in one side of the boundary between a repaired tissue and an adjacent cartilage has a score of 1, and exposure of a subchondral bone on both sides has a score of 0. The repaired tissue was divided into three sections for each 2mm width to form a central region and two boundary regions . Each of the three regions was evaluated by the improved O'Driscoll score. The average of the three scores was the "overall evaluation". Furthermore, the score of the central region was evaluated as the "central region" and the average score of the two boundary regions was evaluated as the "boundary region". The standard classifications "Integration with adjacent cartilage", "Free of degeneration of adjacent cartilage", and "Exposure of subchondral bone" were only evaluated by "overall evaluation".

In order to quantify the degree of repair of a subchondral bone and cartilage, bone and cartilage formation ratios of the repaired tissue were calculated at one, two, and six months. For bone formation ratios, the length of repaired bone tissue was divided by the length of an artificial bone. The results are shown in percentages (Figure **2a****).** The cartilage formation ratios were calculated by the same method (Figure **2b****).** Furthermore, the correlation of the bone formation ratio and cartilage formation ratio was calculated.

### (Biochemical test)

A cylindrical sample with a 4 mm diameter and 5 mm depth was extracted from implantation sites of the TEC group and the control group. Similarly, a cylindrical sample was extracted from the interchondylar center of femurs of an untreated normal knee. AFM (Nan scope IIIa, Veeco Instruments, Santa Barbara, CA, USA) and silicone probe (spring constant: 0.06 N/m, DNP-S, Veeco Instruments, Santa Barbara, CA, USA) were used. Each sample was mounted on an AFM sample stage and dipped in a saline solution at room temperature. Microcompression tests were conducted on these samples. Furthermore, a surface image of a sample was obtained in a contact mode in a 30 um × 30 um scan region, and microcompression tests were then conducted with a scan rate of 0.3 Hz on the samples at a 5.12 um pushing rate.

### (Statistical analysis)

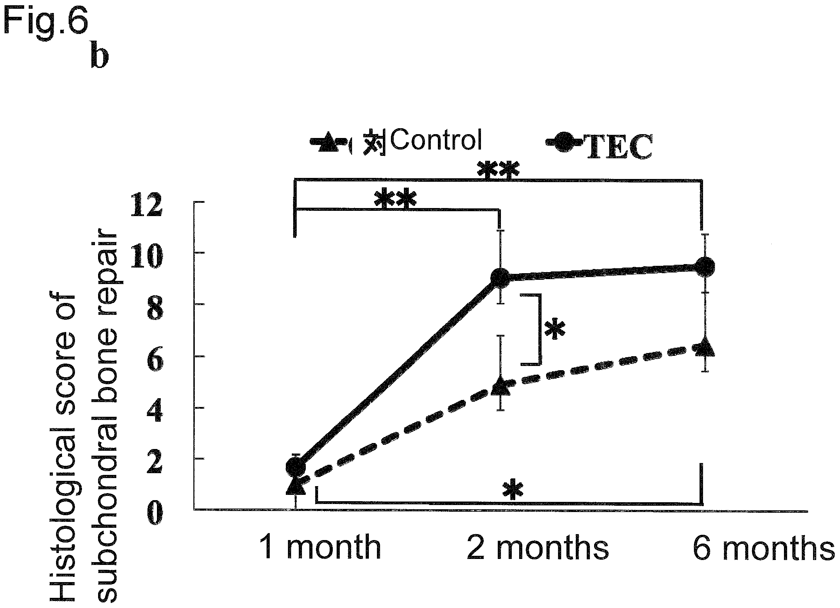

For statistical analysis, analysis of variance (ANOVA) was carried out. Comparative tests were run ex post facto for post-operation changes in overall histological score and bioengineering tests (Figures **6a****,** **6b****,** **8a****,** and **8e****)** . Comparisons of other experimental parameters between the reference group and the TEC group were analyzed by the Mann-Whitney U-test (Figures **7a****-7c,** Tables 1 and 2). The correlation of bone and cartilage formation was calculated with Spearman's rank correlation coefficient (Figure **7d****)** . The results are represented by mean ± SD (standard deviation) . The data was analyzed with JMP9 (SAS Institute, Cary, NC, USA) . Statistical significance was set at p<0.05 for judgment.

### (Results)

### (Formation of composite tissue (hybrid) of TEC with artificial bone)

A TEC immediately integrated on an artificial bone block to form a complex with sufficient strength for surgical implantation.

### (Evaluation of repaired tissue with naked eye)

Defects were partially or completely covered with a repaired tissue in the TEC group one month after a surgical operation. However, the artificial bone was exposed for all of the subjects in the control group (Figures **3a****-b)** . Two months after the surgical operation, defects were covered with a repaired tissue in both the control group and the TEC group (Figures **4a-b****).** Six months after the surgical operation, the defects were continuously covered with repaired tissue in both groups. Thus, an obvious change due to osteoarthritis was not observed (Figures **5a**-b).

### (Histological evaluation of repaired tissue)

One month after a surgical operation, defects were repaired in fibrous tissue with thickness in the TEC group. In addition, the repaired tissue exhibited excellent biological integration with an adjacent host tissue (Figure **3c****).** On the other hand, a tissue was not repaired and an artificial bone remained exposed (Figure **3d****)** . Two months after the surgical operation, repaired tissues in the TEC group exhibited osteogenesis inmost portions . In addition, formation of a cartilage was in progress in the vicinity of repaired bones (Figures **4c-d****)** . In the control group, defects were repaired by fibrous tissues (Figures **4e-f****).** In images with higher magnification, excellent biological integration of tissues with an adjacent host tissue was obtained and the repaired tissue showed glass-like cartilages in the TEC group (Figures **4i, 4j** and **4l****)** . Meanwhile, defects of the control group exhibited delay in subchondral bone repair and incomplete osteochondral repair with poor biological integration with an adjacent host tissue (Figures **4g, 4h****,** and **4k****)** . Six months after the surgical operation, the repaired tissues in the TEC group were completely repaired with osteochondral tissues, but some of the repaired tissues exhibited growth of a subchondral bone (thinning of cartilage) (Figures **5e-f****).** Some level of osteochondral repair was also observed in the control group. However, the repaired tissue exhibited poor biological integration with an adjacent cartilage and insufficient osteochondral repair in which fibrous tissues and cartilage-like tissues were mixed in a subchondral bone region (Figures **5c, 5d****,** arrow) . Images with higher magnification show that excellent biological integration of a tissue with an adjacent host tissue was maintained and repaired tissue shows a glass-like cartilage (Figures **5i, 5j** and **5l**). In contrast, repaired tissues exhibited poor biological integration of tissues with a host tissue and fibrous cartilage-like tissue with clustering of cells in the control group. These results demonstrate that repaired tissues in the control group were observed in progressing osteoarthritis (Figures **5g****,** **5h****,** and **5k****).**

### (Histological score related to cartilage repair)

Overall histological scores of cartilage repair in the TEC group significantly increased two months after an operation in comparison to the scores at one month (19.16 ± 2.33 versus 14.22 ± 2.02, p=0.0036) . Next, the overall histological scores reached a plateau before six months . In the reference group, the overall histological scores also significantly increased at two months after an operation in comparison to one month after operation (13.67 ± 2.10 versus 5.34 ± 2.59, p= 0.0257) . However, the overall histological scores at 6 months tended to be less than the 2 month scores . However, a significant difference was not detected between 2 months and 6 months (13.67 ± 2.10 versus 9.56 ± 5.17, p=0.2771) (Figure **6a****).**

The overall histological scores for cartilage repair in the TEC group were significantly higher in comparison to the reference group (at one month, 14.22 ± 2.02 versus 5.34 ± 2.59, p=0.0103; at two months, 19.16 ± 2.33 versus 13.67 ± 2.10, p= 0.0179; at six months, 17.8 versus 10.5, p=0.0465) . The overall scores were higher in the TEC group in comparison to the reference group at 2 months after the operation. However, the difference was not significant (19.03 ± 2.15 versus 9.56 ± 5.17, p=0.0119) (Figure **6a****).**

The standard classifications "Cell form" and "Toluidine blue" were significantly higher in the TEC group in comparison to the reference group up to six months after the operation. The results show that cartilage repair in a composite tissue (hybrid graft) of the present invention accelerated. For the standard classification "Integration to adjacent cartilage", the histological scores in the TEC group were significantly higher in comparison to the reference group at one, two and six months after the operation. In addition, the standard classification "Structural integrity" was significantly higher in the TEC group at six months in comparison to the reference group, especially for the subclassification "Boundary region". These results show that adhesive property of a TEC contributes to excellent biological integration to an adjacent host tissue. The standard classifications "Cartilage cell clustering" and "Degeneration of adjacent cartilage" were significantly lower than those in the reference group at six months. These results demonstrate degeneration of repaired tissues observed in the early stages of osteoarthritis (Table 1).
(Table 1: Histological evaluation of cartilage repair)

**[Table 1]**

| Explanation of histological evaluation | | One month after operation | | | Two months after operation | | | Six months after operation | | |
|---|---|---|---|---|---|---|---|---|---|---|
| | | Reference group (N=4) | TEC (N=6) | P value | Reference group (N=4) | TEC (N=7) | P value | Reference group (N=5) | TEC (N=5) | P value |
| Cell form | Overall evaluation | 0 | 1.22±0. 50 | **0.0073** | 1.67± 0.39 | 3.14±0 .63 | **0.0109** | 1.47±1.28 | 3.20+0. 30 | **0.0343** |
| | Central region | 0 | 0.33±0. 82 | 0.4142 | 1.00±1.15 | 2.86±1 .57 | 0.0716 | 1.60±2.19 | 3.60±0. 89 | 0.1202 |
| | Boundary regions | 0 | 1.67±0. 52 | **0.0062** | 2 | 3.29± 0 .76 | **0.0152** | 1.40±1.14 | 3.00±0. 71 | **0.0393** |
| Toluidine blue staining | Overall evaluation | 0 | 0.92±0. 37 | **0.0073** | 1.09±0.42 | 2.14±0 .51 | **0.0171** | 1.27± 0.86 | 2.67±0. 47 | **0.0196** |
| | Central region | 0 | 0.33±0. 52 | 0.2207 | 0.50±0.58 | 2.29±1 .11 | **0.0309** | 1.20± 1.64 | 3 | **0.0495** |
| | Boundary regions | 0 | 092±0. 38 | **0.0073** | 1.38±0.48 | 2.07±0 .61 | 0.0716 | 1.30±0.84 | 2.10±0. 42 | 0.0827 |
| Continuity of surface layer | Overall evaluation | 0.42±0.50 | 1.89±0. 66 | **0.0131** | 1.83±0.43 | 2.24±0 .42 | 0.1420 | 1.20±0.65 | 1.80±1. 30 | 0.0731 |
| | Central region | 0.25±0.50 | 2.17±0. 75 | **0.0114** | 2.75± 0.50 | 2.71±0 .49 | 0.9029 | 2.40±0.89 | 2.80±0. 45 | 0.4386 |
| | Boundary regions | 0.50±0.58 | 1.75±0. 69 | **0.0159** | 1.38±0.48 | 2.00±0 .58 | 0.0977 | 0.60±0.65 | 1.70±0. 91 | 0.0723 |
| (Structural) integrity | Overall evaluation | 0.42±0.50 | 1.50±0. 36 | **0.0131** | 1.09±0.42 | 1.52±0 .38 | 0.1001 | 0.73±0.43 | 1.53±0. 38 | **0.0174** |
| | Central region | 0.50±0.58 | 1.83±0. 41 | **0.0109** | 2 | 2 | 1.0000 | 1.60±0.89 | 2 | 0.3173 |
| | Boundary regions | 0.38±0.48 | 1.33±0. 41 | **0.0201** | 0.63±0.63 | 1.2910 .57 | 0.1001 | 0.30± 0.27 | 1.30±0. 57 | **0.0170** |
| Thickness | Overall evaluation | 0.50±0.58 | 1.39±0. 57 | 0.0765 | 1.83±0.34 | 1.52±0 .38 | 0.1862 | 0.80±0.65 | 1.40±0. 28 | 0.1071 |
| | Central region | 0.50±0.58 | 1.50±0. 55 | **0.0372** | 2 | 1.71±0 .49 | 0.2598 | 1.20±1.10 | 1.80±0. 45 | 0.3662 |
| | Boundary regions | 0.50±0.58 | 1.33± 0. 61 | 0.0765 | 1.75± 0.50 | 1.43±0 .45 | 0.2621 | 0.60±0.55 | 1.20±0. 27 | 0.0652 |
| Integration with adjacent cartilage | Overall evaluation | 0.13±0.25 | 1.50±0. 45 | **0.0089** | 0.38± 0.48 | 1.64±0 .48 | **0.0109** | 0.30± 0.27 | 1.50±0. 61 | **0.0167** |
| Hypocellularity | Overall evaluation | 1.00±1.28 | 2.89±0. 27 | **0.0121** | 3 | 3 | 1.0000 | 1.817±1.12 | 2.74±0. 15 | 0.0837 |
| | Central region | 0.25±0.50 | 2.67±0. 82 | **0.0078** | 3 | 3 | 1.0000 | 1.80±1.30 | 3 | 0.0539 |
| | Boundary regions | 1.25±1.50 | 3 | **0.0177** | 3 | 3 | 1.0000 | 1.80±1.15 | 2.60±0. 22 | 0.1797 |
| Cartilage cell clustering | Overall evaluation | 0 | 0.17±0. 41 | 0.4142 | 0.42±0.50 | 1.24±0 .46 | **0.0325** | 0.40±0.37 | 1.53±0. 30 | **0.0074** |
| | Central region | 0 | 0.17±0. 41 | 0.4142 | 0.25±0.50 | 1.29±0 .76 | **0.0453** | 0.80±0.84 | 2 | **0.0177** |
| | Boundary regions | 0 | 0.17±0. 41 | 0.4142 | 0.5±0.58 | 1.21±0 .39 | **0.0482** | 0.20±0.27 | 1.30±0. 45 | **0.0072** |
| Degeneration of adjacent cartilage | Overall evaluation | 2.88±0.25 | 3 | 0.2207 | 2.38±0.25 | 2.71±0 .27 | 0.0763 | 1.60±0.22 | 2.40± 0. 22 | **0.0073** |
| Overall score | | 5.34±2.59 | 14.22± 2.02 | **0.0103** | 13.67±2.10 | 19.16± 2.33 | **0.0179** | 9.56±5.17 | 19.03±2 .15 | **0.0119** |

Repaired tissues were separated into three sections for scoring, a peripheral section, center, and the opposite peripheral section. Overall scores were computed as the mean of the three scores. Border was calculated as a mean of both peripheral sections. Please see the "Reference Material" (Table 1A) for the details of each score.

### (Table 1A)

**[Table 1A]**

| | | |
|---|---|---|
| Cell form | Glass joint cartilage | 4 |
| | Incomplete cartilage differentiation | 2 |
| | Fibrous tissue or bone | 0 |
| Toluidine blue staining | Normal | 3 |
| | About medium | 2 |
| | Little | 1 |
| | None | 0 |
| Continuity of surface layer | Smooth and unscathed | 3 |
| | Surface is a horizontal thin layer | 2 |
| | 25-100% fissure with respect to thickness | 1 |
| | has severe destruction or fibrillization | 0 |
| (Structural) integrity | Normal | 2 |
| | has small amount of destruction, cyst | 1 |
| | Severe destruction | 0 |
| Thickness | 100% of normal adjacent cartilage | 2 |
| | 50-100% of normal adjacent cartilage | 1 |
| | 0-50% of normal adjacent cartilage | 0 |
| Integration with adjacent cartilage | Integration at both ends of graft | 2 |
| | Integration with one end or partially at both ends | 1 |
| | No integration | 0 |
| Hypocellularity | Normal cellularity | 3 |
| | Small amount of hypocellularity | 2 |
| | Medium level hypocellularity | 1 |
| | Severe hypocellularity | 0 |
| Cartilage cell clustering | No clustering | 2 |
| | Less than or equal to 25% of cells | 1 |
| | 25-100% of cells | 0 |
| Free of degeneration of adjacent cartilage | Normal cellularity, no clustering, normal staining | 3 |
| | Normal cellularity, medium level of clustering, medium level of staining | 2 |
| | Medium level of cellularity, medium level of clustering, medium level of staining | 1 |
| | Severe hypocellularity, weak staining or no staining | 0 |

### (Histological score of subchondral bone repair)

Overall histological scores for subchondral bone repair in the TEC group significantly increased at two months after operation in comparison to one month after operation (9.07 ± 1.84 versus 1.67 ± 0.57, p<0.0001). Next, the overall histological scores reached a plateau before six months. In the reference group, overall histological scores increased along with passage of time (time-dependency), which was significant between one month and six months (1.00 ± 1.15 at p=0.0156) (Figure **6b**) .

Subchondral bone repair was observed in neither the TEC group nor the reference group one month after the operation. Thus, all classifications other than the standard classification "Exposure of subchondral bone" were 0. The overall histological score for a subchondral bone was significantly higher in the TEC group at 2 months after operation in comparison to the reference group (9.07 ± 1.84 versus 4.92 ± 1.91, p=0.0140) . At six months after the operation, the overall scores were higher in the TEC group than in the reference group. However, a significant difference was not observed (9.54 ± 1.26 versus 6.47 ± 3.20, p=0.0937) (Figure **6b**).

For the standard classifications "Alignment of subchondral bone", "Integration of bone", "Bone infiltration into injury region" and "Cell form", there was a significant difference between the TEC group and the reference group at 2 months after the operation. These results demonstrate that a composite tissue (hybrid tissue) of the present invention promoted repair of subchondral bones from an early stage. Even at 6 months after operation, the standard classification "Tidemark continuity" was significantly higher in the TEC group than in the reference group. These results show that a composite tissue of the present invention contributes to promotion of maturation of a subchondral bone. However, standard classifications other than "Tidemark continuity" did not have a significant difference between the TEC group and the reference group. These results demonstrate that repair of a subchondral bone occurs in some form even in the reference group. For the standard classification "Exposure of subchondral bone", the scores worsened in the reference group at 6 months after the operation in comparison to 2 months. These results demonstrate that an artificial bone alone did not contribute to long-term resistivity (Table 2).

### (Table 2: Histological evaluation on repair of subchondral bone

**[Table 2]**

| Explanation of histological evaluation | | One month after operation | | | Two months after operation | | | Six months after operation | | |
|---|---|---|---|---|---|---|---|---|---|---|
| | | Reference group (N=4) | TEC (N=6) | P value | Reference group (N=4) | TEC (N=7) | P value | Reference group (N=5) | TEC (N=5) | P value |
| Alignment of subchondral bone | Overall evaluation | 0 | 0 | 1.0000 | 0.67±0.54 | 1.67±0.34 | **0.0144** | 0.73±0.87 | 1.27±0.43 | 0.3305 |
| | Central region | 0 | 0 | 1.0000 | 0.25±0.50 | 1.57±0.79 | **0.0249** | 0.80±0.84 | 1.00±0.71 | 0.6501 |
| | Boundary regions | 0 | 0 | 1.0000 | 0.88±0.85 | 1.71±0.39 | 0.0904 | 0.70±0.97 | 1.40±0.55 | 0.2328 |
| Integration of bone (biological integration) | Overall evaluation | 0 | 0 | 1.0000 | 0.75±0.57 | 1.79±0.39 | **0.0130** | 1.47± 0.84 | 1.87±0.18 | 0.4189 |
| | Central region | 0 | 0 | 1.0000 | 0.25±0.50 | 1.43±0.98 | 0.0601 | 1.20±0.84 | 1.60±0.55 | 0.4189 |
| | Boundary regions | 0 | 0 | 1.0000 | 1.00±0.91 | 1.57±0.79 | 0.2150 | 1.60±0.89 | 2 | 0.3173 |
| Bone infiltration into injury region | Overall evaluation | 0 | 0 | 1.0000 | 0.67±0.54 | 1.48±0.50 | **0.0437** | 1.60±0.55 | 1.80±0.18 | 0.7290 |
| | Central region | 0 | 0 | 1.0000 | 0.25±0.50 | 1.29±0.95 | 0.0831 | 1.20±0.84 | 1.40±0.55 | 0.7290 |
| | Boundary regions | 0 | 0 | 1.0000 | 0.88±0.85 | 1.79±0.39 | 0.0629 | 1.80±0.45 | 2 | 0.3173 |
| Tidemark continuity | Overall evaluation | 0 | 0 | 1.0000 | 0 | 0.67±0.67 | 0.0763 | 0.40±0.37 | 1.20±0.38 | **0.0192** |
| | Central region | 0 | 0 | 1.0000 | 0 | 0.86±1.07 | 0_1432 | 0.60±0.89 | 1.20±0.84 | 0.2685 |
| | Boundary regions | 0 | 0 | 1.0000 | 0 | 0.57±0.53 | 0.0708 | 0.30±0.27 | 1.20±0.27 | **0.0071** |
| Cell form | Overall evaluation | 0 | 0 | 1.0000 | 0.84 ±0.33 | 1.76±0.32 | **0.0104** | 1.47±0.51 | 1.60±0.28 | 0.8266 |
| | Central region | 0 | 0 | 1.0000 | 0.25±0.50 | 1.43±0.79 | **0.0356** | 1.00±1.00 | 1.40±0.55 | 0.5023 |
| | Boundary regions | 0 | 0 | 1.0000 | 1.13±0.63 | 1.93±0.19 | **0.0281** | 1.70±0.45 | 1.70±0.45 | 1.0000 |
| Exposure of subchondral bone | Overall evaluation | 1.00±1.15 | 1.67±0.52 | 0.2708 | 2 | 1.71±0.49 | 0.2598 | 0.80±0.84 | 1.80±0.45 | 0.0539 |
| Overall score | | 1.00±1.15 | 1.67±0.52 | 0.2708 | 4.92±1.91 | 9.07±1.84 | **0.0140** | 6.47±3.20 | 9.54±1.26 | 0.0937 |

Please see "Reference Material" (Table 2A) for details of each score.

### (Table 2A)

**[Table 2A]**

| | | |
|---|---|---|
| Alignment of subchondral bone | Horizontal | 2 |
| | Depression | 1 |
| | Bulged up or nothing | 0 |
| Integration of bone (biological integration) | Integrated | 2 |
| | Partially integrated | 1 |
| | Not integrated | 0 |
| Bone infiltration into injury region | Nearly completely or completely | 2 |
| | Partially | 1 |
| | None or minimal | 0 |
| Tidemark continuity | present, no gaps | 2 |
| | present, has gaps | 1 |
| | not present | 0 |
| Cell form | Repair to normal subchondral bone | 2 |
| | Cartilage-like tissue is mixed in | 1 |
| | Fibrous tissue is mixed in | 0 |
| Exposure of subchondral bone | None | 2 |
| | Exposure at one of the boundary surfaces | 1 |
| | Exposure at both of the boundary surfaces | 0 |

Presence of exposure of regenerated subchondral bone→degeneration of regenerated tissue was reflected.

### (Formation ratio of bone and cartilage and the correlation thereof)

Bone formation ratios in the TEC group were higher than those of the control group. However, there was no significant difference between the two groups one month, two month or six months after a surgical operation (13.3 ± 14.5 versus 0, p = 0.0547; 82.0 ± 21.4 versus 37.0 ± 31.3, p=0.0565; 91.1 ± 19.8 versus 82.3 ± 31.4, p=0.5775, respectively) (Figures 7a-c). Cartilage formation ratio in the TEC group was significantly higher than that in the control group at 2 months after a surgical operation (93.2 ± 10.3 versus 56.4 ± 38.6, p=0.0203). However, there was no significant difference between the two groups after one month and six months from the surgical operation (14.3 ± 21.7 versus 0, p=0.1155; and 91.0 ± 14.3 versus 73.6 ± 43.0, p = 0.5775, respectively) (Figures **7a****-c**). Formation ratios of bone and cartilage in all subjects (N=31) were significantly correlated (r= 0.8872, p<0.001) (Figure **7d**).

### (Mechanical property)

An osteochondral tissue implanted with a composite tissue (hybrid graft; TEC) of the present invention acquired rigidity that was equivalent to a normal osteochondral tissue (23.2 ± 12.5 versus 16.8 ± 10.0 mN/m, p=0.3472) . However, a significant difference between the TEC group and the control group was not detected (16.8 ± 10.0 versus 11.4 ± 8. 8 mN/m, p=0.4647) (Figure **8a**) . In digital images of the surface, repaired tissues exhibited smooth surfaces for both the TEC group and the untreated normal tissue group. However, the control group similarly exhibited a smooth surface (Figures **8b-d**). For the roughness of surfaces calculated from the digital images, a significant difference was not seen among the untreated normal tissue group, control group and TEC group (Figure **8e**).

### (Discussion)

Osteoarthritis is accompanied not only by a cartilage injury but also an injury of a subchondral bone. Thus, it is important to promote band-like recovery for each layer of a subchondral bone and cartilage. Recently, numerous studies have targeted regeneration in these two types of structures, using diphasic and triphasic grafts [Hung CT, Lima EG, Mauck RL, Takai E, LeRoux MA, Lu HH, et al., J Biomech 2003; 36:1853-1864; Marquass B, Somerson JS, Hepp P, Aigner T, Schwan S, Bader A, et al., J Orthop Res 2010; 28:1586-1599; Oliveira JM, Rodrigues MT, Silva SS, Malafaya PB, Gomes ME, Viegas CA, et al., Biomaterials 2006; 27:6123-6137; Sherwood JK, Riley SL, Palazzolo R, Brown SC, Monkhouse DC, Coates M, et al., Biomaterials 2002; 23:4739-4751; Ahn JH, Lee TH, Oh JS, Kim SY, Kim HJ, Park IK, et al., Tissue Eng Part A 2009; 15:2595-2604; Alhadlaq A, Mao JJ., J Bone Joint SurgAm2005; 87:936-944; Gao J, Dennis JE, Solchaga LA, Goldberg VM, Caplan AI. , Tissue Eng 2002; 8:827-837; Kandel RA, Grynpas M, Pilliar R, Lee J, Wang J, Waldman S, et al., Biomaterials 2006; 27:4120-4131; Chen J, Chen H, Li P, Diao H, Zhu S, Dong L, et al., Biomaterials 2011; 32:4793-4805]. With regard to materials for forming a cartilage layer of a diphasic structure, agarose, hydrogel, hyaluronan or chitosan material has been developed as a scaffold. These scaffolds are experimentally and clinically used in autologous cartilage implantation and demonstrated to exhibit excellent clinical results [Kon E, Gobbi A, Filardo G, Delcogliano M, Zaffagnini S, Marcacci M., Am J Sports Med 2009; 37:33-41; Basad E, Ishaque B, Bachmann G, Sturz H, Steinmeyer J., Knee Surg Sports Traumatol Arthrosc 2010; 18:519-527] . However, scaffolds generally contain a synthetic polymer or biomaterial. Thus, several issues associated with long-term safety of these materials are still present. Meanwhile, since the TEC used in the present invention is scaffold-free, the TEC does not contain any animal-derived substance or chemical substance. In addition, HA or β-TCP are used as materials for forming a subchondral bone layer of a diphasic structure. These materials are extensively used in clinical settings for supplementing an injury of a bone due to a fracture or cutting and removing of a bone tumor [Tamai N, Myoui A, Hirao M, Kaito T, Ochi T, Tanaka J, et al., Osteoarthritis Cartilage 2005; 13:405-417; Tamai N, Myoui A, Kudawara I, Ueda T, Yoshikawa H. , J Orthop Sci 2010; 15:560-568; Shen C, Ma J, Chen XD, Dai LY., Knee Surg Sports Traumatol Arthrosc 2009; 17:1406-1411]. In summary, since a TEC are not dependent on a scaffold, a TEC does not contain animal or chemical material derived substance. Further, HA is extensively used in clinical settings. Thus, the composite tissue (hybrid material) of the present invention is possibly suitable to be applied effectively and safely for repairing an osteochondral injury.

It is particularly noteworthy that a composite tissue (hybrid material) made of a TEC and an artificial bone promoted excellent osteochondral repair at least up to six months from a surgical operation in comparison to a control group. In addition, a repaired tissue with excellent biological integration to an adjacent host tissue was maintained up to six months from a surgical operation in the TEC group. Meanwhile, poor biological integration with an adjacent host tissue and an increase in cell clustering, which were observed in the control group at six months, can be lead to degeneration/destruction of a cartilage over a long period of time after implantation, and these abnormal cells were observed especially in the boundary regions around a fissure between a repaired tissue and an adjacent normal cartilage. Considering the above, TECs could contribute to osteochondral repair with longer term durability and excellent biological integration. This is because a TEC has fibronectin and vitronectin in abundance, thus having excellent adhesion capability and has paracrine action that promotes maturation to tissues surrounding an implant.

Furthermore, a composite tissue (hybrid material) of the present invention contributes to repair of a subchondral bone, particularly the repair in the early stages after an operation. It is important to stabilize a subchondral bone for osteochondral repair [GomollAH, MadryH, KnutsenG, vanDijkN, SeilR, Brittberg M, et al., Knee Surg Sports Traumatol Arthrosc 2010; 18:434-447; Kon E, Delcogliano M, Filardo G, Busacca M, Di Martino A, Marcacci M. , Am J Sports Med 2011; 39:1180-1190] . This is because a decline in therapeutic results of cartilage cell implantation was observed in the presence of an injury in a subchondral bone [Minas T, Gomoll AH, Rosenberger R, Royce RO, Bryant T., Am J Sports Med2009; 37:902-908] . Meanwhile, the present inventors reported the usefulness of an HA-based artificial bone for repairing a subchondral bone [Tamai N, Myoui A, Hirao M, Kaito T, Ochi T, Tanaka J, et al., Osteoarthritis Cartilage 2005; 13: 405-417]. In addition, the present inventors have demonstrated that progress in repair of a subchondral bone and reliable and excellent biological integration of a tissue with an adjacent host tissue were maintained until six months after a surgical operation that uses a composite tissue (hybrid graft) of the present invention. These results demonstrate that a three-dimensional synthetic tissue used in the present invention promotes osteogenesis from an adjacent bone marrow cells through some type of bioactive paracrine effect. Furthermore, it is demonstrated that a composite tissue (hybrid graft) of the present invention can guarantee long-term durability and be useful in the introduction of a rehabilitation program at an early stage after an operation in a clinical situation.

Formation ratios of a bone and cartilage were significantly correlated in a model of the present inventors. These results can be useful in elucidating the repair process of a cartilage and a subchondral bone. The present inventors presume the following from these results. In view of cartilage-like cells being observed around a repaired new bone, first, it is believed that cartilage differentiation of MSCs derived from a native bone marrow or an implanted TEC is promoted, and then osteogenesis is promoted due to ossification in a cartilage. However, in some examples, a fibrous tissue or cartilage-like tissue remained in a tissue region under the cartilage. These observations are less frequently observed in the TEC group in comparison to the control group. Thus, a TEC is believed to provide an environment suitable for bone repair. It is believed that a reason therefor is that TECs can prevent infiltration of joint effusion because TECs can readily adhere to an adjacent host tissue.

In a technical aspect, the present inventors implanted an artificial bone at a depth of 2 mm from a joint surface and under an adjacent natural cartilage. Although it is not desired to be constrained by theory, the present inventors believe that a suitable depth of an implanted composite tissue and a suitable strength of an implantation site can contribute to supplementing mesenchymal stem cells. It is understood that a suitable depth can be appropriately set by those skilled in the art while referring to the descriptions herein.

A repaired osteochondral tissue treated with a hybrid graft recovered rigidity that is equivalent to a normal osteochondral tissue. Furthermore, surface roughness calculated from digital images did not exhibit a significant difference between the untreated normal tissue group and the TEC group. However, rigidity and surface roughness also did not have a significant difference between the control group and the TEC group. In the present application, only the surface of a cartilage layer is represented in biodynamic test results. When the entire osteochondral tissue is evaluated, it is contemplated that a significant difference may be observed between the control group and the TEC group.

As a potential constrain of the present application, the present inventors did not use a large animal model, such as pigs, goats and sheep. However, the present inventors demonstrated that a hybrid graft of the present invention clearly promoted the osteochondral repair with excellent biological integration with tissues and maintained excellent quality of repaired tissue and excellent biological integration with a tissue until six months after an operation. Thus, the same result is expected even when a hybrid graft of the present invention is used in a large animal model. As another issue, excessive growth of a subchondral bone (thinning of cartilage) or exposure of a subchondral bone was observed in some cases in both groups, six months after a surgical operation. Such phenomenon was previously reported in a rabbit osteochondral defect model using MSCs [Wakitani S, Goto T, Pineda SJ, Young RG, Mansour JM, Caplan AI, et al. , J Bone Joint Surg Am 1994; 76:579-592] . Suchphenomenon can be induced by acceleration of ossification in a cartilage or deterioration of implanted MSCs [Chen H, Chevrier A, Hoemann CD, Sun J, Ouyang W, Buschmann MD. , Am J Sports Med 2011; Bedi A, Feeley BT, Williams RJ, 3rd., J Bone Joint Surg Am 2010; 92:994-1009; Vasara AI, Hyttinen MM, Lammi MJ, Lammi PE, Langsjo TK, Lindahl A, et al. , Calcif Tissue Int 2004; 74:107-114] . Thus, for improvement in durability and for excellent quality, it would be ideal to use a cocktail of growth factors against ossification in a cartilage to maintain a permanent cartilage [Liu G, Kawaguchi H, Ogasawara T, Asawa Y, Kishimoto J, Takahashi T, et al., J Biol Chem 2007; 282:20407-20415] or to select and use MSCs having a strong cartilage forming capability.

### (Conclusion)

In summary, the present inventors demonstrated that a composite tissue (hybrid graft) produced from a TEC and an artificial bone significantly improves osteochondral repair histologically and biodynamically. In particular, progress of subchondral bone repair and reliable and excellent biological integration of a tissue with an adjacent host tissue can secure long term durability. Since TECs are not dependent on a scaffold, TECs do not contain an animal or chemical material-derived substance. Furthermore, HA is extensively used in clinical settings. Thus, a hybrid material of the present inventors is suitable for efficient and safe repair of an osteochondral injury.

### (Example 2: Example of composite tissue using porous β-tricalcium phosphate)

A similar composite tissue was produced by using a production method similar to Example 1 to examine the therapeutic effect thereof. NEOBONE^{™} in Example 1 was replaced with porous β-tricalcium phosphate (OSferion, Olympus) to carry out the Example.

### (Collection of synovial tissue and isolation of cell)

All animal experiments were approved by the Osaka University Faculty of Medicine animal experiment facility. Rabbit synovial membrane was aseptically collected from a knee joint of a rabbit with a mature (24 weeks of age) skeleton within 12 hours post mortem. The protocol of cell isolation is substantially the same as the protocol previously used in isolation of MSCs derived from a human synovial membrane [Ando W, Tateishi K, Katakai D, Hart DA, Higuchi C, Nakata K, et al., Tissue Eng Part A 2008; 14:2041-2049] . Briefly stated, synovial membrane samples were rinsed with phosphate buffered saline (PBS), cut thoroughly, and digested with 0.4% collagenase XI (Sigma-Aldrich, St. Louis, MO, USA) for two hours at 37°C. After the collagenase was neutralized with a growth medium containing a high-glucose Dulbecco's modifiedeagle's medium (HG-DMEM; Wako, Osaka, Japan) added with 10% fetal bovine serum (FBS; HyClone, Logan, UT, USA) and 1% penicillin/streptomycin (Gibco BRL, Life Technologies Inc., Carlsbad, CA, USA), cells were collected by centrifugation, washed with PBS, resuspended in a growth medium, and plated on a culture dish. The characteristics of rabbit cells were similar to human synovial membrane-derived MSCs in terms of form, growth characteristics, and pluripotency (into bone forming system, cartilage forming system and adipocyte system) [Ando W, Tateishi K, Katakai D, Hart DA, Higuchi C, Nakata K, et al., Tissue Eng Part A 2008; 14:2041-2049; Tateishi K, Higuchi C, Ando W, Nakata K, Hashimoto J, Hart DA, et al., Osteoarthritis Cartilage 2007; 15: 709-718] . For cell growth, these cells were grown in a growth medium at 37°C in a humidified atmosphere of 5% CO₂. The medium was changed once a week. When cells reached confluence 7-10 days after primary culture, the cells were washed twice with PBS, collected after treatment with trypsin-EDTA (0.25% trypsin and 1 mM EDTA: Gibco BRL, Life Technologies Inc. , Carlsbad, CA, USA), and diluted to 1:3 ratio for the first subculture. The cells were again plated. When culture cells substantially reached confluence, the cells were diluted to 1:3 ratio to continue passage of cells with the same method. The present application used cells of 3-7 passages.

### (Production of three-dimensional tissue (TEC))

Synovial membrane MSCs were plated on a 6-well plate (9.6 cm²) at a density of 4.0 × 10⁵ cells/cm² in a growth medium containing 0.2 mM of ascorbate-2-phosphate (Asc-2P), which is the optimal concentration from previous studies [AndoW, Tateishi K, Katakai D, Hart DA, Higuchi C, Nakata K, et al., Tissue Eng Part A 2008; 14:2041-2049; Ando W, Tateishi K, Hart DA, Katakai D, Tanaka Y, Nakata K, et al., Biomaterials 2007; 28:5462-5470; Shimomura K, Ando W, Tateishi K, Nansai R, Fujie H, Hart DA, et al., Biomaterials 2010; 31:8004-8011]. The cells reached confluence within a day. Furthermore, after continuous culturing for 7-14 days, a complex of culture cells and ECM synthesized by said cells was detached from the lower layer by applying shearing stress by using a light pipette. The detached monolayer complex was maintained in a suspension to allow formation of a three-dimensional structure by active self tissue contraction. This tissue was called a three-dimensional TEC that is not dependent on a scaffold (also called scaffold-free).

### (Production of composite tissue (hybrid graft) consisting of TEC and artificial bone)

Sufficiently interconnected porous β-tricalcium phosphate (OSferion, Olympus) (diameter 5 mm, height 4 mm) was used as an artificial bone. A TEC was detached from a culture dish immediately prior to an implant surgery and connected to an artificial bone without using an adhesive to produce an osteochondral hybrid (Figure **1a**). Once the TEC integrates with the artificial bone, the integration is so strong that separation thereof is difficult.

### (Implantation of hybrid graft to osteochondral defect)

8 New Zealand white rabbits with a mature skeleton (24 weeks of age or older) were maintained in a cage while freely providing food and water. The rabbits were administered with anesthesia by an intravenous injection of 1 ml of pentobarbital [50 mg/ml (Nembutal^{®}, Dainippon Pharmaceutical Co. Ltd., Osaka, Japan)) and 1ml of xylazine hydrochloride (25 mg/ml (Seractal^{®}, Bayer, Germany)). The rabbits were shaved and disinfected. After covering with a sterilized fabric, a straight 3 cm incision was made on the side of the inside patella of the right knee. The patella was moved outward to expose the femoral fossa. A joint osteochondral defect through the entire thickness of 5 mm diameter and 6 mm depth was mechanically made in the femoral fossa of the right distal femur. A hybrid of a TEC and artificial bone was formed immediately prior to implantation. Then, a diphasic graft was implanted within the defect of the right knee of 4 rabbits (TEC group) . In a control group, only an artificial bone was implanted in a defect of the right knee of 4 rabbits. The right limbs of all the animals were secured in a cast for 7 days. The rabbits were then euthanized under anesthesia at 6 months after the surgical operation. The implantation sites were secured for use in the following paraffin section production and histological analysis.

### (Histological evaluation of repairs tissue)

For histological evaluation, tissues were fixed with 10% formalin neutral buffer, decalcified with K-CX (Falma, Tokyo, Japan) and embedded in paraffin to produce a 4 mm section. The section was stained with hematoxylin-eosin (H&E) and toluidine blue.

### (Results)

Six months after a surgical operation, a repaired tissue in the TEC group was completely repaired with an osteochondral tissue. A certain degree of osteochondral repair was also observed in the control group. However, the repaired tissue exhibited insufficient osteochondral repair with fibrous tissues and cartilage-like tissues mixed in at a subchondral region and poor biological integration with an adjacent cartilage.

### (Example 3: Experiments in another example of mesenchymal-like stem cells)

In Example 3, for cells other than those used in Example 1, for example, a three-dimensional synthetic tissue can be produced by using the methods described in Production Examples 1-6 to produce therewith a composite tissue by a method similar to Example 1 or 2 to carry out an efficient and safe repair experiment of osteochondral injury in a method similar to Example 1 or 2.

### (Example 4: Example in pigs/rats/humans as a hypothetical Example)

A composite tissue of pigs/rats/humans is produced instead of a synthetic tissue of a rabbit in Production Examples 1-6, Example 1 or 2 to conduct tissue evaluation or biodynamic evaluation that is similar to Examples 1 and 2.

### (Example 5: Experiments in which size (depth) is changed)

In accordance with a protocol similar to Example 1 or 2, the depth of an artificial bone was set as follows while referring to the procedures of Production Examples 1-6 as needed. Repairing capability of each of (3)-(5) was compared: (1) position of artificial bone is the same as the cartilage surface layer; (2) same depth as subchondral bone (bone/cartilage boundary); (3) about 2.0 mm deeper than the cartilage surface layer; (4) about 3. 0 mm deeper than the cartilage surface layer; and (5) about 4 mm deeper than the cartilage surface layer. (1) was omitted because it is believed to be evident that an artificial bone physically blocks cartilage repair. (2) was omitted because repair of the foundation, subchondral bone, is predicted to be impeded. Tissue evaluation and biodynamic evaluation similar to Examples 1 and 2 were conducted on the produced composite tissues. Figure **11** shows the cases (3) - (5) . As shown in Figure **11****,** it has become clear that regeneration differs depending on the implant depth of a complex. When shallow as in about 2 mm, a subchondral bone is repaired quickly, but the cartilage is poorly repaired. When deep as in about 4 mm, the cartilage is repaired well, but the repair of subchondral bone is prolonged. Thus, although it depends on the case, it can be said that it is possible to use at about 1-6 mm from the cartilage surface layer, more preferably at about 2-4 mm from the cartilage surface layer, and more preferably at about 3 mm from the cartilage surface layer.

### (Example 6: Experiments in another example of mesenchymal-like stem cells induced from iPS cells)

The present Example will confirm that an experiment of an efficient and safe repair of an osteochondral injury can be conducted by a method similar to Example 1 by inducing mesenchymal stem cells, also called mesenchymal-like stem cells, from iPS cells to produce a three-dimensional synthetic tissue therewith and producing a composite tissue by a method similar to Example 1.

Induction from iPS cells into mesenchymal-like stem cells can be carried out by referring to Jung et al, STEM CELLS, 2011; doi:10.1002/stem.727.

### (Example 7: Implementation with ES cells)

The present Example confirmed that an experiment of an efficient and safe repair of an osteochondral injury can be conducted by a method similar to Example 1 by inducing mesenchymal stem cells, also called mesenchymal-like stem cells, from rabbit ES cells to produce a three-dimensional synthetic tissue therewith and producing a composite tissue by a method similar to Example 1. Experiment was carried out by obtaining an approval of Osaka University Faculty of Medicine animal experiment facility and an approval for genetic engineering experiment upon implementation.

For induction from ES cells into mesenchymal stem cells, also called mesenchymal-like stem cells, the following can be referred for example: de Peppo et al., TISSUE ENGINEERING: Part A, 2010; 16; 3413-3426; Toh et al., Stem Cell Rev.and Rep., 2011; 7:544-559; Varga et al., Biochem.Biophys.Res.Commun., 2011; doi:10.1016/j.bbrc.2011.09.089; Barbet et al., Stem Cells International, 2011, doi:10.4061/2011/368192; Sanchez et al., STEM CELLS, 2011; 29:251-262; Simpson et al., Biotechnol.Bioeng., 2011; doi:10.1002/bit.23301.

### (Induction of mesenchymal stem cells (MSCs) induced from embryonic stem cells (ESCs) to differentiate)

Inner cell mass was collected and cultured in a feeder cell (MEF) to induce ESCs. Next, an embryoid body (EB) was made and induced to differentiate into MSCs (ES-MSCs) in plate culture under controlled oxygen partial pressure.

### (Production of TEC and TEC/artificial bone hybrid implant)

ES-MSCs inoculated at 4 × 10⁵/cm² were cultured for two weeks in a culture solution (HG-DMEM) containing ascorbic acid 2-phosphate. The cells were detached from the bottom surface by shear stress to form a three-dimensional structure to produce TECs. Furthermore, the TECS were placed on an artificial bone with any shape for integration by the adhesion property thereof to produce a TEC/artificial bone hybrid implant.

### (Cartilage repair of rabbit osteochondral defect)

An integrated implant of a TEC made with ES-MSCs and an artificial bone with φ 5mm × height 4 mm was implanted in a φ 5mm × height 6 mm osteochondral defect in a rabbit knee joint. Evident cartilage repair due to a TEC/artificial bone hybrid implant in comparison to a knee with only a defect was observed at one month after an operation (Figure **10**).

### (Example 8: Comparative experiment on integration (comparable data between integration with composite tissue and integration with BMP))

Results of integration using BMP and integration with a composite tissue of the present invention are compared in accordance with the procedure of Non Patent Literature 16 (Tamai N, Myoui A, Hirao M, Kaito T, Ochi T, Tanaka J, et al., Osteoarthritis Cartilage 2005; 13: 405-417) . As already stated, biological integration is excellent when integration with a composite tissue of the present invention is studied based on Example 1 or the like, whereas excellent biological integration is not observed when using BMP, as described in Non Patent Literature 16. Significance of the effect of the present invention is confirmed even in comparison to a case using a cytokine such as bone morphogenetic proteins.

### [Industrial Applicability]

The present invention usefully provides a basic therapeutic method, technique, pharmaceutical agent, and medical device for diseases which are conventionally difficult to treat. Particularly, the present invention provides an epoch-making therapy and prevention because it promotes recovery to a substantially native state. The present invention also provides a pharmaceutical agent, cell, tissue, composition, system, kit, and the like, which are used for such an epoch-making therapy and prevention.

There is a demand for repair and regeneration of joint tissues, mainly including bones and cartilages which are targeted by the present invention. The number of bone fracture patients, which are targeted by bone regeneration, reaches several hundreds of thousands per year. It is also said that there are 30 million potential patients having osteoarthritis which is the main target of cartilage regenerative therapy. Thus, the potential market is huge. The present invention is also highly useful for peripheral industries. Acute competition has been started in the regenerative medical research on joint tissues, mainly including bone and cartilage. The synthetic tissue of the present invention is a safe and original material made of cells collected from an organism, such as a patient or the like, and is highly useful in view of the lack of side effects or the like.

## Claims

1. A composite tissue comprising a three-dimensional synthetic tissue and an artificial bone for use in a method of treating or preventing a disease, disorder, or condition associated with an osteochondral defect, wherein the artificial bone is smaller in size than a depth of a defect of a bone section in the osteochondral defect by 2-4 mm, the three-dimensional synthetic tissue is substantially made of a cell and an extracellular matrix derived from the cell, the extracellular matrix contains fibronectin, collagen I, collagen III, and vitronectin, the extracellular matrix is diffusedly distributed in the tissue, the extracellular matrix and the cell biologically integrate to form a three-dimensional structure together, and the composite tissue has an ability to biologically integrate with surroundings when implanted and has sufficient strength to provide a self-supporting ability.

2. The composite tissue for use according to claim 1, wherein a total of depths of the artificial bone and the three-dimensional synthetic tissue is the same or 1 mm shorter than the depth of the osteochondral defect.

3. The composite tissue for use according to claim 1 or 2, wherein the artificial bone is smaller in size than the depth of the defect of the bone section in the osteochondral defect by twice the thickness of a cartilage or less.

4. The composite tissue for use according to any of claims 1 to 3, wherein the artificial bone is smaller in size than the depth of the defect of the bone section in the osteochondral defect by 2-3 mm.

5. The composite tissue for use according to any of claims 1 to 4, wherein the three-dimensional synthetic tissue and the artificial bone are diphasic, or the three dimensional synthetic tissue and the artificial bone are attached to each other.

6. The composite tissue for use according to any of claims 1 to 5, wherein the osteochondral defect is in a mammal.

7. The composite tissue for use according to any of claims 1 to 6, wherein the artificial bone is made of a material selected from the group consisting of hydroxyapatite and β-tricalcium phosphate.

8. The composite tissue for use according to any of claims 1 to 7, wherein the disease, disorder, or condition is selected from the group consisting of osteoarthritis, osteochondral injury, osteochondral lesion, osteonecrosis, rheumatoid arthritis and bone tumor.

9. The composite tissue for use according to any of claims 1 to 8, wherein the cell is selected from the group consisting of a myoblast, mesenchymal stem cell, adipocyte, synovial cell, and bone marrow cell and an extracellular matrix derived from the cell, the extracellular matrix contains more of the collagen I and/or collagen III than collagen II, and the extracellular matrix is diffusedly distributed in the tissue.

10. A kit comprising a composite tissue comprising a three-dimensional synthetic tissue and an artificial bone for use in a method of treating or preventing a disease, disorder, or condition associated with an osteochondral defect, wherein the artificial bone is smaller in size than a depth of a defect of a bone section in the osteochondral defect by 2-4 mm, the three-dimensional synthetic tissue is substantially made of a cell and an extracellular matrix derived from the cell, the extracellular matrix contains fibronectin, collagen I, collagen III, and vitronectin, the extracellular matrix is diffusedly distributed in the tissue, the extracellular matrix and the cell biologically integrate to form a three-dimensional structure together, and the composite tissue has an ability to biologically integrate with surroundings when implanted and has sufficient strength to provide a self-supporting ability.

## Patentansprüche

1. Verbundgewebe, das ein dreidimensionales synthetisches Gewebe und einen künstlichen Knochen umfasst, zur Verwendung in einem Verfahren zur Behandlung oder Vorbeugung einer Krankheit, einer Störung oder eines Zustands, der mit einem osteochondralen Defekt verbunden ist, wobei der künstliche Knochen um 2 bis 4 mm kleiner ist als die Tiefe eines Defekts eines Knochenabschnitts in dem osteochondralen Defekt, das dreidimensionale synthetische Gewebe im Wesentlichen aus einer Zelle und einer von der Zelle abgeleiteten extrazellulären Matrix hergestellt ist, die extrazelluläre Matrix Fibronektin, Kollagen I, Kollagen III und Vitronektin enthält, die extrazelluläre Matrix diffus in dem Gewebe verteilt ist, die extrazelluläre Matrix und die Zelle sich biologisch integrieren, um zusammen eine dreidimensionale Struktur zu bilden, und das zusammengesetzte Gewebe die Fähigkeit hat, sich biologisch in die Umgebung zu integrieren, wenn es implantiert wird, und eine ausreichende Festigkeit hat, um eine selbsttragende Fähigkeit bereitzustellen.

2. Verbundgewebe zur Verwendung nach Anspruch 1, wobei die Gesamttiefe des künstlichen Knochens und des dreidimensionalen synthetischen Gewebes gleich oder 1 mm kürzer als die Tiefe des osteochondralen Defekts ist.

3. Verbundgewebe zur Verwendung nach Anspruch 1 oder 2, wobei der künstliche Knochen um die doppelte Dicke eines Knorpels oder weniger kleiner ist als die Tiefe des Defekts des Knochenabschnitts in dem osteochondralen Defekt.

4. Verbundgewebe zur Verwendung nach einem der Ansprüche 1 bis 3, wobei der künstliche Knochen um 2 bis 3 mm kleiner ist als die Tiefe des Defekts des Knochenabschnitts in dem osteochondralen Defekt.

5. Verbundgewebe zur Verwendung nach einem der Ansprüche 1 bis 4, wobei das dreidimensionale synthetische Gewebe und der künstliche Knochen diphasisch sind oder das dreidimensionale synthetische Gewebe und der künstliche Knochen miteinander verbunden sind.

6. Verbundgewebe zur Verwendung nach einem der Ansprüche 1 bis 5, wobei der osteochondrale Defekt bei einem Säugetier vorliegt.

7. Verbundgewebe zur Verwendung nach einem der Ansprüche 1 bis 6, wobei der künstliche Knochen aus einem Material hergestellt ist, das aus der Gruppe ausgewählt ist, die aus Hydroxyapatit und β-Tricalciumphosphat besteht.

8. Verbundgewebe zur Verwendung nach einem der Ansprüche 1 bis 7, wobei die Krankheit, Störung oder der Zustand ausgewählt ist aus der Gruppe bestehend aus Osteoarthritis, osteochondraler Verletzung, osteochondraler Läsion, Osteonekrose, rheumatoider Arthritis und Knochentumor.

9. Verbundgewebe zur Verwendung nach einem der Ansprüche 1 bis 8, wobei die Zelle aus der Gruppe ausgewählt ist, die aus einem Myoblasten, einer mesenchymalen Stammzelle, einem Adipozyten, einer Synovialzelle und einer Knochenmarkszelle besteht, und eine extrazelluläre Matrix, die von der Zelle abgeleitet ist, wobei die extrazelluläre Matrix mehr Kollagen I und/oder Kollagen III als Kollagen II enthält und die extrazelluläre Matrix diffus im Gewebe verteilt ist.

10. Kit, das ein zusammengesetztes Gewebe umfasst, das ein dreidimensionales synthetisches Gewebe und einen künstlichen Knochen umfasst, zur Verwendung in einem Verfahren zur Behandlung oder Vorbeugung einer Krankheit, Störung oder eines Zustands, die mit einem osteochondralen Defekt verbunden sind, wobei der künstliche Knochen um 2 bis 4 mm kleiner ist als die Tiefe eines Defekts eines Knochenabschnitts in dem osteochondralen Defekt, das dreidimensionale synthetische Gewebe im Wesentlichen aus einer Zelle und einer von der Zelle abgeleiteten extrazellulären Matrix hergestellt ist, die extrazelluläre Matrix Fibronektin, Kollagen I, Kollagen III und Vitronektin enthält, die extrazelluläre Matrix diffus in dem Gewebe verteilt ist, die extrazelluläre Matrix und die Zelle sich biologisch integrieren, um zusammen eine dreidimensionale Struktur zu bilden, und das zusammengesetzte Gewebe die Fähigkeit hat, sich biologisch in die Umgebung zu integrieren, wenn es implantiert wird, und eine ausreichende Festigkeit hat, um eine selbsttragende Fähigkeit bereitzustellen.

## Revendications

1. Tissu composite comprenant un tissu synthétique tridimensionnel et un os artificiel destiné à être utilisé dans un procédé de traitement ou de prévention d'une maladie, d'un trouble ou d'un état associé à un défaut ostéochondral, dans lequel l'os artificiel a une taille plus petite de 2 à 4 mm qu'une profondeur d'un défaut d'une section d'os dans le défaut ostéochondral, le tissu synthétique tridimensionnel est constitué substantiellement d'une cellule et d'une matrice extracellulaire dérivée de la cellule, la matrice extracellulaire contient de la fibronectine, du collagène I, du collagène III et de la vitronectine, la matrice extracellulaire est répartie de manière diffuse dans le tissu, la matrice extracellulaire et la cellule s'intègrent biologiquement pour former ensemble une structure tridimensionnelle, et le tissu composite a une capacité à s'intégrer biologiquement avec le milieu extérieur lorsqu'il est implanté et a une résistance suffisante pour donner une capacité rigide.

2. Tissu composite destiné à être utilisé selon la revendication 1, dans lequel le total des profondeurs de l'os artificiel et du tissu synthétique tridimensionnel est égal ou inférieur d'un millimètre à la profondeur du défaut ostéochondral.

3. Tissu composite destiné à être utilisé selon la revendication 1 ou 2, dans lequel l'os artificiel a une taille plus petite que la profondeur du défaut de la section d'os dans le défaut ostéochondral de deux fois l'épaisseur d'un cartilage ou moins.

4. Tissu composite destiné à être utilisé selon l'une quelconque des revendications 1 à 3, dans lequel l'os artificiel a une taille plus petite de 2 à 3 mm que la profondeur du défaut de la section d'os dans le défaut ostéochondral.

5. Tissu composite destiné à être utilisé selon l'une quelconque des revendications 1 à 4, dans lequel le tissu synthétique tridimensionnel et l'os artificiel sont diphasiques, ou le tissu synthétique tridimensionnel et l'os artificiel sont fixés l'un à l'autre.

6. Tissu composite destiné à être utilisé selon l'une quelconque des revendications 1 à 5, dans lequel le défaut ostéochondral est chez un mammifère.

7. Tissu composite destiné à être utilisé selon l'une quelconque des revendications 1 à 6, dans lequel l'os artificiel est fait d'un matériau choisi dans le groupe comprenant l'hydroxyapatite et le phosphate β-tricalcique.

8. Tissu composite destiné à être utilisé selon l'une quelconque des revendications 1 à 7, dans lequel la maladie, le trouble ou l'état est choisi dans le groupe comprenant l'arthrose, la blessure ostéochondrale, la lésion ostéochondrale, l'ostéonécrose, la polyarthrite rhumatoïde et la tumeur des os.

9. Tissu composite destiné à être utilisé selon l'une quelconque des revendications 1 à 8, dans lequel la cellule est choisie dans le groupe comprenant un myoblaste, une cellule souche mésenchymateuse, un adipocyte, une cellule synoviale et une cellule de moelle osseuse et une matrice extracellulaire dérivée de la cellule, la matrice extracellulaire contient plus du collagène I et/ou collagène III que du collagène II, et la matrice extracellulaire est répartie de manière diffuse dans le tissu.

10. Nécessaire comprenant un tissu composite comprenant un tissu synthétique tridimensionnel et un os artificiel destiné à être utilisé dans un procédé de traitement ou de prévention d'une maladie, d'un trouble ou d'un état associé à un défaut ostéochondral, dans lequel l'os artificiel a une taille plus petite de 2 à 4 mm qu'une profondeur d'un défaut d'une section d'os dans le défaut ostéochondral, le tissu synthétique tridimensionnel est constitué substantiellement d'une cellule et d'une matrice extracellulaire dérivée de la cellule, la matrice extracellulaire contient de la fibronectine, du collagène I, du collagène III et de la vitronectine, la matrice extracellulaire est répartie de manière diffuse dans le tissu, la matrice extracellulaire et la cellule s'intègrent biologiquement pour former ensemble une structure tridimensionnelle, et le tissu composite a une capacité à s'intégrer biologiquement avec le milieu extérieur lorsqu'il est implanté et a une résistance suffisante pour donner une capacité rigide.
